(19) **Europäisches Patentamt**
**European Patent Office**
**Office européen des brevets**

(11) **EP 4 772 879 A1**

(12) **EUROPEAN PATENT APPLICATION**

(43) Date of publication:
**08.07.2026  Bulletin 2026/28**

(21) Application number: **25150474.2**

(22) Date of filing: **07.01.2025**

(51) International Patent Classification (IPC):
**G01N 33/50** *(2006.01)*       **G01N 33/68** *(2006.01)*
**C12N 5/10** *(2006.01)*

(52) Cooperative Patent Classification (CPC):
**G01N 33/6872; A61K 31/53; A61K 31/55;
A61P 25/08; C07K 14/47; G01N 33/5058;
G01N 33/5088;** G01N 2800/28; G01N 2800/2857

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Rheinische Friedrich-Wilhelms-
Universität Bonn**
**53113 Bonn (DE)**

(72) Inventors:
• **BECK, Heinz**
  **53127 Bonn (DE)**

• **ARAKI, Kunihiko**
  **53127 Bonn (DE)**

(74) Representative: **Vossius & Partner**
**Patentanwälte Rechtsanwälte mbB**
**Siebertstrasse 3**
**81675 München (DE)**

Remarks:
•The complete document including Reference Table(s) and the Sequence Listing(s) can be downloaded from the EPO website
•Amended claims in accordance with Rule 137(2) EPC.

(54) **SCREENING FOR AND DEVELOPMENT OF SODIUM CHANNEL BLOCKERS FOR THE TREATMENT OF PHARMACORESISTANT NEUROLOGICAL DISORDERS BASED ON A POLYAMINE MECHANISM**

(57)    The present invention relates to a method of screening for a voltage-gated sodium (NaV) channel blocker (NaVCB), or for an NaVCB which is pharmacologically effective in the treatment of a pharmacoresistant neuronal hyperexcitability disease/disorder (PRNHD), wherein a test compound is contacted with a functional NaV channel in the presence of an increased level of N1-acetylspermidine (N1aSPD) and said test compound is identified as said NaVCB if it blocks said functional NaV channel in the presence of said increased level of N1aSPD. The present invention further relates to a two-step method of screening for an NaVCB comprising operating one of said methods in a high-throughput mode and subsequently operating one of said methods in a high-reliability mode. The present invention further relates to an NaVCB as identified according to any of these methods, to an NaVCB which blocks a functional NaV channel in the presence of an increased level of N1aSPD and to a pharmaceutical composition comprising such an NaVCB. The present invention further relates to a pharmaceutical composition comprising oxcarbazepine (OXC) and/or Eslicarbazepine acetate/Eslicarbazepine (ESL) for use in treating a PRNHD.

EP 4 772 879 A1

## Description

The present invention relates to a method of screening for a voltage-gated sodium (Nav) channel blocker (Na$_V$CB), or for Na$_V$CB which is pharmacologically effective in the treatment of a pharmacoresistant neuronal hyperexcitability disease or disorder (PRNHD), in particular in the treatment of a pharmacoresistant Nav channel-hyperactivity disease or disorder (PRNavCHD). In the context of the method of screening of the invention, a test compound is contacted with a functional Na$_V$ channel (Na$_V$C) in the presence of an increased level of N1-acetylspermidine (N1aSPD), and said test compound is identified as said NavCB if it blocks said functional Nav channel in the presence of said increased level of N1aSPD. The present invention further relates to a two-step method of screening for an NavCB comprising a first step of operating one of the methods of screening of the invention in a high-throughput mode (for example *in vitro* or *ex vivo* and/or by using a cell model or neuronal culture system model) and a second step of operating one of the methods of screening of the invention as a confirmatory testing in a high-reliability mode (for example by using an *in vivo* animal model). The present invention further relates to an Na$_V$CB as identified according to any of the methods of screening of the invention; and further to an NavCB which blocks a functional Nav channel in the presence of an increased level of N1aSPD. The present invention further relates to a pharmaceutical composition comprising such an Na$_V$CB. The pharmaceutical composition may be for use in the treatment of a neuronal hyperexcitability disease/disorder (NHD), for example in the treatment of a Nav channel-hyperactivity disease/disorder (Na$_V$CHD); in particular in the treatment of a PRNHD, more particular in the treatment of a PRNHD, which is based on a polyamine mechanism (for example, increased N1aSPD level, increased ratio of N1aSPD/N1aSPM levels, and/or decreased N1aSPM level). Moreover, the present invention relates to a pharmaceutical composition comprising oxcarbazepine (OXC) and/or Eslicarbazepine/Eslicarbazepine acetate (ESL), or a structurally closely related NavCB, for use in treating of a PRNHD, for example a PRNavCHD. The present invention further relates to a device which is suitable for and adapted to a screening method of the invention; for example a control device or a test device. The invention also relates to a kit which, for example, comprises an intracellular solution in accordance with the invention, and also to a method for developing a Na$_V$CB which is pharmacologically effective in the treatment of a (PR)PRNHD, for example in the treatment of a (PR)Na$_V$CHD.

More than 65 million people worldwide are affected by epilepsy. Around 30% of these patients (approx. 20 million) are unable to control their seizures by medication (pharmacoresistant seizures (PRSs); also sometimes termed drug-resistant seizures (DRSs)), with considerable personal and socio-economic consequences. Similar situations exist for other neurological diseases and disorders; and seizures which may come along with these diseases/disorders (see, for example, Wiffen, Cochrane Database Syst Rev., 2011(1), 1-40; Wiffen, Cochrane Database Syst Rev., 2014(4), 1-41; Tham, Journal of Pain Research 11, 2018, 1689-98; Fischer, Ann Neurol. 65(6), 2009, 733-41). This has led to a high level of activities in the development of, for example, new antiseizure medications (ASMs), with numerous new ASMs entering the market in the last 20 years. While some of these new substances have a more favorable effect and side effect profile in certain patient groups, the percentage of pharmacoresistant patients remains high at around 30% over the last 10 years (Perucca, The Lancet. Neurology 6(9), 2007, 793-804; Perucca, The Lancet. Neurology 19 (6), 2020, 544-56; Golyala, Seizure 44, 2017, 147-56; Chen, JAMA neurology 75 (3), 2018, 279-86). The reasons for this are to be seen in a lack of understanding of the specific biological mechanism(s) which underly pharmacoresistance.

Most ASMs (sometimes also termed anticonvulsants or anticonvulsant drugs (ACDs)), and respective antiepileptic medications (AEMs; sometimes also termed antiepileptic drugs (AEDs); see Perucca, Epilepsia 65, 2024, 533-41 for the respective terminology to be used recently), suppress abnormal seizure-associated neuronal activity through a complex voltage- and use-dependent inhibition of Na$^+$ currents. In particular, the use-dependent block causes a preferential reduction in Na$^+$ channel availability during high-frequency discharges, as occur in, for example, epileptic seizures. In previous work, it was shown that transient Na$^+$ channels in chronically epileptic tissue are less susceptible to a use-dependent block by the AED carbamazepine (CBZ) in both, human and experimental epilepsy. This has been suggested as one candidate mechanism that may underlie the pharmacoresistance to CBZ (Remy, Brain: A Journal of Neurology 129 (1), 2006, 18-35; Remy, Annals of Neurology 53(4), 2003, 469-79). Specifically, in both, the pilocarpine model of epilepsy and therapy-resistant patients, CBZ effects on the recovery from sodium channel inactivation were strongly reduced (Remy 2003 *loc. cit.*).

Resistance to sodium channel blockers, however, has been observed not only in the context of epilepsy, but also in the context of other neurological disorders (see, for example, Tham *loc.* cit.; Fischer *loc. cit.*). For example, substantial sub-groups of patients with pain disorders, like trigeminal neuralgia, postherpetic neuralgia, diabetic neuropathy, erythromelalgia, fibromyalgia, and other forms of neuropathic pain and hereditary pain syndromes, are also unresponsive to the treatment with sodium channel blockers (see, for example, Tham *loc. cit.;* Fischer *loc. cit.*).

The findings concerning PRSs and pharmacoresistant epilepsy (PRE; also known as drug-resistant epilepsy (DRE); or termed epilepsy in AEM-resistant patients), and concerning other PRNHDs, like other pharmacoresistant neurological disorders (PRNHDs), triggered a year-long search for the fundamental change affecting the Na$^+$ channel molecule that leads to a loss of the use-dependent block for classical Na$_V$CBs, such as CBZ (see, for example, Remy 2003 *loc. cit.*). However, neither evidence was found that an altered expression of Na$^+$ channel subunit underlies this phenomenon (Ellerkmann, Neuroscience 119 (2), 2003, 323-33), nor evidence for an expression of accessory subunits (Uebachs, The Journal of Neuroscience 30 (25), 2010, 8489-501). The potential role of polyamines in epilepsy and epilepsy-related pathophysiological changes was also discussed in the art (e.g. Liu, Biomolecules 12, 1596, 2022, 1-25). Methods and medical device systems for treating seizures, making use of an association between the probability or likelihood of seizure occurrence and the concentrations or activity levels of putrescine, spermidine, spermine or GABA were also proposed (US-A1 2013/0345526). In addition, the potential role of spermine in pharmacoresistant epilepsy was discussed, as well as the upregulation of spermidine/spermine-$N_1$-acetyltransferase (SSAT) in chronic epilepsy (Beckonert, The Journal of Neuroscience 38 (24), 2018, 5596-605). However, many questions concerning a potential interrelation of polyamines and sodium channel pharmacology still remain unanswered.

All in all, the actual cause(s) and mechanism(s) underlying sodium channel pharmacoresistance, and pharmacoresistance in epilepsies and other PRNHDs, respectively, has/have not been discovered to date. Without this specific knowledge, however, it is difficult to identify and develop medications with modes of action that can overcome the existing pharmacoresistance. There is thus an unmet need in the field of sodium channel pharmacoresistance for means and methods which allow an efficient and reliable screening for and identification of such particular medications.

The problem underlying the present invention is therefore the provision of efficient and reliable means and methods for screening and identifying NavCBs which are pharmacologically effective in the treatment of PRNHDs.

The technical problem is solved by the provision of the embodiments characterized herein and in the claims.

The present invention solves the technical problem because, as documented herein below and in the appended examples, the core cell-biological basis of sodium channel resistance against $Na_V$CBs (like, for example, ASMs) was identified, namely a not yet considered interrelation between $Na_V$CBs and particular intracellular polyamines. Based on this resistance mechanism, means and methods for a reliable and rapid/efficient screening and identification for/of NavCBs which overcome respective pharmacoresistancies are provided herein. Moreover, the present invention allows for the design of specific NavCBs which overcome the cellular pharmacoresistance mechanism which has been identified in the context of the invention. Novel treatments for PRNHDs, and for respective PRSs, are thus also provided; and can further be developed on the basis of the present invention.

In particular, as also demonstrated in the appended examples, it was surprisingly found in the context of the invention that the magnitude of the response of NavCs to NavCBs (like the ASMs Carbamazepine (CBZ), Oxcarbazepine (OXC), Eslicarbazepine (ESL) and Lamotrigine (LTG)), does not depend on the amount/presence of the intracellular polyamines spermine and/or spermidine *as such,* but is actually dictated by the amount of intracellular N1-acetylspermidine (N1aSPD) and/or N1-acetylspermine (N1aSPM). More particular, it was surprisingly shown that increased intracellular N1aSPD concentrations significantly reduce NavC-blocking efficacy of multiple, commonly used $Na_V$CBs (like the ASMs, CBZ, OXC, ESL and LTG).

It was further demonstrated in the appended examples that structural modifications of NavCBs (even minor ones, like the modification of CBZ to OXC) can quantitatively modify the pharmacoresistance effect. Therefore, the means and methods described herein also provide for the option to do hit-to-lead development, and the option to systematically design and generate $Na_V$CBs which are quantitatively less susceptible to a modulation by $N_1$-acetyl-polyamines, and which are, thus, more suitable in treating PRNHDs and pharmacoresistant seizures.

In addition, it was shown in the context of the appended examples that the reduction of spermine synthase (SMS) resulted in an N1aSPD increase and in NavCB-resistant $Na^+$ channel activity, respectively. Moreover, the appended examples provide evidence that SMS is downregulated, and N1aSPD is increased, not only across models of both, acquired and genetic epilepsies, but also in human pharmacoresistant epilepsy. The cell-biological mechanism underlying $Na_V$C pharmacoresistance as identified in the context of the present invention can therefore be generalized to a broad range of PRNHDs, including respective seizures.

In a first aspect, the present invention relates to a method of screening for a NavCB,

wherein said method comprises (the step of) contacting a test compound with a NavC in the presence of N1aSPD, and wherein said test compound is identified as said NavCB if it inhibits/blocks (the activity of) said NavC in the presence of N1aSPD.

The present invention further relates to a method of screening for a NavCB,

wherein said method comprises (the step of) contacting a test compound with a NavC in the presence of an increased level of N1aSPD, and
wherein said test compound is identified as said NavCB if it inhibits/blocks (the activity of) said NavC in the presence of said increased level of N1aSPD.

The present invention further relates to a method of screening for a NavCB,

wherein said method comprises (the step of) contacting a test compound with a NavC in the presence of an N1aSPD concentration of $\geq 0.5$ μM, and
wherein said test compound is identified as said NavCB if it inhibits/blocks (the activity of) said NavC in the presence of said N1aSPD concentration.

In a second aspect, present invention relates to a method of screening for an NavCB which is pharmacologically effective in the treatment of a PRNHD,

wherein said method comprises (the step of) contacting a test compound with a NavC in the presence of N1aSPD, and
wherein said test compound is identified as said NavCB if it inhibits/blocks (the activity of) said NavC in the presence of N1aSPD.

The present invention further relates to a method of screening for an NavCB which is pharmacologically effective in the treatment of a PRNHD,

wherein said method comprises (the step of) contacting a test compound with a NavC in the presence of an increased level of N1aSPD, and
wherein said test compound is identified as said NavCB if it inhibits/blocks (the activity of) said NavC in the presence of said increased level of N1aSPD.

The present invention further relates to a method of screening for an NavCB which is pharmacologically effective in the treatment of a PRNHD,

wherein said method comprises (the step of) contacting a test compound with a NavC in the presence of an N1aSPD concentration of $\geq 0.5$ μM, and
wherein said test compound is identified as said NavCB if it inhibits/blocks (the activity of) said NavC in the presence of said N1aSPD concentration.

In the context of the first and the second aspect, the (step of) contacting may be in the presence of an increased ratio of the levels of N1aSPD/N1aSPM, and the test compound may be identified as said $Na_VCB$ if it inhibits/blocks (the activity of) said $Na_VC$ in the presence of said increased ratio of the levels of N1aSPD/N1aSPM.
The present invention also relates to a method of screening for a NavCB,

wherein said method comprises (the step of) contacting a test compound with a NavC in the presence of an increased ratio of the levels of N1aSPD/N1aSPM, and
wherein said test compound is identified as said NavCB if it inhibits/blocks (the activity of) said NavC in the presence of said increased ratio of the levels of N1aSPD/N1aSPM.

The present invention also relates to a method of screening for an NavCB which is pharmacologically effective in the treatment of a PRNHD,

wherein said method comprises (the step of) contacting a test compound with a NavC in the presence of an increased ratio of the levels of N1aSPD/N1aSPM, and
wherein said test compound is identified as said NavCB if it inhibits/blocks (the activity of) said NavC in the presence of said increased ratio of the levels of N1aSPD/N1aSPM.

The present invention also relates to a method of screening for a NavCB,

wherein said method comprises (the step of) contacting a test compound with a NavC in the presence of a decreased level of N1aSPM, and
wherein said test compound is identified as said NavCB if it inhibits/blocks (the activity of) said NavC in the presence of said decreased level of N1aSPM.

The present invention also relates to a method of screening for an NavCB which is pharmacologically effective in the treatment of a PRNHD,

wherein said method comprises (the step of) contacting a test compound with a NavC in the presence of a decreased level of N1aSPM, and

wherein said test compound is identified as said NavCB if it inhibits/blocks (the activity of) said NavC in the presence of said decreased level of N1aSPM.

In general, and if not explicitly indicated differently or contradicted by context, if reference is made herein to "NavC", a functional NavC is meant (see, for example, also below for further details). This particularly means that the $Na_VC$ is functional under the screening conditions in accordance with the invention; for example at the respective N1aSPD (and/or N1aSPM) levels/concentrations (see, for example, also below for further details).

In principle, the magnitude of the N1aSPD concentration and the increased level of N1aSPD, the decreased level of N1aSPM, and the increased ratio of the levels of N1aSPD/N1aSPM, in accordance with the invention is not particularly limited for the screening methods of the invention. Generally, an increased level of N1aSPD, a respective N1aSPD concentration, or an increased ratio of the levels of N1aSPD/N1aSPM, in accordance with the invention is a level of N1aSPD, a N1aSPD concentration, or a ratio of the levels of N1aSPD/N1aSPM, which is higher than the typical/normal level of N1aSPD, the typical/normal N1aSPD concentration, and the typical/normal ratio of the levels of N1aSPD/N1aSPM, respectively (for example in a typical/normal neuron). In other words, a level of N1aSPD, a N1aSPD concentration, or a ratio of the levels of N1aSPD/N1aSPM, is increased in accordance with the invention, if it is increased as compared to the typical/normal level of N1aSPD, as compared to the typical/normal N1aSPD concentration, and as compared to a typical/normal ratio of the levels of N1aSPD/N1aSPM, respectively. Typical/normal levels of N1aSPD and typical/normal N1aSPD concentrations, and typical/normal ratios of the levels of N1aSPD/N1aSPM, also represent control levels/ratios in accordance with the screening methods of the invention.

Increased levels of N1aSPD, the N1aSPD concentrations, and increased ratios of the levels of N1aSPD/N1aSPM, and/or decreased levels of N1aSPM, may equal those levels/ratios which typically occur in cells of affected individuals. "Affected" in this respect particularly means affected by a NHD, more particular affected by a PRNHD. For example, increased levels of N1aSPD, the N1aSPD concentrations, decreased levels of N1aSPM, and/or increased ratios of the levels of N1aSPD/N1aSPM, may correspond to the respective levels/ratios which occur intercellularly in neurons of affected individuals (preferably in neurons of affected human patients, in particular human PRNHD patients; but also in neurons of affected animals, including neurons of animal models of PRNHDs). More particular, the increased levels of N1aSPD, the N1aSPD concentrations, and the increased ratios of the levels of N1aSPD/N1aSPM (and/or the decreased levels of N1aSPM), may equal the respective levels/ratios in cells/neurons of a model of PRNHD. Said model of PRNHD may be a cell model of PRNHD, a neuronal culture system model of PRNHD or an animal model of PRNHD. Examples of animal models of PRNHD are the pilocarpine epilepsy model (as, for example, described in Remy *2003 loc. cit.;* Sanabria, J Physiol 532, 2001, 205-16) or SMS knockout mouse models (e.g. C57BL/6J/Sms[em6Lutzy]/J, C57BL/6Smoc-[SMSem1(flox)] [Smoc] or C57BL/6JGpt-Sms-flox). Cell models of PRNHD or neuronal culture system models of PRNHD may be derived from those animal models. Examples of cell, neuronal culture system and animal models of PRNHD are also described herein below and in the appended examples (for example SMS knockdown HEK cells (or other SMS knockdown cells), SMS knockdown primary neurons, human-derived neuronal systems, SMS knockdown mice or rats).

Further, in accordance with the invention, the levels of N1aSPD, the N1aSPD concentrations, and the ratios of the levels of N1aSPD/N1aSPM, may be increased as compared to the respective levels/ratios in a healthy/unaffected model (for example healthy/unaffected cell, neuronal culture system or animal model). They may even be increased as compared to the respective levels/ratios in a (cell, neuronal culture system or animal) model of pharmacoresponsive NHD.

Typical/normal reference levels/concentrations of N1aSPD and N1aSPM, and typical/normal reference ratios of the levels of N1aSPD/N1aSPM, may correspond to those which typically occur in cells of healthy/unaffected individuals. "Healthy/unaffected" in this respect particularly means unaffected by an NHD, more particular unaffected by a PRNHD (affected by a pharmacoresponsive NHD may be included here). For example, typical/normal reference levels/concentrations of N1aSPD and N1aSPM, and typical/normal reference ratios of the levels of N1aSPD/N1aSPM, may correspond to the respective levels/ratios which occur intercellularly in neurons of unaffected individuals (preferably neurons of unaffected human individuals; but also neurons of unaffected animals/unaffected animal models, including neurons of animal models of pharmacoresponsive NHDs). The typical/normal levels/concentrations of N1aSPD, and the typical/-normal ratios of the levels of N1aSPD/N1aSPM, may equal the respective levels/ratios in cells/neurons of such models. Such unaffected models may be unaffected cell models, unaffected neuronal culture system models or unaffected animal models. Typical/normal reference levels of N1aSPD, and typical/normal reference ratios of the levels of N1aSPD/N1aSPM, respectively, may represent control levels/ratios in accordance with the screening methods of the invention. The absence of N1aSPD is also included in this respect.

"Level" or levels" with respect to N1aSPD or N1aSPM, or with respect to any other polyamine, particularly means the respective concentration/concentrations. It is envisaged in the context of the screening methods of the invention that the increased levels of N1aSPD, the N1aSPD concentrations, and the increased ratios of the levels of N1aSPD/N1aSPM, and/or decreased levels of N1aSPM, and also the typical/normal reference levels/concentrations of N1aSPD and N1aSPM, and typical/normal reference ratios of the levels of N1aSPD/N1aSPM, are present at the intracellular side of (a) functional Nav channel(s), for example in the lumen of a cell which expresses (a) functional $Na_V$ channel(s) in its (outer) cell membrane. This means that they are present intracellularly.

In the context of the screening methods of the invention, the increased level of N1aSPD, the N1aSPD concentration, and/or the increased ratio of the levels of N1aSPD/N1aSPM, is envisaged to be so that a control NavCB (e.g. CBZ; or another NavCB which is pharmacologically ineffective in the treatment of a PRNHD, like a PRE) shows a reduced inhibition/blocking of the functional Nav channel in the presence of said level/concentration/ratio. This reduced inhibition/blocking may be as compared to a control/normal level of N1aSPD, and/or a control/normal ratio of the levels of N1aSPD/N1aSPM; or as compared to a situation when N1aSPD is absent. Further, this reduced inhibition/blocking may be as compared to a control NavCB which is effective, or expected to be effective, in the treatment of a PRNHD, like a PRE, (e.g. OXC), i.e. which is effective, or expected to be effective, in the presence of said increased level of N1aSPD, said N1aSPD concentration, and/or said increased ratio of the levels of N1aSPD/N1aSPM. The setting(s) of the screening methods of the invention may be adapted accordingly; the respective increased levels/ratios or concentrations may be chosen accordingly.

In the context of the screening methods of the invention, the increased level of N1aSPD, the N1aSPD concentration, and/or the increased ratio of the levels of N1aSPD/N1aSPM, may be increased by $\geq$2-fold, $\geq$3-fold, $\geq$4-fold, $\geq$5-fold, $\geq$6-fold, $\geq$7-fold, $\geq$8-fold, $\geq$9-fold, $\geq$10-fold, $\geq$11-fold, $\geq$12-fold, $\geq$13-fold, $\geq$14-fold, $\geq$15-fold, $\geq$20-fold, $\geq$25-fold or $\geq$30-fold; as compared to a respective control level/control ratio; for example a control level/ratio or typical/normal reference level/ratio as described above. More preferably, the respective increased level/ratio may be increased by 2- to 15-fold, 3- to 11-fold or 4- to 10-fold. Further, in the context of the screening methods of the invention, the increased level of N1aSPD, the N1aSPD concentration, and/or the increased ratio of the levels of N1aSPD/N1aSPM, may be increased by $\geq$10%, $\geq$20%, $\geq$30%, $\geq$40%, $\geq$50%, $\geq$60%, $\geq$70%, $\geq$80%, $\geq$90% or $\geq$100% as compared to a respective control level/ratio; for example like a control level/ratio or typical/normal reference level/ratio as described above.

In general, for the purposes of the screening methods of the invention, the specific value of an increased level of N1aSPD, of an N1aSPD concentration, or of an increased ratio of the levels of N1aSPD/N1aSPM, is not particularly crucial/decisive. Most relevantly, the respective levels/concentrations/ratios are envisaged to be so that the NavC-blocking efficacy of a NavCB (like, for example, CBZ) which would normally block NavC-function/activity (for example in pharmacoresponsive patients, or in respective (control) models described herein; and/or in the absence of N1aSPD; and/or at typical/normal reference levels/ratios) would significantly be reduced; i.e. reduced so that a difference in $Na_VC$-function/activity blocking is detectable. Based on the teaching provided herein and in the appended examples, the skilled person is readily able to establish suitable values/ranges for an increased level of N1aSPD, for an N1aSPD concentration, and/or for an increased ratio of the levels of N1aSPD/N1aSPM, on the basis of which the screening methods can be performed (i.e. discrimination between positive and negative hits is possible). This can be done for any particular screening method, respective test system or screening apparatus etc. For example, a minimal threshold, and also an optimal range, of workable increased N1aSPD levels, workable N1aSPD concentrations, or workable increased ratios of levels of N1aSPD/N1aSPM, can readily be established by the skilled person for a given screening method/test system/screening apparatus. In this context, the skilled person may rely on a less-sensitive NavCB (for example OXC) and/or a sensitive NavCB (for example CBZ). Once a significant difference in the N1aSPD-driven inhibition of the NavC-block occurs between such NavCBs, for example during an increase of N1aSPD concentrations, the respective N1aSPD concentration range (above the respective threshold), and/or the respective N1aSPD/ N1aSPM ratio range (above the respective threshold), can be used for screening for other NavCBs in accordance with the invention. A particular optimal (increased) N1aSPD concentration/level; and/or a particular optimal (increased) ratio of the levels of N1aSPD/N1aSPM, may also be established; for example on the basis of a situation where the difference in the N1aSPD-driven inhibition of the NavC-block occurs between such a less-sensitive NavCB (for example OXC) and such a sensitive NavCB (for example CBZ) is maximal. On the basis of the above, especially new screening methods/test systems/apparatuses in accordance with the invention can be established rapidly and easily, and, if necessary, optimized.

In addition to that, suitable examples of particular values and ranges of values for increased levels of N1aSPD, for N1aSPD concentrations, and for increased ratios of the levels of N1aSPD/N1aSPM, are provided herein. On the basis of these values/ranges, the screening methods of the invention can conveniently be established and performed. Examples of particular values and ranges of values for increased levels of N1aSPD, and for increased ratios of the levels of N1aSPD/N1aSPM, respectively, are given below. The screening methods of the invention may be performed by contacting a test compound with a functional NavC in the presence of a respective N1aSPD concentration, and/or in the presence of a respective ratio of the concentrations of N1aSPD/N1aSPM.

Examples of particular values and ranges of values for increased levels of N1aSPD, and for present respective N1aSPD concentrations, are $\geq$0.5 $\mu$M, $\geq$1 $\mu$M, $\geq$10 $\mu$M, $\geq$20 $\mu$M, $\geq$30 $\mu$M, $\geq$50 $\mu$M, $\geq$75 $\mu$M, $\geq$100 $\mu$M, $\geq$150 $\mu$M or $\geq$200 $\mu$M. Examples of more specific values/ranges are 50 $\mu$M to 2 mM, 75 $\mu$M to 1.5 mM, 100 $\mu$M to 1.5 mM, 100 $\mu$M to 1.4 mM, 100 $\mu$M to 1.3 mM or 100 $\mu$M to 1.2 mM; preferably 100 $\mu$M to 1.1 mM; more preferably 100 $\mu$M to 1 mM. Another particular range may be 0.5 nM-1 $\mu$M. Examples of particular values are (about) 80 $\mu$M, (about) 100 $\mu$M, (about) 200 $\mu$M, (about) 300 $\mu$M, (about) 400 $\mu$M, (about) 500 $\mu$M, (about) 600 $\mu$M, (about) 700 $\mu$M, (about) 800 $\mu$M, (about) 900 $\mu$M, (about) or (about) 1 mM. 75 $\mu$M to 1.5 mM or, more specifically, 100 $\mu$M to 1 mM may be two different ranges of the levels/concentration values on the basis of which the screening methods of the invention can be performed conveniently and with high reliability (in particular in *in vitro* or *ex vivo* approaches where, for example, patch-clamp or microelectrode techniques are used; and (a) respective device(s); cf. the appended examples for illustration). About 100 μM and/or about 1 mM may be two different levels/concentration values on the basis of which the screening methods of the invention can be performed conveniently and with high reliability (in particular in *in vitro* or *ex vivo* approaches where, for example, patch-clamp or microelectrode techniques are used; and (a) respective device(s); cf. the appended examples for illustration).

Examples of particular values and ranges of values for increased ratios of the levels of N1aSPD/N1aSPM, and for present respective ratios of the concentrations of N1aSPD/N1aSPM, respectively, are ≥0.4, ≥0.5, ≥0.6, ≥0.7, ≥0.8, ≥0.9 or ≥1.0; or >1.1, ≥1.2, ≥1.3, ≥1.4, ≥1.5, ≥1.6, ≥1.7, ≥1.8, ≥1.9 or ≥2.0.

Normal/reference levels, and control levels, respectively, of N1aSPD, and normal/reference ratios, and control ratios, respectively, of the levels of N1aSPD/N1aSPM, may be substantially below the above values/ranges (for example $\leq\frac{1}{2}$, $\leq\frac{1}{4}$, $\leq\frac{1}{8}$, $\leq\frac{1}{16}$ of the above values/ranges). Preferably, normal/reference levels/ratios, and control levels/ratios, respectively, are zero or close to zero (for example ≥0.5 μM, or even ≥0.5 nM for the N1aSPD levels; for example ≥0.4 for the N1aSPD/N1aSPM ratios). Especially in screening methods with an external setting/addition/control of the N1aSPD level, or of the ratios of the levels of N1aSPD/N1aSPM, (see Embodiment A and Example 7, below, for illustration), the control levels may be zero. The skilled person can readily take into account that, for other screening methods which make use of increased N1aSPD levels and/or increased N1aSPD/N1aSPM ratios based on, for example, genetically engineering (e.g. SMS knockdown; see the Embodiments B-E and Examples 8-11, below, for illustration), the normal/reference levels, and control levels, respectively, of N1aSPD, and normal/reference ratios, and control ratios, respectively, of the levels of N1aSPD/N1aSPM, may not be zero, but only at a low level/close to zero (for example in the respective cells, neurons, neuronal systems, cells of animal models; see also the Embodiments B-E and Examples 8-11, below, for illustration). The skilled person is readily in the position to establish the particulars and details of respective screening methods (for example so as to reach low normal/reference/control levels/ratios vs. increased experimental levels/ratios) so that the screening in accordance with the invention can successfully be performed (i.e. discrimination between positive and negative hits is possible).

In general, the skilled person is readily able to set, control, and, if desired to modify the increased levels of N1aSPD, the N1aSPD concentrations, and/or the increased ratios of the levels of N1aSPD/N1aSPM to/at suitable values or in suitable ranges (for example to/at values, or in ranges, as given above). In this context, the skilled person may rely on respective control devices (for example as disclosed herein), or on a modification of (an) enzyme(s) of the polyamine metabolism (so that an increased N1aSPD level or an increased ratio of the N1aSPD/N1aSPM levels is achieved; for example by SMS knockdown). In the context of a screening method of the invention which is based on a ratio of the levels of N1aSPD/N1aSPM, or on several different respective ratios, also the level of N1aSPM may be set, controlled and/or modified.

The technical meaning of "voltage-gated sodium (Nav) channel (NavC)", including its structure and biological function/activity, is well known in the art (see, for example, Männikkö, Handb Clin Neurol 203, 2024, 1-23 (doi: 10.1016/B978-0-323-90820-7.00009-4); Beckonert *loc. cit.;* Remy 2003 *loc. cit.;* Uebachs *loc. cit.;* Ellerkmann *loc. cit.;* Tham *loc. cit.,* Doeser, Brain 138(2), 371-87; https://en.wikipedia.org/wiki/Voltage-gated_sodium_channel). "NavC" is used accordingly herein and in the context of the invention. NavCs are sometimes also abbreviated as VGSCs or termed voltage-dependent sodium channels (VDSCs). If not explicitly indicated differently herein elsewhere, a "Na$_V$C" is also meant, if terms like "voltage-gated Na$^+$ channel", "Na$^+$ channel", or just "sodium channel" or "natrium channel" are used herein and in the context of the invention. Further, when reference to "NavC", "Na$^+$ channel", "sodium channel", "natrium channel" etc. is made herein, a functional NavC is meant. The core functional unit of a Na$_V$C is the voltage-sensitive α-subunit. A Na$_V$C may further be associated with accessory proteins, such as beta subunits. As mentioned, however, the α-subunit forms the core of a functional NavC and is functional on its own. When the α-subunit is expressed, for example in a cell, it is able to form a pore in the cell's membrane that conducts Na$^+$ in a voltage-dependent manner into the cell; even if beta subunits or other modulating proteins are not expressed. "Na$_V$C", and "functional Na$_V$C", respectively, in accordance with the invention thus means that at least the α-subunit is comprised. As mentioned, if reference is made herein to "NavC", a functional Na$_V$C is meant (if not explicitly indicated differently or contradicted by context). Typically, a Na$_V$C is capable of/has the (biological) function/activity of

(i) conducting sodium ions (Na$^+$) through a phospholipid bilayer membrane (for example through a cellular membrane);

(ii) displaying voltage-dependent activation and inactivation of Na$^+$ currents conducted over a phospholipid bilayer membrane (for example over a cellular membrane); the activation and inactivation is typically fast; fast in this respect means within (about) ≤ 15 ms; and/or

(iii) displaying recovery from inactivation of inactivated Na$^+$ currents over a phospholipid bilayer membrane (for example over a cellular membrane); the recovery is typically fast; fast in this respect means that most of the recovery process is completed within ≤ 20 ms; and/or

(iv) exhibiting other voltage- and time-dependent properties; for example voltage- and time-dependent properties at slower time scales; slow or slower in this respect means within >15 ms (for example ≥20 ms, ≥30 ms, ≥40; sometimes up to minutes). Examples of such voltage- and time-dependent properties are slow inactivation processes.

Any, several or all of these functions/activities may be assessed (directly (e.g. via patch-clamping) or indirectly (e.g. via *in vivo tests* on animal models)) in the context of the screening methods of the invention (for example by respective *(in vitro* or *ex vivo)* test devices or by respective *(in vivo)* tests/models; see below for further details). Respective tests, test devices, assays and models are known in the art and are also described herein and in the appended examples. Further, on this basis, a test compound/NavCB may be identified as the NavCB in accordance with the invention.

A $Na_VC$ is located in a phospholipid bilayer membrane and constitutes a transmembrane protein, respectively. Typically, a $Na_VC$ is located in the (outer) cell membrane of a cell, especially of a neuronal cell/neuron. A $Na_VC$ may also be located in the (outer) cell membrane of a large variety of non-neuronal excitable cells (for example glia, also called glial cells (astrocytes) or neuroglia). Several $Na_VCs$ are known in the art. These are all included, when reference to a NavC is made herein. Non-limiting examples of NavCs are the $Na_V1.1$ channel, $Na_V1.2$ channel, $Na_V1.3$ channel, $Na_V1.4$ channel, $Na_V1.5$ channel, $Na_V1.6$ channel, $Na_V1.7$ channel, $Na_V1.8$ channel or $Na_V1.9$ channel. Respective human versions are preferred (respective UniProt database entries are: P35498 ($Na_V1.1$ channel), Q99250 ($Na_V1.2$ channel), Q9NY46 ($Na_V1.3$ channel), P35499 ($Na_V1.4$ channel), Q14524 ($Na_V1.5$ channel), Q9UQD0 ($Na_V1.6$ channel), Q01118 ($Na_V1.7$ channel), Q9Y5Y9 ($Na_V1.8$ channel)). For example, the $Na_V1.2$ channel and/or the $Na_V1.6$ channel is/are of particular relevance when ASMs are to be screened in accordance with the invention. For example, the $Na_V1.7$ and/or $Na_V1.8$ channels is/are of particular relevance when medicaments for pain/pain-related diseases/disorders are to be screened in accordance with the invention. In principle, when reference to a particular NavC is made herein, the respective wild-type NavC is meant. However, NavCs in accordance with the invention generally also include those $Na_VCs$ which harbour one or more mutations (as compared to the respective wild-type $Na_VCs$); for example one or more mutations which are relevant/causative in (a) (human or animal) disease(s) (see, for example, Comini, Handb Clin Neurol, 2024, 89-109; Yamakawa, Sodium Channelopathies in Human and Animal Models of Epilepsy and Neurodevelopmental Disorders, In: Jasper's Basic Mechanisms of the Epilepsies. 5th ed., New York: Oxford University Press, 2024, Chapter 44). The screening methods of the invention may be adapted to these particular mutated NavCs. Such methods would then be particularly useful for the screening for respective NavCBs. These NavCBs may then be used in the treatment of the respective NHDs, in particular the respective PRNHDs.

The technical meaning of "NavC blocker (NavCB)" is well known in the art (see, for example, Perucca, Epilepsia 65, 2024, 533-41; Pal, Bioorg Chem 115, 2021, 105230). If not explicitly indicated differently or contradicted by context, "NavCB" is used accordingly herein and in the context of the invention. Typically, $Na_VCBs$ suppress abnormal seizure-associated neuronal activity through a voltage- and use-dependent inhibition of $Na^+$ currents . In particular, the use-dependent block by a $Na_VCB$ causes a reduction in $Na_VC$ availability during high-frequency discharges, as occur in, for example, epileptic seizures. Without being bound by theory, a main mechanism in this respect is the inhibition of the recovery from inactivation of $Na^+$ currents. In general, NavCBs in accordance with the invention encompass compounds which block NavCs. "Blocker" and "blocking" is used in this respect in the same sense as "inhibitor" and "inhibiting", respectively. This means that the function(s)/activity(ies) of a NavC is either blocked completely, or is inhibited to a certain degree (i.e. is reduced relative to the un-blocked/un-inhibited function(s)/activity(ies); as in most cases). The function/activity of a NavC may, for example, be inhibited/reduced by ≥5%, ≥10%, ≥20%, ≥30%, ≥40%, ≥50%, ≥60%, ≥70%, ≥80% or ≥90%, ≥95% (relative to the un-blocked/un-inhibited function(s)/activity(ies)).

NavCBs include medicaments which can be used in the treatment of (pharmacosensitive or pharmacoresistant) NHDs. These may be medicaments which are already used in the field in the treatment of (for example pharmacosensitive) NHDs. Compounds and potential medicaments which will be screened and identified in accordance with the invention (but which might not have been known as medicaments for NHDs) are also encompassed when reference to NavCB/NavCBs is made herein. In principle, ASMs, AEMs, AEDs, ACDs, and also hyperexcitability suppressors and pain suppressors etc., are encompassed when reference to a NavCB/NavCBs is made herein; as long as these medicaments/drugs/suppressors block NavCs (and, as such, may be suitable in treating, for example, NHDs). These medicaments/drugs may have additional functions/effects, like, for example, functions/effects which are not related to seizure symptoms (seizure-unrelated functions/effects; for example curative effects targeting the cause of a disease, e.g. epilepsy), and/or blocking effects on other channels (e.g. potassium channels).

In principle, a $Na_VCB$ in accordance with the invention is meant to block any, several or all of the (biological) functions/activities of a $Na_VC$. Most relevantly, however, the core (biological) function/activity of a $Na_VC$ is meant to be blocked by a $Na_VCB$. This is the voltage-gated $Na^+$ flux through the $Na_VC$ (typically from the outside of a cell (e.g. neuron) to the inside). The most relevant mechanism how a NavCB function is blocked (pertaining to use-dependent block) is a slowdown of the recovery from a $Na_VC$'s inactivation state. This state follows the open state of the $Na_VC$ and the inward $Na^+$ flux, respectively.

The meaning of "neuronal hyperexcitability disease/disorder (NHD)" is clear to the person skilled in the art. If not explicitly indicated in a different manner or contradicted by context, "NHD" is used accordingly herein and in the context of the invention. In general, NHDs in accordance with the invention refer to diseases/disorders which relate to/come along with neuronal hyperexcitability. In the context of NHDs, the neuronal hyperexcitability is due to (at least partially) the function/activity of NavCs. As such, the NHDs may be treated with NavCBs. In principle, not all NHDs are NavCHDs, i.e. not all NHDs relate to/come along with a hyperactivity of $Na_V$Cs (see also below for details). The most relevant diseases and disorders in the context of the present invention are, however, NavCHDs.

The meaning of "$Na_V$ channel-hyperactivity disease/disorder ($Na_V$CHD)" is also clear to the person skilled in the art (see, for example, Männikkö *loc. cit.*). If not explicitly indicated in a different manner or contradicted by context, "NavCHD" is used accordingly herein and in the context of the invention. In general, NavCHDs in accordance with the invention refer to diseases/disorders which relate to/come along with a hyperactivity of NavCs.

The meaning of NHDs and NavCHDs in accordance with the invention also encompasses the respective symptoms (for example seizures (in case of, for example, epilepsy), pain (in case of, for example, pain/pain-related diseases/disorders), or hyperexcitability (in case of, for example, neurodegenerative diseases/disorders)). NHDs/NavCHDs in accordance with the invention encompass seizure disorders (i.e. disorders which relate to/come along with seizures resulting from $Na_V$C-hyperactivity), as well as hyperexcitability disorders (i.e. disorders which relate to/come along with other symptoms resulting from NavC-(hyper)activity, like hyperexcitability, pain etc.). Typical seizure disorders are epilepsies; for example chronic epilepsies (like, for example, chronic temporal lobe epilepsy (cTLE)), refractory epilepsies, acquired epilepsies, genetic epilepsies (like, for example, epilepsies harbouring a gain-of-function (GOF) missense mutation, e.g. p.!335N in KCNT1 (KCNT1 p.I335N)). Some neurodegenerative or neurological disorder, such as a pain disorders/pain-related disorders (e.g. neuropathic pain; hereditary pain syndromes etc.), trigeminal neuralgia, postherpetic neuralgia, diabetic neuropathy, Alzheimers disease (AD) or stroke, may also be subsumed under hyperexcitability diseases/disorders. When reference to NHDs/NavCHDs is made herein, such diseases/disorders are also meant (provided that they relate to/come along with/result from the activity or hyperactivity of NavCs).

In principle, when reference is made herein to "pharmacoresistant NHD (PRNHD)" or "pharmacoresistant NavCHD (PRNavCHD)", a pharmacoresistant form of an NHD or NavCHD, respectively, is meant; in particular a pharmacoresistant form of an NHD or NavCHD as disclosed herein (see, for example, above). Particular pharmacoresistant forms of NHDs/NavCHDs are known in the art (cf., for example, Perucca, 2007 *loc. cit.*; Perucca, 2020, *loc. cit.*; Golyala *loc. cit.;* Chen *loc. cit.*). In general, pharmacoresistance and pharmacoresistant, respectively, means that a NHD or $Na_V$CHD patient does not respond, or does not fully respond, to a respective medication (with a NavCB). On molecular level, pharmacoresistance and pharmacoresistant, respectively, means that $Na_V$C activity/function, in particular a $Na_V$C hyperactivity is not, or only partially, blocked by a $Na_V$CB. In other words, the $Na_V$CB is ineffective in this respect; or its effect is reduced. Predominant PRNHDs/PRNa$_V$CHDs in accordance with the invention are PREs. PRSs (sometimes also termed herein DRSs) are also meant in this context. Other PRNHDs/PRNavCHDs, like other pharmacoresistant seizure disorders (PRSDs) and pharmacoresistant hyperexcitability disorders (PRHDs), are also within the scope of the invention. Non-limiting examples of such other PRNHDs/PRNavCHDs in accordance with the invention are pharmacoresistant neurological disorders (see, for example, Tham *loc. cit.*; Fischer *loc. cit.*). For example, substantial fractions of patients with pain disorders, like trigeminal neuralgia, postherpetic neuralgia, diabetic neuropathy, erythromelalgia, fibromyalgia, and other forms of neuropathic pain and hereditary pain syndromes, are also unresponsive to the treatment with sodium channel blockers and are pharmacoresistant in accordance with the invention (see, for example, Tham *loc. cit.;* Fischer *loc. cit.).* In a PRNHDs/PRNavCHD in accordance with the invention, the pharmacoresistancy is due to an increased level of N1aSPD; it may also be due to a decreased level of N1aSPM and/or an increased ratio of the levels of N1aSPD/N1aSPM; for example in cells, in particular neuronal cells, of a respective patient. This leads to the respective lack/reduction of the efficacy of (a) NavCB(s).

In the context of the methods of screening of the invention, it is particularly preferred that the increased level of N1aSPD, increased ratio of the levels of N1aSPD/N1aSPM and/or decreased level of N1aSPM is present at the intracellular side of the functional Nav channel. For example, the presence may be in the lumen of a cell (intracellularly) which may be used in the context of the methods of screening of the invention (for example as a cell model in a neuronal network system model or in an animal model as described herein).

A method of screening of the invention may be an *in vitro, ex vivo* or an *in vivo* method. In principle, a method of screening of the invention may also be performed in an artificial mode; for example, by relying on an artificial phospholipid bilayer membrane with (a) NavC(s) located therein as (a) transmembrane protein(s). For example, in the context of *in vitro* or *ex vivo* methods of screening of the invention, a cell or a neuronal culture system, or a cell model or a neuronal culture system model, may be used (for example as described herein). In the context of *in vivo* methods of screening of the invention, an animal or an animal model may also be used (for example as described herein). In the context of *in vitro* or *ex vivo* methods of screening of the invention, the respective step(s) (e.g. contacting, controlling and/or testing) may be practiced on a cell/cell model or on a neuronal culture system/neuronal culture system model. In the context of *in vivo* methods of screening of the invention, the respective step(s) (e.g. contacting, controlling and/or testing) may be practiced on an animal/animal model.

A method of screening in accordance with the invention may comprise one, two or several particular steps. (A) respective step(s) may be (a) step(s) of contacting, controlling and/or testing (as, for example, further described herein). (A) respective device(s) may be used in his respect; for example (a) contact, control and/or test device(s), respectively (as, for example, further described herein).

Most relevantly, a step of contacting a test compound with a functional NavC in the presence of an increased level of N1aSPD, an increased ratio of the levels of N1aSPD/N1aSPM and/or a decreased level of N1aSPM is envisaged to be comprised in a method of screening of the invention. In the context of the methods of screening of the invention, it is particularly preferred that the test compound is contacted with the extracellular side of said functional NavC. For example, the compound may be contacted from the outside of a cell (extracellularly) which may be used in the context of the methods of screening of the invention (for example as a cell model described herein, or within a neuronal culture system described herein; or within an animal model described herein).

In the context of the methods of screening of the invention, and in the context of the (step of) contacting, respectively, the test compound may be contacted with a cell which expresses the functional $Na_V$ channel in its cell membrane. In the cell, the concentration/increased level of N1aSPD and/or the increased ratio of the levels of N1aSPD/N1aSPM, is/are envisaged to occur intracellularly. In an *in vitro* or *ex vivo* situation, this is also termed cell model for a PRNHD or a $PRNa_V$CHD in accordance with the invention. In principle, the cell may be any suitable cell in accordance with the invention. The cell may be an animal cell or, preferably, a human cell. The cell may be derived from an animal cell line or, preferably, from a human cell line. The cell may be a Human Embryonic Kidney (HEK) cell (e.g. a HEK293 cell, like a HEK293T, HEK293F, HEK293FT or HEK293-AAV cell), a Chinese Hamster Ovary (CHO) cell, a xenopus oocyte, or a cell of a neuroblastoma cell line. The cell may also be a neuron or a glia, also called a glial cell (gliocyte) or a neuroglia. In the context of a specific embodiment of the invention, the cell is a neuron, preferably a primary neuron. On the basis of its common general knowledge, the skilled person is readily able to establish and provide cells/cell models which are suitable in accordance with the invention. Respective guidance is also provided herein; for example, in the context of Embodiments A, B and D; and Examples 7, 8 and 9, respectively.

In the context of the methods of screening of the invention, and in the context of the (step of) contacting, respectively, the test compound may be contacted with a (human) neuronal culture system, preferably a human neuronal culture system (e.g. a (human) iPSC-derived neuronal culture system). The neuronal culture system is envisaged to comprise cells which express a functional NavC in their cell membranes. In (the cell(s) comprised in) the neuronal culture system, the concentration/increased level of N1aSPD and/or the increased ratio of the levels of N1aSPD/N1aSPM (and/or decreased level of N1aSPM), is/are envisaged to occur intracellularly. This is also termed neuronal culture system model for a PRNHD or a PRNavCHD in accordance with the invention. Means and methods for establishing and providing neuronal culture systems (e.g. (human) iPSC-derived neuronal culture systems) are well known in the art; and are, for example, described in Habibey (Front Neurosci. 16, 2022, 1-18) and Schoch (J Neurosci. 41(39), 2021 8111-25). Embodiment D and Example 10, respectively, provide further guidance in this respect.

In the context of the methods of screening of the invention, and in the context of the (step of) contacting, respectively, the test compound may be contacted with an animal. The animal is envisaged to comprise cells, in particular neuronal cells/neurons, which express a functional NavC in their cell membranes. In (the cell(s) comprised in) the animal, the concentration/increased level of N1aSPD and/or the increased ratio of the levels of N1aSPD/N1aSPM (and/or decreased level of N1aSPM), is/are envisaged to occur intracellularly. This is also termed animal model for a PRNHD or a PRNavCHD in accordance with the invention. On the basis of its common general knowledge, the skilled person is readily able to establish and provide animals/animal models which are suitable in accordance with the invention. Respective guidance is also provided herein; for example, in the context of Embodiment C, D and E, and Examples 9, 10 and 11, respectively.

(A) cell(s), neuronal culture system, animal to be used in accordance with the invention may be (a) recombinant (genetically engineered) cell(s), neuronal culture system, animal, respectively. In this context, the increased intracellular level of N1aSPD, decreased level of N1aSPM and/or increased intracellular ratio of the levels of N1aSPD/N1aSPM may be achieved by modifying the expression of at least one enzyme of the polyamine metabolism in the cell(s), neuronal culture system, animal, respectively; provided that this leads to an increased level of N1aSPD, decreased level of N1aSPM and/or increased ratio of the levels of N1aSPD/N1aSPM. In this respect, the expression of the spermine synthase (SMS) may be reduced in the cell(s), neuronal culture system, animal; for example, by recombinant techniques/techniques of genetic engineering. Other means and methods for reducing SMS function and modifying of (an)enzyme(s) of the polyamine metabolism are also within the scope of the invention. Other examples of modifications of (an)enzyme(s) of the polyamine metabolism are an increased expression/function of the spermine oxidase (SMO), or increased acetylation via SSAT.

An animal to be used in accordance with the invention may be an animal model of a PRNHD or a $PRNa_V$CHD (e.g. a model of a pharmacoresistant seizure disorder (PRSD)). The animal may be a model of epilepsy, in particular a model of PRE. For example, the animal may be a model of chronic epilepsy, chronic temporal lobe epilepsy (cTLE), refractory epilepsy, acquired epilepsy and/or genetic epilepsy. The animal model of genetic epilepsy may be a KCNT1 model of genetic epilepsy. Such an animal/animal model may harbour a gain-of-function (GOF) missense mutation; for example, the GOF missense mutation p.!335N in KCNT1 (KCNT1 p.I335N). A non-limiting example of an animal/animal model which may conveniently be used in accordance with the screening methods of the invention is a pilocarpine model of epilepsy (as, for example, described in Remy 2003 *loc. cit.;* Sanabria, *loc. cit.*). The animal/animal model also may be an SMS knockout animal/animal model, preferably a conditional SMS knockout animal/animal model. In principle, the animal/animal model may be any suitable animal. For example, a rat, a mouse, a rabbit, a guinea pig, a monkey may be employed in this respect. Preferably, a mouse or a rat is employed; for example, a pilocarpine rat or a pilocarpine mouse. Another animal/model example may be a mouse selected from the group consisting of C57BL/6J/Sms[em6Lutzy]/J (Strain #: 032064, The Jackson Laboratory), C57BL/6Smoc-[SMSem1(flox)Smoc] (Cat. No. NM-CKO-2117943, Shanghai Model Organisms) and C57BL/6JGpt-Sms-flox (T016254, GemPharmatech Co., Ltd.).

The skilled person in readily able to provide (a) recombinant and/or genetically engineered cell(s), neuronal culture system and animal in accordance with the invention. In particular, cell(s), neuronal culture system and animal with a reduced SMS expression/function, or with a modified (e.g. reduced) function of (an)other enzyme of the polyamine metabolism, can readily be provided. Respective guidance is also provided herein; see, for example, Embodiments B, C, D and F and Examples 8, 9, 10 and 11, herein below. Knockdown and reduced/inhibited function/activity encompasses both options, the complete loss of function/activity and a partial loss of function/activity (for example $\leq 90\%, \leq 80\%, \leq 70\%, \leq 60\%, \leq 50\%, \leq 40\%, \leq 30\%, \leq 20\%$, or $\leq 10\%$, of the initial/reference, non-reduced/inhibited function/activity). siRNA, RNAi or shRNA approaches may be applied in the context of providing recombinant and/or genetically engineered cell(s), neuronal culture system and animal in accordance with the invention. Other respective means and methods are antisense approaches. Other recombination methods and/or methods of genetically engineering may also be employed. Mutation technologies may also be employed in this respect, like the CRISPR-Cas technology (e.g. CRISPR/Cas9 technology). Techniques relying on sgRNAs or (lenti)viruses (e.g. expressing sgRNAs) may also be employed in this respect. Respective means and methods are, for example, described in Neggers (Nat Commun 9(1), 2018, 502) and Prommersberger (Current Protocols in Immunology, 128, 2020, e93). AAV vector technology may also be employed. Respective guidance is also provided in, for example, Embodiments B, C, D and F and Examples 8, 9, 10 and 11, herein below.

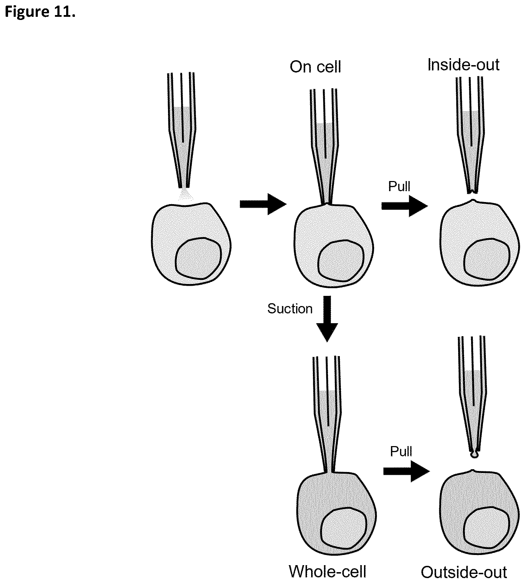

In the context of a screening method of the invention, voltage-clamping may be applied, and a voltage-clamp apparatus may be used, respectively. Voltage-clamping and voltage-clamp apparatuses are also termed patch-clamping and patch-clamp apparatuses herein. "Patch-clamp" as used herein is not limited to clamping techniques where a particular "patch" of a membrane is clamped (for example in the context of the cell-attached mode or inside out-mode; see below and Figure 11 for details). The term is meant to cover also other voltage-clamping techniques, like whole-cell voltage-clamping (see also below and Figure 11 for details). Voltage/patch-clamp apparatuses; and also typical parts thereof (like micropipettes, amplifiers, super fusion devices, bath electrodes, microelectrodes, etc.), are also termed patch-clamp devices herein. (A) patch-clamp device(s) may be used in the context of the (steps of) contacting, controlling and/or testing of a method of screening of the invention; for example, as the respective contact device(s), control device(s) and/or testing device(s). In general, patch-clamp techniques and respective patch-clamp apparatuses, as well as their modes of action and relevant parts, are well known in the art and are, for example, described in Patch Clamp Techniques (From Beginning to Advanced Protocols, 2012, Editor Yasunobu Okada, Springer Protocols Handbooks, Electronic ISSN 1949-2456 ). Typically, a patch-clamp device comprises (one or more of) the following parts: a micropipette (or a planar chip; or the like; the micropipette or planar chip (or the like) may comprise a microelectrode and/or an intracellular or extracellular solution), an amplifier, an analog-digital converter, a storage device, a chamber (also sometimes termed "vial" herein) comprising the bath solution (e.g. a superfusion device), and a bath reference electrode. These parts are also termed patch-clamp devices herein. Typical patch-clamp modes of action are the whole-cell mode, outside-out mode, cell-attached mode (also termed on-cell mode) and inside-out mode (see Figure 11 for illustration). The whole-cell mode is preferred in accordance with the invention. Depending on the particular patch-clamp mode, a particular patch-clamp device may be used as a contact, control and/or test device in the context of the screening methods of the invention. The skilled person is readily able to adapt a screening method to a particular patch-clamp mode applied.

For example, when a cell-attached patch-clamp mode or an inside-out patch-clamp mode is applied, a micropipette or a planar chip may be used as a contact device. When a whole-cell or outside-out patch-clamp mode is applied, a micropipette or planar chip may be used as a control device. For example, when a cell-attached, whole-cell or outside-out mode is applied, a vial (for example vial comprising the bath) may be used as a contact device. When an inside-out patch-clamp mode is applied, a vial may be used as a control device. In all these cases, a patch-clamp micropipette or a patch-clamp planar chip may be used as the testing device (for example the same micropipette or planar chip as the one used as the control/contact device).

In the context of the (step of) contacting, and by a respective contact device, a test compound is contacted with a functional $Na_VC$. Most preferably, this is done so that the test compound is contacted with the extracellular part of the NavC. In the context of the (step of) setting/controlling/modifying, and by the respective control device, the increased level of N1aSPD, the N1aSPD concentration, the decreased level of N1aSPM, and/or the ratio of the levels of N1aSPD/N1aSPM is set, controlled and/or, optionally, modified (most preferably at the intracellular part of th NavC (for example in the lumen of a cell). In the context of the (step of) testing, and by the respective testing device, a function/activity of the NavC is detected (for example, any, several or all of the herein described function(s); in particular, the discharge/inactivation of Na$^+$ current over a phospholipid bilayer membrane and/or the recovery from inactivation of Na$^+$ currents).

In the context of a screening method of the invention, a microelectrode or a microelectrode array (MEA), for example in form of a MEA plate, may be used. The microelectrode/MEA technique is well known in the art; and is typically used for measuring neuronal activity by assessing neuronal spiking (see, for example, Johnstone, Neurotoxicology 31, 2010, 331-50; Spira, Nature Nanotech 8, 2013, 83-94). Any other method/technique which is suitable for assessing neuronal spiking may also be used as an alternative. A non-limiting example of a MEA technique is the Maestro Edge System (Axion Biosystems). Respective MEA plates may be CytoView-MEA24 wellplates (Axion Biosystems). A microelectrode/MEA apparatus, and also typical parts thereof (like MEA plates etc.; see also Embodiment C, step 5 and Example 9 for illustration and further details) may be used in the context of the (steps of) contacting, controlling and/or testing of a method of screening of the invention; for example as the respective contact device(s), control device(s) and/or testing device(s). Typically, a microelectrode/MEA apparatus/device may comprise the following parts: i) a well or multi-well system (within which a cell system can be maintained in appropriate medium; for instance a neuronal culture); The well system may be equipped with a multielectrode grid suitable for sensing the activity of juxtaposed neurons; ii) a device for exchanging the solution within the wells; iii) an amplifying system for amplifying and digitizing the signals obtained from the multielectrode contacts; and iv) an analysis suite capable of storing and analyzing the data (usually large amounts of data).

In the context of the method of screening of the invention, the concentration/increased level of N1aSPD and/or the increased ratio of the levels of N1aSPD/N1aSPM (and/or decreased level of N1aSPM) may be set, controlled and/or modified by a control device. Most preferably, this is done at the intracellular side of the functional NavC. For example, the concentration/increased level of N1aSPD and/or the increased ratio of the levels of N1aSPD/N1aSPM (and/or decreased level of N1aSPM) is set/controlled/modified by the control device in a cell/cell model.

In the context of the method of screening of the invention, in particular in the context of the (step of) contacting the test compound with the functional NavC, a contact device may be used. Most preferably, the test compound is contacted with the extracellular side of the functional Na$_V$C; for example by using said contact device, for example from the outside of a cell/cell model.

The control or contact device may be or may comprise a micropipette, a planar chip or a vial. The vial may be a super fusion device (which may comprise a patch-clamp bath).The control or contact device may be or may comprise a patch-clamp device, for example a planar patch-clamp device (like a patch-clamp chip), a patch-clamp micropipette, or a patch-clamp vial (e.g. patch-clamp super fusion device). A control device may be or may comprise a whole-cell patch-clamp device, an outside-out patch-clamp device, or an inside-out patch-clamp device. In the context of the (step of) contacting the test compound with the functional NavC, a vial of a microelectrode or of a microelectrode array (MEA) plate may be used in/as the contact device. A vial may also be used in/as the control device for setting/controlling/modifying the concentration/increased level of N1aSPD and/or the increased ratio of the levels of N1aSPD/N1aSPM (and/or decreased level of N1aSPM) at the intracellular side of the functional Na$_V$C. The vial may also be a super fusion device or a vial of a microelectrode/MEA plate). When the (step of) contacting a setting, controlling and/or modifying is applied to a cell, the cell may be placed in a vial as the respective contact or control device (e.g. in the bath of a super fusion device), or a micropipette a planar chip may be used accordingly as the contact or control device. The contact and control device then comprises the test compound and intracellular solution, respectively. When the (step of) contacting is applied to an animal, the test compound may be administered to an animal; for example by a common route of administration of NavCBs (e.g. orally, IV, SC etc.). Further, when an animal is used, the (step of) setting/controlling/modifying may be done by respective genetic engineering techniques, like, for example, by conditionally or unconditionally modifying the expression/function of (an) enzyme of the polyamine metabolism (e.g. SMS).

The method of screening of the invention may comprise (a step of) testing whether, upon the (step of) contacting the test compound with the functional NavC, the test compound inhibits/blocks (said activity of) the functional NavC in the presence of the concentration/increased level of N1aSPD and/or the increased ratio of the levels of N1aSPD/N1aSPM. A test device may be used in this respect; for example a test device as described herein. The (step of) testing may be practiced on a cell (for example as described herein). The cell is envisaged to express a functional NavC and to contain the concentration/increased level of N1aSPD and/or the increased ratio of the levels of N1aSPD/N1aSPM.

The test device may comprise a voltage-clamp device or a patch-clamp device. The test device may comprise a planar voltage/patch-clamp device (like a voltage/patch-clamp chip) or a voltage/patch-clamp micropipette. Especially in the (semi-)automated or high-throughput mode, the test device may be or may comprise a multichannel voltage/patch-clamp device and/or a semi-automated, automated or high-throughput voltage/patch-clamp device. A non-limiting example of such a device, and of a planar voltage/patch-clamp device (like a voltage/patch-clamp chip), is the Sophion Bioscience Qube system, e.g. QPatch Compact, QPatch or Qube 384, and the like.

Also the test device may be or may comprise a whole-cell patch-clamp device, an outside-out patch-clamp device, a cell-attached patch-clamp device or an inside-out patch-clamp device. As mentioned, a (patch-clamp) contact and/or control, device may also be used as a (patch-clamp) test device, depending on the particular (patch-clamp) mode action. By use of the test device, voltage-clamp recordings can be performed. The activity/function of the NavC may so be assessed.

As mentioned, also the test device may be or may comprise a microelectrode or a microelectrode array (MEA), and/or (an) other device(s) of a microelectrode apparatus, like a MEA plate. Such testing devices are particularly useful when the NavC function/activity is tested in neurons or neuronal culture systems (and respective models), and the neuronal activity may be measured accordingly (by assessing neuronal spiking).

The method of screening of the invention may comprise (the step of) testing in an animal whether, upon the (step of) contacting the test compound with said functional NavC, the test compound inhibits/blocks (the activity of) the functional NavC in the presence of the concentration/increased level of N1aSPD and/or the increased ratio of the levels of N1aSPD/N1aSPM. The animal may be an animal as described herein or an animal of model as described herein. In this respect, respective tests are well known in the art and are, for example, described in Wilcox, (Neuropharmacology 166, 2020, 1-20) and Guignet (In: Noebels, Jasper's Basic Mechanisms of the Epilepsies. 5th ed., New York: Oxford University Press; 2024. Chapter 66). Particular examples of such tests, and the respective model(s) and device(s) are:

(a) an electrical stimulation (e.g. at 6 Hz);
(b) the use of an electroshock model (like, for example, a maximal electroshock model);
(c) the use of a kindling model (like, for example, a corneal kindling model) and/or kindling testing (like, for example, corneal kindling testing); and/or
(d) the use of a spontaneous bursting slice model from (a) post-kainic acid model(s).

The $Na_VCB$ may be a NHD medication or a $Na_VCHD$ medication. The NHD/$Na_VCHD$ medication may be an antiseizure medication (ASM), an antiepilepsy medication (AEM; sometimes also termed antiepileptic drug (AED)) or a medication for a hyperexcitability disorder. The hyperexcitability disorder may be a neurodegenerative or neurological disorder, such as a pain disorder/pain-related disorder (e.g neuropathic pain; hereditary pain syndromes etc.), trigeminal neuralgia, postherpetic neuralgia, diabetic neuropathy, Alzheimers disease (AD) or stroke. The PRNHD or PRNavCHD as referred to herein, in particular in accordance with the screening method of the second aspect of the invention, may be a pharmacoresistant seizure disorder (PRSD) or a pharmacoresistant hyperexcitability disorder (PRHD). The PRSD may be a pharmacoresistant epilepsy (PRE). The PRHD may be a be a pharmacoresistant form of a hyperexcitability disorder, for example of a hyperexcitability disorder as defined herein.

In principle, the method of screening of the invention may be performed in any suitable mode, e.g. in a high reliability manual mode or in a semi-automated, automated or high-throughput mode. In one particular aspect, the method of the invention may be a high-throughput method of screening and may be operated in a high-throughput mode, respective. In another particular aspect, the method of the invention may be a high-reliability (low capacity) method of screening. Both of these modes may also be combined. This advantageously results in a method of screening with high capacity and high reliability, and as such, a highly efficient method for screening and developing effective NavCBs, in particular NavCBs to be used in the treatment of PRNHDs/PRNavCHDs.

In a third aspect, the present invention thus also relates to two-step method of screening for an NavCB comprising

(i) (a first step of) operating a method of screening for an NavCB in accordance with the invention (for example *in vitro* or *ex vivo* method and/or by using a cell or neuronal culture system model);
(ii) (a second step of) operating a method of (confirmatory) testing whether the NavCB as identified according to (i) inhibits/blocks (the activity of) a functional Nav channel in the presence of said increased level of N1aSPD according to a method of screening of the invention (for example in an *in vivo* method of screening and/or by using an animal model).

In the context of the two-step method of screening, (i) may be operated in a semi-automated, automated or high-throughput mode; and/or (ii) may be operated in a high-reliability (low capacity) mode. Further, (i) may be operated in an (automated) patch-clamp- or MEA-based mode (e.g. as defined herein elsewhere); and/or (ii) may be based on the use of an animal model (e.g. as defined herein elsewhere). What is said herein elsewhere with respect to the methods of screening of the other aspects of the invention, and with respect to the respective particulars and details, also applies here, *mutatis mutandis.*

In a fourth aspect, the present invention relates to an NavCB as screened/identified according to a method of screening according to the invention. In a fifth aspect, the present invention relates to a pharmaceutical composition comprising the NavCB as screened/indicated according to a method of screening of the invention. The pharmaceutical composition may be for use in treating an NHD, like a NavCHD; in particular a PRNHD/PRNavCHD. What has been said herein elsewhere with respect to the screening methods of the invention, the NavCB and/or the (PR)NHD and (PR)Na$_V$CHD also applies here, *mutatis mutandis* (if not contradicted by context). In particular, the NHD or PRNHD, or Na$_V$CHD or PRNa$_V$CHD, may be as defined herein elsewhere. For example, it may be a seizure disorder, like an epilepsy. The pharmaceutical composition may also be for use in treating a seizure, like a seizure in/coming along with epilepsy. The epilepsy may be defined as herein elsewhere. In particular, the pharmaceutical composition may be for use in treating an NHD, like a PRNHD or a PRNavCHD. The PRNHD or PRNavCHD may be a pharmacoresistant form of disease or disorder as defined here in elsewhere. In a sixth aspect, the present invention relates to a NavCB which inhibits/blocks (the activity of) a functional NavC in the presence of an increased level of N1aSPD; and/or which inhibits/blocks (the activity of) a functional Na$_V$ channel in the presence of an increased ratio of the levels of N1aSPD/N1aSPM (and/or in the presence of decreased N1aSPM level). In a seventh aspect, the present invention relates to a pharmaceutical composition comprising such a NavCB. The NavCB of the sixth aspect of the invention or the pharmaceutical composition of the seventh aspect of the invention may also be for use in treating an NHD, like a NavCHD, in particular a PRNHD/PRNavCHD.

In an eighth aspect, the present invention relates to a pharmaceutical composition comprising OXC and/or Eslicarbazepine/Eslicarbazepine acetate (ESL) for use in treating a PRNHD, in particular a PRNavCHD. What is said herein elsewhere with respect to the NavCB, NavC, levels of N1aSPD and/or N1aSPM and/or the respective ratios, NHD, PRNHD, NavCHD and PRNavCHD etc. also applies here, *mutatis mutandis;* if not contradicted by context.

In the context of the pharmaceutical composition of the invention, or of the medical use of the Na$_V$CBs which are screened/identified in accordance with the invention, further/other medicaments may be applied; for example, as a comedication together with the pharmaceutical composition or the NavCBs. For example, medicaments which increase the intercellular level of Na1SPM in neurons may be used in this respect. Agonists of SMS may be used in this respect. In a ninth aspect, the present invention relates to a device which is suitable for and adapted to the methods of screening of the invention. What is said herein elsewhere with respect to the methods of screening of the invention and/or the respective device(s) (e.g. control device(s), contact device(s), test device(s)) applies also here, *mutatis mutandis.* The device may be any respective patch-clamp apparatus/device, or any respective part thereof. Examples are a (patch-clamp) micropipette or a planar (patch-clamp) chip. The device may also be any respective microelectrode/MEA apparatus/device, or any respective part thereof. A non-limiting example is a MEA plate. The device may also be a vial as described herein (for example a super fusion device). Most relevantly, the device of the invention comprises a solution (also termed intracellular solution herein) which comprises the increased N1aSPD level, increased ratio of N1aSPD/N1aSPM levels, and/or decreased N1aSPM level in accordance with the invention. The device thus may be a control device as described herein. Any apparatus or device which comprises such a control device is within the scope of the invention. Respective apparatus'/devices may be patch-clamp apparatus'/devices or microelectrode/MEA apparatus'/devices.

In a tenth aspect, the present invention relates to a kit/kit of contends comprising a solution (also termed intracellular solution herein) which comprises the increased N1aSPD level, increased ratio of N1aSPD/N1aSPM levels, and/or decreased N1aSPM level in accordance with the invention. The kit may further comprise a device as described herein, for example a control and/or test device, and/or contact device. The device may contain the intracellular solution. A kit in accordance with the invention may (further) comprise (a) cell(s)/cell model(s) as described herein, one or more test compounds, and/or one or more control NavCBs (e.g. CBZ and/or OXC). What is said herein elsewhere with respect to the levels of N1aSPD and/or N1aSPM, and/or the respective ratios, and with respect to the device(s), also applies here, *mutatis mutandis;* if not contradicted by context.

In an eleventh aspect, the present invention relates to a method for designing and/or developing a NavCB, in particular a NavCB which is pharmacologically effective in the treatment of a PRNHD, in particular of a PRNavCHD. In the context of this method, a screening method of the invention may be performed; and/or a device, and/or a kit of the invention may be used. What is said in this respect herein elsewhere also applies to this eleventh aspect, *mutatis mutandis;* if not contradicted by context. In the context of this method, compound libraries of test compounds may be generated (for example of test compounds which are structurally more closely related to OXC than CBZ), and the libraries may then be screened for Na$_V$CBs in accordance with the invention by applying a screening method of the invention. On this basis, effective Na$_V$CB candidates can systematically be designed and developed.

In a further aspect, the present invention relates to a cell, a neuronal culture system or an animal as described herein; and to a cell model, a neuronal culture system model or an animal model as described herein. In this cell/cell model, neuronal culture system/neuronal culture system model and animal/animal model, the level of N1aSPD (and/or the ratio of the levels of N1aSPD/N1aSPM) is increased in accordance with the invention, and/or the level of N1aSPM is decreased in accordance with the invention. In one embodiment of this further aspect of the invention, the cell/cell model, neuronal culture system/neuronal culture system model and animal/animal model is genetically engineered as described herein; for example so that the SMS activity is inhibited.

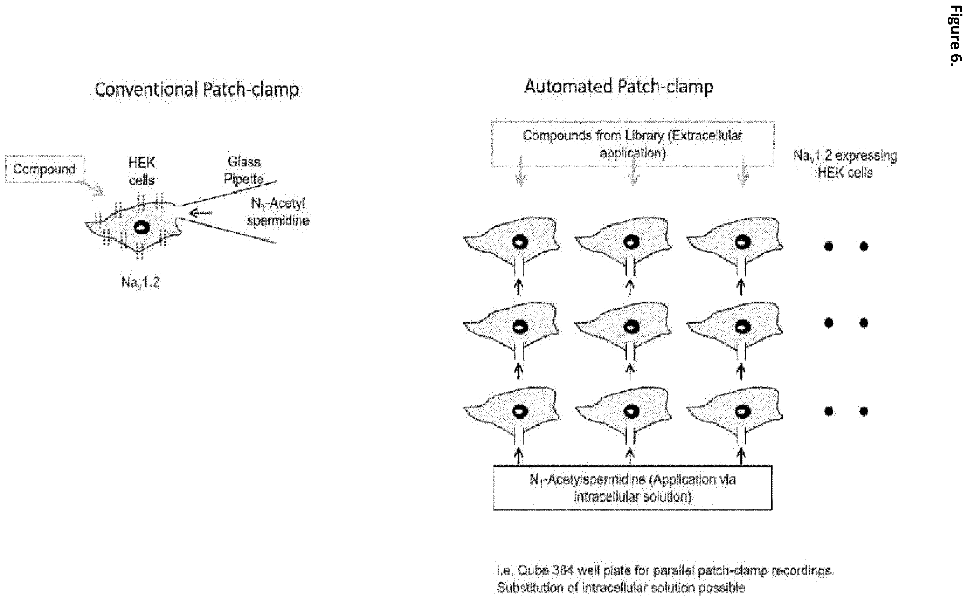

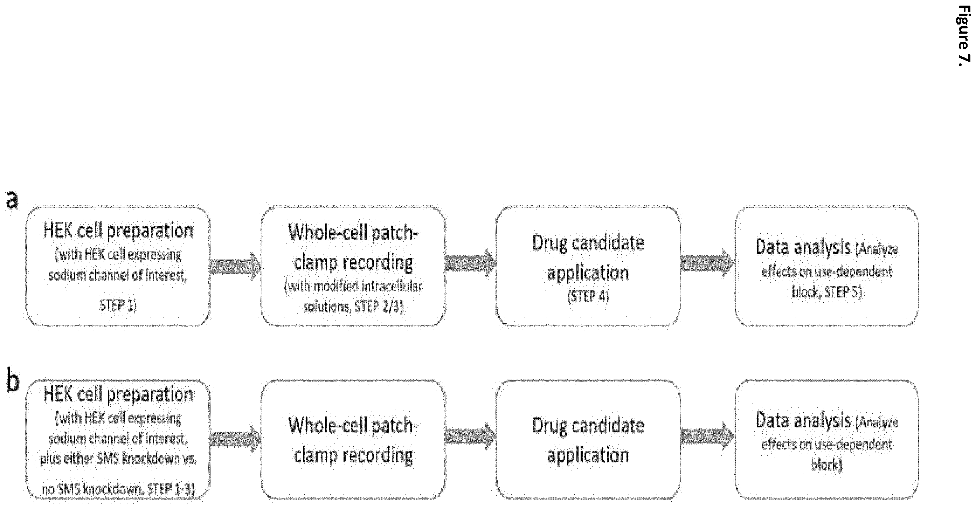

In the following, five non-limiting embodiments of the screening methods of the invention are described (Embodiment A-E, below). The presence of N1aSPD, and of an increased level of N1aSPD, respectively, (and/or of an increased ratio of the levels of N1aSPD/N1aSPM) is reflected in all those embodiments. If not indicated differently or contradicted by context, any, several or all steps (preferably in the described order), and/or any, several or all details, which are described in the context of these embodiments may also be performed in the context of, and/or applied to, the screening methods as described herein above and in the claims. *Vice versa,* if not indicated differently or contradicted by context, any, several or all steps (preferably in the described order), and/or any, several or all details, which are described in the context of the screening methods as described herein above and, in the claims, may also be performed in the context of, and/or applied to, the screening methods as described in these embodiments. Embodiment A relates to a method of screening for a NavCB, wherein the levels of N1aSPD (and/or N1aSPM), in particular the increased level of N1aSPD (and/or the increased ratio of the levels of N1aSPD/N1aSPM), is set, controlled and/or modified intercellularly by a control device. Such a method is also termed (interaction-based) screening with a modified intracellular solution. A scheme of a stepwise protocol for a respective exemplary HEK cell-based screening method is shown in **Fig. 7a.** Illustrative schemes of exemplary settings for such HEK cell-based screening methods in the whole-cell voltage-clamp mode is shown in **Fig. 6.** A non-limiting example of such a method is also described in detail in appended Example 7.

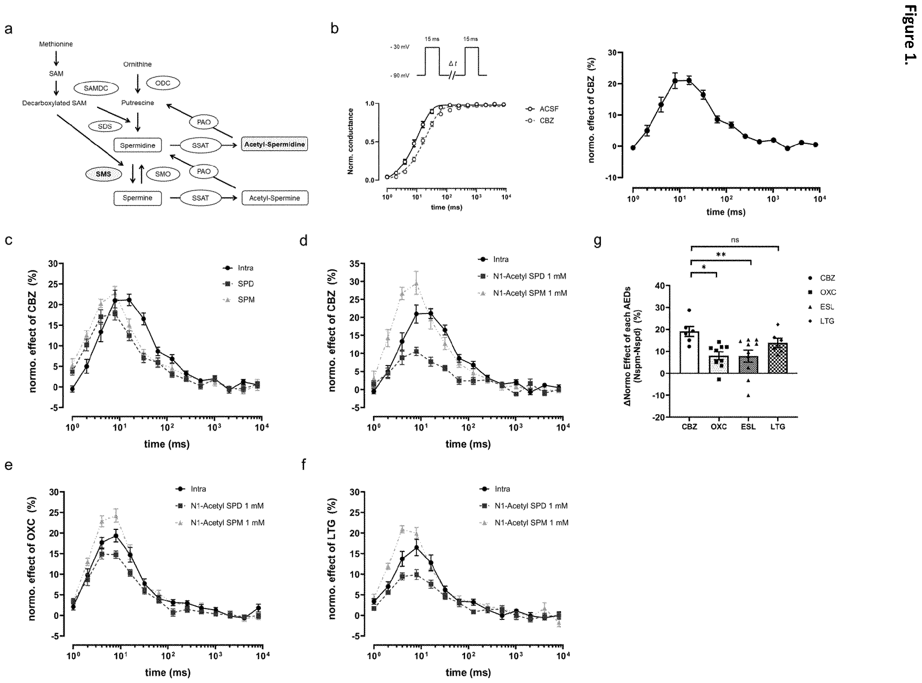

Embodiment A is based on the finding that N1aSPD (at an increased level) significantly reduces the efficacy of some $Na_V$CBs (and/or N1aSPM (at increased levels) increases the efficacy of some NavCBs) (cf., for example, **Fig. 1d-f**). In the context of the method of screening of this embodiment (a) measurement(s)/(a) testing step(s) based on (i) a control intracellular solution, and (ii) an intracellular solution with (an increased level of) N1aSPD, may be performed. The method of screening of this embodiment may, for example, be performed so that a control curve (for example effect vs. time of sodium channel recovery from sodium channel inactivation) is generated for a given test compound/NavCB (no added N1aSPD (or N1aSPM); see, for example, "Bath" in **Fig. 1d**). Further, a curve (for example effect vs. time of sodium channel recovery from inactivation) for said/a test compound/NavCB in the presence of an increased level of N1aSPD (and/or an increased ratio of the levels of N1aSPD/N1aSPM) is generated (see, for example, "N1-Acetyl SPD" in **Fig. 1d**). The/a compound/NavCB may be identified in accordance with the invention when the curve generated in the presence of an increased level of N1aSPD (and/or an increased ratio of the levels of N1aSPD/N1aSPM) overlaps with the control curve (for example overlapping at a deviation of $<\_30\%$, $<\_20\%$, $<\_10\%$, $<\_5\%$ or $\leq 2\%$, relative to the respective integrals below the respective curves; in principle, the lower deviation values are preferred).

The method of screening of this embodiment is particularly useful in primarily identifying, for example from compound libraries, compounds (NavCBs) which efficacy in sodium channel blocking is not (or only little/less or minimally) affected (or which efficacy may even be increased) by an addition of N1aSPD and an increased level of N1aSPD, respectively.

The method of screening of this embodiment may be performed with manual patch-clamping (e.g. by using a micropipette), as well as with semi-automated, automated or high-throughput patch-clamping (e.g. by using a planar patch-clamp device like a patch-clamp chip); cf., for example, **Fig. 6,** left and right panel, respectively, for illustration only. All those clamping techniques allow for adding/setting/controlling/modifying N1aSPD (and/or N1aSPM; or other polyamine species) in the intracellular milieu (cf., for example, **Fig. 6** for illustration only). Semi-automated, automated or high-throughput approaches, and the respective devices, are particularly useful in primarily identifying compounds as the $Na_V$CBs in accordance with the invention, for example from compound libraries.

The method of screening of this embodiment is also particularly useful for whole-cell patch-clamping/a whole-cell patch-clamp configuration. This is because this method entails obtaining low-resistance whole-cell access with a solution-filled clamping device (like a micropipette or a planar patch-clamp chip). The solution in the clamping device (sometimes also termed "intracellular solution" herein) equilibrates with the intracellular milieu (solution in the cell lumen)), allowing to add/set/control/modify (a) specific polyamine species, like N1aSPD (and/or N1aSPM). In the context of this method, the effect of other polyamine species (on a compound's/$Na_V$CB's sodium channel-blocking efficacy) can also readily be tested.

In the following, an exemplary, non-limiting detailed protocol of the method of screening of Embodiment A is provided. Any, several or all respective particular step(s) may be performed in this respect (preferably in the described order); with any, several or all of the respective particular details.

**Step 1: Provision of cell model (HEK cell model or another cell model).**

Any HEK cell line or other cell line expressing the sodium channel of interest (e.g. $Na_V$1.2 and/or Nav1.6; or $Na_V$1.7 and/or $Na_V$1.8) may be used. In case of screening for an AEM, CNS sodium channel isoforms like $Na_V$1.2 and/or $Na_V$1.6 would be preferred. In the case of screening for other $Na_V$CBs (for example for the treatment of pain syndromes), other sodium channel isoforms may be used (including, for example, $Na_V$1.7 and Nav1.8). In the context of Embodiment A, HEK293T cells may be used, a line derived from human embryonic kidney at passage 10-20. The HEK cell line may stably express the endogenous human *SCN2A* and/or *SCN8A* gene encoding $Na_V$1.2 and $Na_V$1.6 voltage-gated sodium channels respectively. The cells may be cultured in Dulbecco's modified Eagle's medium (61965026, Thermo Fisher Scientific) which may contain 10% (vol/vol) FBS (10270106, Thermo Fisher Scientific) with 500 $\mu$g/mL Geneticin (10131035, Thermo Fisher Scientific), 1 mM sodium pyruvate (11360039, Thermo Fisher Scientific), and 1% (vol/vol) MEM Non-essential amino acids solution (11140035, Thermo Fisher Scientific). Culturing may be at 37 °C and 10% $CO_2$. HEK293T cells may then be plated on poly-D-lysine coated glass for voltage- clamp recordings, e.g. whole-cell voltage-clamp recordings.

**Step 2: Preparation of intra- and extracellular solutions.**

For recording sodium currents, standard solutions can be used for patch-clamp experiments. In the context of this embodiment, an extracellular solution for super fusion during patch-clamp experiments may be used; the extracellular solution may contain (in mM): 40 $CH_3SO_3Na$, 90 TEA-Cl, 10 HEPES (free), 1.6 $CaCl_2 \cdot 2H_2O$, 2 $MgCl_2 \cdot 6H_2O$, 0.2 $CdCl_2 \cdot 2H_2O$, five 4-Aminopyridine (4-AP), and 15 glucose. The pH may be adjusted to 7.4 (e.g. with HCl) and the osmolarity to 310 mOsm/L (e.g. with glucose).

The control intracellular solution may contain the following (in mM): 110 CsF, 10 HEPES-Na, 11 EGTA, 2 $MgCl_2$, 20 tetraethylammonium chloride (TEA-Cl), 0.5 GTP-Na, 5 ATP-$Na_2$, pH 7.25 adjusted with, for example, CsOH and 300 mOsm/L, for example, with sucrose.

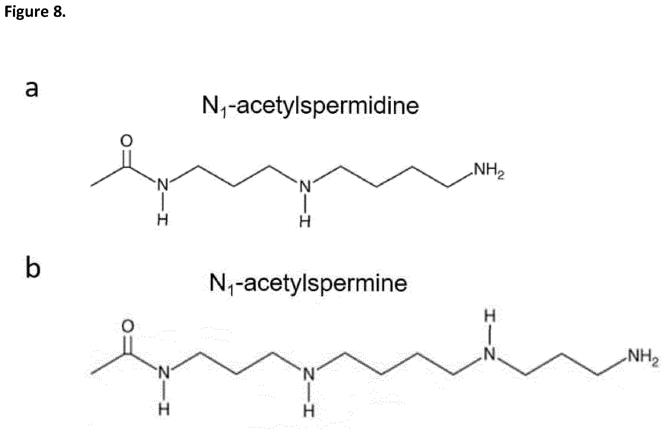

To generate an intracellular milieu with polyamines, these may be added to this intracellular solution. $N_1$-Acetylspermidine dihydrochloride (e.g. 100 $\mu$M to e.g. 1 mM; Cayman chemical, Cay9001535-10) or $N_1$-Acetylspermine trihydrochloride (e.g. 100 $\mu$M to e.g. 1 mM; Sigma-Aldrich, 01467-25MG) may be added. The structures of $N_1$-Acetylspermidine and $N_1$-Acetylspermine are displayed in Fig. 8.

**Step 3: Establishing of a (for example whole-cell) voltage-clamp recording.**

Voltage-clamp recordings can be carried out by using standard methods for recording voltage-gated channels (for example by using respective test devices; see herein elsewhere for details). In the context of this embodiment, manual patch-clamp approaches may be used, but (semi-)automated a high-throughput patch-clamp approaches will also be applicable. All voltage-clamp experiments are usually performed at room temperature (22 ± 1 °C). The applicability at room temperature further facilitates use of this method for screening. Patch pipettes may be pulled with a GB150F-8P borosilicate glass (e.g. Science Products) with a micropipette puller (e.g. Sutter Instruments). The tip resistance may be 3.0 to 5.0 M$\Omega$. Tight-seal whole-cell recordings may be obtained with a seal resistance of >1 G$\Omega$ using a patch-clamp amplifier (e.g. Axopatch 200B, Molecular Devices). Series resistance may be compensated by 70-90%, with a maximal residual voltage error of < 5.3 nA. At least 10 minutes may be allowed to pass after establishing whole-cell configuration to allow the intracellular solution and the internal milieu of the cell to equilibrate. During voltage-clamp recordings data may be sampled with a 50 kHz sampling rate using, for example, a Digidata 1440A AD converter (Molecular Devices), and may be filtered with a 10 kHz low-pass filter. High sampling rates and filter settings are important in the case of voltage-gated sodium channels, as the kinetics of the respective currents are very fast. The skilled person, however, can readily adjust these. The Clampex 10.7 acquisition suite (Molecular Devices), for example, may be used for data acquisition. A liquid junction potential of 10 mV may be calculated and may be corrected offline for all measured (HEK293T) cells.

**Step 4: Application of Medicament/Drug (test compound/potential $Na_VCB$ to be tested/screened).**

The potential NavCB to be tested (for example, carbamazepine (CBZ, e.g. Sigma-Aldrich, C4024-5G), oxcarbazepine (OXC, e.g. Merck, PHR 1635-1G), eslicarbazepine acetate (ESL, e.g. Merck, B5435-10MG), and lamotrigine (LTG, e.g. Merck, PHR 1392-1G) as controls), may be dissolved in dimethyl sulfoxide (DMSO) and may be added to the extracellular solution (final concentration; e.g. 100 $\mu$M). Any $Na_VCB$ candidate may be dissolved in an analogous manner, taking care to include, for example, the same DMSO concentration in all measurement conditions. Drug solutions may be applied with any suitable device leading to exchange of the extracellular solution. In the context of the herein exemplified, non-limiting particular case, a super fusion device was employed that leads to an extracellular solution exchange in 1-2 minutes. After solution change, 5 minutes may be left to elapse to ensure complete solution exchange.

**Step 5: Setting of Voltage-clamp paradigms & data analysis.**

During (for example whole-cell) recordings of sodium channels, the voltage-clamp model of the amplifier may be used to run different voltage protocols. The most important voltage protocol is the one assessing recovery from fast inactivation of $Na^+$ currents. For this purpose, a double-pulse experiment with increasing intervals t = 1ms * 2n (n = 0, 1, 2, 3, 4, 5, 6, 7, 8, 9, 10, 11, 12, 13) between the first and second pulse may be applied for evaluation of recovery from fast inactivation. To ensure full recovery from inactivation, an interval of 5 s at -80 mV holding potential may be interspersed between single protocols. The exemplary, non-limiting results from such a protocol are depicted in **Fig. 1b** (left panel). Subsequently, the NavCB of choice may be super fused. Following complete exchange of the extracellular solution, the double pulse protocol may be repeated (as exemplarily and illustratively shown in **Fig. 1b,** left panel for CBZ). This allows quantitative evaluation of $Na_VCB$ effects on recovery from inactivation. The following analysis steps may be performed in this respect:

First, peak sodium channel amplitude from the second pulse may be normalized to that from the first one. Secondly, a subtraction may be carried out, subtracting the normalized peak values after $Na_VCB$ application from those under control conditions (as exemplarily and illustratively shown for CBZ in **Fig. 1b,** right panel). The peak of this curve indicates the magnitude of the effect on recovery from inactivation, underlying use-dependent block, and may be averaged across cells. The overall magnitude of the effects of acetyl-polyamine variants may then be assessed as a difference in CBZ effects (or effects of other but compounds/Na$_V$CBs), for example, for 1 mM N$_1$-acetylspermidine on the one hand and, for example, 1 mM N$_1$-acetylspermine on the other hand. This value indicates how prominently the acetylpolyamine modulation is influencing the use-dependent effects of a given compound (as exemplarily and illustratively shown for the Na$_V$CBs CBZ, OXC, and LTG in **Fig.1g**). The patch-clamp data analyses may be done with Clampfit 10.7 software (Molecular Devices) and/or GraphPad Prism 8 (GraphPad Software).

In addition to the recovery from inactivation, other voltage and time-dependent properties of sodium channels, and their dependency on polyamines (like N1aSPD) may also be readily assessed by adding other types of voltage protocols, which are known to the skilled person.

Embodiment B relates to a method of screening for a NavCB by using a genetically modified cell line/(a) genetically modified cell(s). In the context of this embodiment, an increased intracellular level of N1aSPD (and/or an increased intracellular ratio of the levels of N1aSPD/N1aSPM) is achieved by modifying the expression of one (or more) of the enzymes of the polyamine metabolism (see, for example, **Fig. 1a** for illustration purposes). In particular, the expression of SMS may be reduced in this context. This represents the reduction in SMS expression as, for example, found in experimental and human epilepsies. The HEK system may be used in this respect (see, for example, Embodiment A above for non-limiting details). Any genetic engineering technique may be used for genetically modifying the cell(s)/cell line; for example, for knocking down or reducing the SMS expression/function. One convenient respective example is the siRNA approach. An illustrative scheme of a stepwise protocol for a respective exemplary HEK cell-based screening method is shown in **Fig. 7b.** A non-limiting example of such a method is described in detail in appended Example 8.

In the context of this embodiment, any cell(s)/cell line that expresses the functional sodium channel(s) of interest (see above for non-limiting details), but with a modified expression of one (or more) of the enzymes of the polyamine metabolism (for example with a reduced expression of SMS) may be used. In addition, a variant of the same cell line with a non-modified expression of one (or more) of the enzymes of the polyamine metabolism (for example with a non-reduced SMS expression) may be employed as a control. In the context of this embodiment, a HEK cell line, or other cell(s)/cell line, stably expressing (a) sodium channel(s) of interest can be used. Reduction in SMS expression (and/or modification of expression of (an) other enzyme(s) of the polyamine metabolism) may be achieved by a siRNA/shRNA-based modification; for example, by an siRNA/sh-based knockdown of SMS.

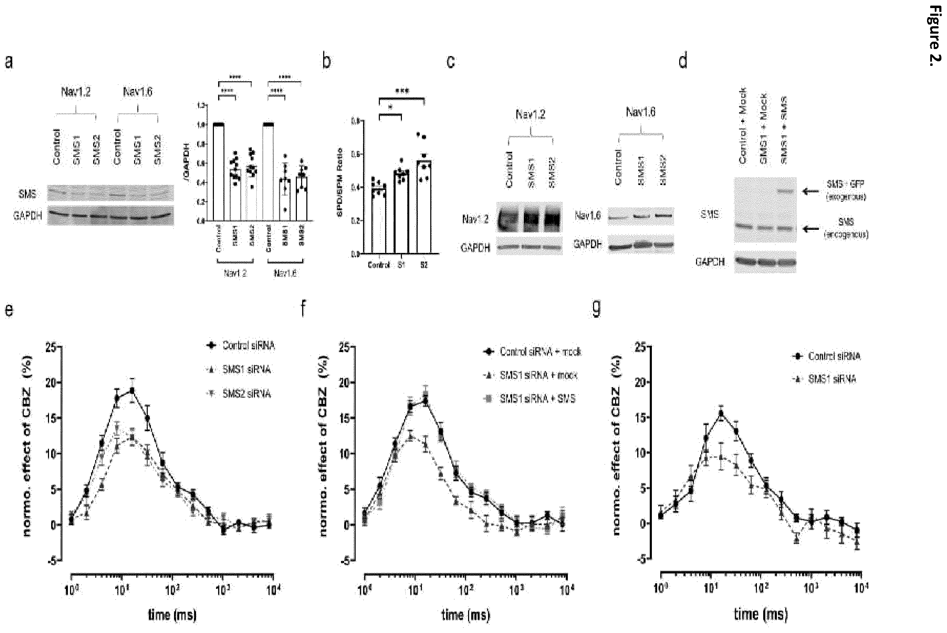

Embodiment B is also based on the finding that N1aSPD (at an increased level) significantly reduces the efficacy of some Na$_V$CBs (and/or N1aSPM (at increased levels) increases the efficacy of some NavCBs) (cf., for example, **Fig. 2,** in particular **Fig. 2b, e, g**). In the context of the method of screening of this embodiment, (a) measurement(s)/(a) testing step(s) based on (i) a control intracellular environment (without (a) modified enzyme(s) of the polyamine metabolism; and without an addition of N1aSPD), and (ii) an intracellular environment with (a) modified enzyme(s) of the polyamine metabolism (e.g. reduced SMS function/expression), may be performed. The latter situation corresponds to a situation of the presence of an increased level of N1aSPD (and/or an increased ratio of the levels of N1aSPD/N1aSPM); cf., for example, Embodiment A, above. In the context of Embodiment B, measurements of the use-dependent block of a compound of interest (NavCBs to be tested/screened) in a control cell line (non-modified expression of one (or more) of the enzymes of the polyamine metabolism; e.g. no reduction/loss of SMS) may be compared with an experimental cell line (modified expression of one (or more) of the enzymes of the polyamine metabolism; e.g. reduction/loss of SMS) for the compound NavCBs. Positive hits would be compounds/NavCBs that do not exhibit a loss of efficacy, or only a slight reduction of efficacy (e.g. if SMS expression is reduced). The method of screening of this embodiment may, for example, be performed so that a control curve (for example effect vs. time of sodium channel recovery from sodium channel inactivation) is generated for a given test compound/NavCB (based on (a) cell(s)/cell line with non-modified expression of one (or more) of the enzymes of the polyamine metabolism; e.g. no reduction/loss of SMS; see, for example, "Control siRNA" and "Without siRNA" in **Fig. 2e** for illustration only). Further, a curve (for example effect vs. time of sodium channel recovery from inactivation) is generated for said/a test compound/NavCB in a respective cell line/cell(s) with modified expression of one (or more) of the enzymes of the polyamine metabolism is generated (e.g. cell line/cells with reduction/loss of SMS expression; see, for example, "SMS1 siRNA" and "SMS2 siRNA" in **Fig. 2e** for illustration only). The latter situation corresponds to the situation of the presence of an increased level of N1aSPD (and/or an increased ratio of the levels of N1aSPD/N1aSPM) (cf. **Fig. 2b, e** for illustration only). The compound/NavCB may be identified in accordance with the invention when the curve generated with the modified expression of one (or more) of the enzymes of the polyamine metabolism overlaps with the control curve (for example overlapping at a deviation of $\leq 30\%$, $\leq 20\%$, $\leq 10\%$, $\leq 5\%$ or $\leq 2\%$; relative to the respective integrals below the respective curves; in principle the lower deviation values are preferred). The method of screening of also this embodiment is particularly useful in primarily identifying, for example from compound libraries, compounds (NavCBs) which efficacy in sodium channel blocking is not (or only little/less or minimally) affected (or which efficacy may even be increased) by an increased level of N1aSPD.

The method of screening of this embodiment may be performed with manual patch-clamping (e.g. by using a micropipette), as well as with semi-automated, automated or high-throughput patch-clamping (e.g. by using a planar patch-clamp device like a patch-clamp chip; cf., for example and illustration, **Fig. 6,** left and right panel, respectively; but without the external N1aSPD application). All those clamping techniques provide respective testing devices (see also, for example, **Fig. 6** for illustration only). Semi-automated, automated or high-throughput approaches, and the respective testing devices, are particularly useful in primarily identifying compounds as the NavCBs in accordance with the invention, for example from compound libraries

The method of screening of this embodiment is also particularly useful for whole-cell patch-clamping/a whole-cell patch-clamp configuration. This is because this method entails obtaining low-resistance whole-cell access with a solution-filled clamping device (like a micropipette or a planar patch-clamp chip). In the context of this method, the effect of other polyamine species (on a compound's/$Na_V$CB's sodium channel-blocking efficacy) can also readily be tested (for example by modifying the expression of one (or more) other/different enzymes of the polyamine metabolism so as to achieve modulation of other polyamines which affect Nav channel sensitivity).

In the following, an exemplary, non-limiting detailed protocol of the method of screening of Embodiment B is provided. Any, several or all respective particular step(s) may be performed in this respect (preferably in the described order); with any, several all of the respective particular details.

### Step 1: Provision of cell model (HEK cell model or another cell model).

The cell model may be provided as described in the context of Embodiment A above.

### Step 2: Performing SMS knockdown (or modification of (an)other enzyme(s) of the polyamine metabolism).

Any approach to knock down or reduce SMS function (or to modify function of (an)other enzyme(s) of the polyamine metabolism) would be suitable to generate a screening system in accordance with this embodiment. For example, twenty-four hours after the last passage, the cells may be transfected with siRNAs by using, for example, lipofectamine 2000 (11668027, Thermo Fisher Scientific) according to the manufacturer's instructions. Oligonucleotide siRNA duplexes may be synthesized (e.g. by Sigma-Aldrich) with the following sequences:

- control-siRNA (e.g. mission® sirna universal negative control #1, sic001-10nmol; Sigma-Aldrich)
- SMS siRNA-set1-S1: 5'-CGACAGUAUUUUGAACAAA-3' (SEQ ID NO. 1)
- SMS siRNA-set2-S2: 5'-GAUUCACCUUAUCAAAAUA-3' (SEQ ID NO. 2)

As set1 and set2 siRNA yielded equivalent results regarding the knockdown efficacy for SMS in non-limiting appended examples (see **Fig. 2a, b**) without affecting sodium channel expression in HEK cells expressing either $Na_V$1.2 or $Na_V$1.6 (**Fig. 2c**), only one of such siRNAs, for example set1 siRNA (S1), may be used for SMS knockdown throughout the experiments. For validation that any effects of knockdown are specific to SMS reduction (or to modification of (an)other enzyme(s) of the polyamine metabolism), rescue experiments may be performed; in particular in case a new system is established. For example, SMS rescue experiments may be performed by seeding HEK cells on a 24-well plate and transfecting them with 50 pmol control siRNA, 250 ng mock plasmid, 50 pmol S1-siRNA, and 250 ng SMS plasmid using lipofectamine 2000 as described above (this may be adapted to (an)other target enzyme(s). Exemplarily, the pCMV6-SMS-GFP construct may be purchased as SMS plasmid from Origene (rg233729). Rescue experiments are successful when resulting in re-establishment of enzyme function/expression (e.g. SMS expression/function; see **Fig. 2d** for illustration). As was also exemplarily shown herein, rescue results in reinstatement of NavCB efficacy (e.g. CBZ efficacy) on the use-dependent block. This confirms that enzyme modification (e.g. SMS reduction) is causal in modification of these effects (see **Fig. 2f** for illustration).

### Step 3: Verifying SMS knockdown (or modification of (an)other enzyme(s) of the polyamine metabolism).

To quantitatively verify SMS knockdown (or modification of (an)other enzyme(s)), immunoblotting may be used. To this end, the following approach may be applied. The protein samples may be homogenized with RIPA buffer (182-02451, Wako) containing 10% protease inhibitors (4693116001, Roche) and phosphatase inhibitors (4906845001, Roche). Homogenates may be subsequently incubated on ice for 20 min and centrifuged at, for example, 10,000 rpm for 10 min at 4°C. The lysates may be mixed with $4 \times$ NuPAGE LDS sample buffer (NP0008, Novex) and may be heated at 95°C for 5 min. After denaturation, each cell lysate may be separated by SDS-PAGE (e.g. 10% gel) for anti-SMS antibody (or antibody against (an)other enzyme) and by SDS-PAGE (e.g. 8% gel) for anti-Nav1.2 antibody (or antibody against (an)other $Na_V$C), and transferred to a nitrocellulose membrane, which may be blocked in, for example, 5% milk subsequently. The primary and secondary antibodies may be as the follows: Recombinant anti-spermine synthase rabbit monoclonal [EPR9252(B)] (1:2000, ab156879, abcam); anti-Nav1.2 rabbit polyclonal (1:200;

ACC-002, Alomone Labs); anti- GAPDH mouse monoclonal (1:1000, NB300-221, Novus); IRDye® 800CW goat anti-rabbit IgG secondary antibody (1:10000, 926-32211, LI-COR Biosciences); IRDye® 680LT donkey anti-mouse IgG secondary antibody (1:10000, 926-68022, LI-COR Biosciences). The primary antibodies may be incubated for 2 hours at RT; while the fluorescently labeled IRDye anti-rabbit 800 nm IgG (LI-COR Biosciences) or IRDye anti-mouse 680 nm may be incubated for 45 minutes at RT; and may be detected with the infrared Odyssey system (LI-COR Biosciences). The band intensity may be quantified by Image Studio Lite version 5.2 software (LI-COR Biosciences). The protein band intensity may be determined as a ratio of a housekeeping protein, for example glycerinaldehyde-3-phosphate-dehydrogenase (GAPDH), within the same membrane, and may be normalized to the average ratio obtained in sham-control. The quantitative reduction of SMS expression (or of an(other) enzyme) at the protein level may be calibrated to be similar to that observed in animal models of epilepsy (around 50%).

**Step 4: Establishing of a (for example whole-cell) voltage-clamp recording; Step 5: Application of Medicament/Drug (test compound/potential Na$_V$CB to be tested); Step 6: Setting of Voltage-clamp paradigms & data analysis.**

Subsequently, patch-clamp analyses may be performed (for example with respective test devices; see herein elsewhere for details). These analyses (and test devices) may be methodically identical to those described in steps 2-5 of the screening method as described for Embodiment A, above. However, in the context of Embodiment B, all recordings are performed with a control intracellular solution (i.e. a solution in the patch-clamp devise) without the addition of polyamines (e.g. N1aSPD). In Embodiment B, measurements of a use-dependent block of compounds of interest in a control cell line (no loss of, for example, SMS), would be compared with an experimental cell line (reduction of, for example, SMS) for each compound. Positive hits would be compounds that do not exhibit a loss of efficacy if SMS expression is reduced (or if modification of (an)other enzyme(s) of the polyamine is performed).

Embodiment C relates to a method of screening for a NavCB by using genetically modified-neurons, in particular primary neurons. The purpose of this screening method is to assess the activity of Na$_V$CBs on neuronal network activity, more specifically in a neuronal population with reduced expression of SMS (or of (an)other enzyme(s) of the polyamine metabolism; corresponding to a situation of the presence of an increased level of N1aSPD (and/or an increased ratio of the levels of N1aSPD/N1aSPM)). In the context of this embodiment, primary neuronal cells/cultures may be used. A non-limiting example of such a method is described in detail in appended Example 9.

In the context of this embodiment, an increased intracellular level of N1aSPD (and/or the increased intracellular ratio of the levels of N1aSPD/N1aSPM) in primary neuronal cells is (like in Embodiment B, above) achieved by modifying the expression of one (or more) of the enzymes of the polyamine metabolism (see, for example, **Fig. 1a** for illustration). In particular, the expression of SMS may be reduced in the context of this embodiment. Any genetic engineering technique may be used for genetically modifying the primary neuronal cells/cultures in the context of Embodiment C; for example, for knocking down or reducing the SMS expression/function. One convenient respective example is the siRNA or shRNA approach. To generate a modified expression of one (or more) of the enzymes of the polyamine metabolism (for example a reduced expression of SMS) either primary neuronal cultures may be genetically engineered accordingly (cf. also Embodiment B, above, for non-limiting details). Alternatively, primary neuronal cultures may be generated from accordingly genetically engineered animals (like, for example, heterozygous SMS knockout mice; or mice with knockout of (an) other enzyme(s) of the polyamine metabolism). In the context of this embodiment, any (primary) neuronal cells/cultures that express the functional sodium channel(s) of interest (see above for non-limiting details), but with a modified expression of one (or more) of the enzymes of the polyamine metabolism (for example with a reduced expression of SMS) may be used. In addition, a variant of the same cells/cell line with a non-modified expression of one (or more) of the enzymes of the polyamine metabolism (for example with a non-reduced SMS expression) may be employed as a control. Reduction in SMS expression (and/or modification of expression of (an) other enzyme(s) of the polyamine metabolism) may be achieved by a siRNA/shRNA-based modification; for example, by an siRNA-based knockdown of SMS.

Embodiment C is also based on the finding that N1aSPD (at an increased level) significantly reduces the efficacy of some Na$_V$CBs (and/or N1aSPM (at increased levels) increases the efficacy of some NavCBs). In the context of the method of screening of this embodiment, (a) measurement(s)/(a) testing step(s) based on a (i) control intracellular environment (without (a) modified enzyme(s) of the polyamine metabolism; and without addition of N1aSPD), and (ii) an intracellular environment with (a) modified enzyme(s) of the polyamine metabolism (e.g. reduced SMS function/expression), may be performed. The latter situation corresponds to a situation of the presence of an increased level of N1aSPD (and/or an increased ratio of the levels of N1aSPD/N1aSPM); cf., for example, Embodiment A, above. In the context of Embodiment C, measurements of use-dependent block of a compound of interest (test compounds/NavCBs to be tested/screened) in a (primary) neuronal cells/culture control (non-modified expression of one (or more) of the enzymes of the polyamine metabolism; e.g. no reduction/loss of SMS) may be compared with an experimental (primary) neuronal culture/experimental primary neuronal cells (modified expression of one (or more) of the enzymes of the polyamine metabolism; e.g. reduction/loss of SMS) for the compound/NavCB. Positive hits would be compounds/NavCBs that do not exhibit a loss of efficacy for the experimental setting (e.g. if SMS expression is reduced; or only a slight reduction of efficacy).

The method of screening of this embodiment is likewise very useful in primarily identifying, for example from compound libraries, compounds (NavCBs) which efficacy in sodium channel blocking is not (or only little/less or minimally) affected (or which efficacy may even be increased) by an increased level of N1aSPD. The method of screening of this embodiment may be performed manually, as well as with semi-automated, automated or high-throughput approaches. Measuring neuronal activity may be performed with any suitable method that assesses neuronal spiking. For example, microelectiodes, e.g. in form of multielectrode arrays (MEAs), may be used. These enable (semi-)automated or high-throughput measurements. All those techniques provide respective testing devices. Semi-automated, automated or high-throughput approaches, and the respective testing devices, are particularly useful in primarily identifying compounds as the $Na_V$CBs in accordance with the invention, for example from compound libraries.

Also in the context of this embodiment, the effect of other polyamine species (on a compound's/$Na_V$CB's sodium channel-blocking efficacy) can readily be tested (for example by modifying the expression of one (or more) other/different enzymes of the polyamine metabolism so as to achieve modulation of other polyamines which affect NavC sensitivity).

In the following, an exemplary, non-limiting detailed protocol of the method of screening of Embodiment C is provided. Any, several or all respective particular step(s) may be performed in this respect (preferably in the described order); with any, several all of the respective particular details.

**Step 1: Generating SMS loss-of-function mice (or generating mice with a (for example loss of function) modification of (an)other enzyme(s) of the polyamine metabolism).**

SMS loss-of-function, or other enzyme modification, can be generated by several ways. Firstly, shRNA-/siRNA-based knockdown may be used; for example, with methods for knockdown which are analogous to those as described herein elsewhere (for example in the context of Example 8, below or in the context of Embodiment B described above). What is described there may also apply here, *mutatis mutandis.* In addition, (primary) neurons generated from mice (or other animals) with a genetic deletion of SMS (or of (an)other enzyme(s)) may be used. This includes mice with a spontaneously occurring loss-of-function of SMS (i.e. gyro mice; or mice with loss-of-function (or increased function) of (an)other enzyme). Male Gyro mice lacking SMS due to a deletion of part of the X chromosome are viable on the B6C3H background. However, these mice are less preferred. Conditional knockout mouse lines may thus also be used (preferred option). Commercially available mouse lines are listed as follows:

- C57BL/6J/Sms[em6Lutzy]/J (Strain #: 032064, The Jackson Laboratory)
- CS7BL/6Smoc-[SMSem1(flox)Smoc] (Cat. No. NM-CKO-2117943, Shanghai Model Organisms)
- C57BL/6JGpt-Sms-flox (T016254, GemPharmatech Co., Ltd.)

Neuron cultures, in particular primary neuron cultures, from different regions (forexample, CNS neurons for applications such as epilepsy, DRG neurons for pain-related applications) may be generated from such mice/other animals. Viral gene transfer, for example, of Cre-recombinase, may then lead to a loss of SMS expression (or to a modification of (an)other enzyme of the polyamine metabolism).

**Step 2: Generating neuronal culture (for example primary neuronal culture).**

To generate (primary) neuronal cultures multiple protocols are available. For example, embryos staged at E14-16 may be taken under deep anaesthesia with isoflurane inhalation; and may be transferred to a 10-cm Petri dish on ice. The heads may be decapitated; and the brains may be dissected under a stereo microscope at room temperature. The dissected cortices may be transferred in 1.8 ml HBSS (-) (14170-088, 500 ml; e.g. Gibco) and 200 $\mu$L Trypsin (15090046, 2.5% Trypsin, 100 ml; e.g. Gibco), and may then be incubated at 37°C and 5% $CO_2$ for 15-20 minutes. The cortical tissues may be triturated with 1ml of 10 mg/mL DNase I (Final concentration 1mg/mL) (11284932001, 100 mg; e.g. Roche) and 120 $\mu$L of 1M $MgSO_4$-$7H_2O$ and 9 ml of cold HBSS (-). The triturated tissue may be washed with the NeuroBasal medium (21103-049, 500ml; e.g. Gibco) complemented with 2% B27 (17504-044, 10ml; e.g. Gibco) and 0.4% L-glutamine (25030-024, 2ml, Invitrogen; e.g. Gibco). The cell suspension containing, for example, 40,000 neurons per 5-20 $\mu$L may be plated at 1 well on 24 MEA plate (e.g. CytoView MEA 24, M384-tMEA-24W, Axion Biosystems); and may be incubated at 37°C and 5% $CO_2$ for 30-60 minutes. It may be added with the NeuroBasal medium 700 $\mu$l/well (21103-049, 500ml; e.g. Gibco) complemented with 2% B27 and 0.4% L-glutamine.

**Step 3: Producing an AAV vector (for example (SMS-)v-knockdown construct or Cre driven by Synapsin-1 promotor construct).**

Targeting sequences of, for example, shRNA may be Sms; shControl, shSms-1, and shSms-2 as described herein elsewhere. Then, pAAV-Syn1-shControl-EGFP, pAAV-msyn1-shSms-1-T2A-EGFP, and pAAV-Syn1-shSms-2-T2A-EGFP as described herein elsewhere may be ordered to VectorBuilder. Regarding Cre-driver, the pAAV-msyn1-EGFP and pAAV-Syn1-Cre-T2A-EGFP may be purchased from VectorBuilder. Such AAV vector plasmids may contain synapsin promotor and, for example, two fluorescent proteins. Recombinant AAV1/2 genomes may be generated by large scale triple transfection of HEK293-AAV cells; the AAV plasmid, helper plasmids encoding *rep* and *cap* genes (e.g. pRV1 and pH21), and adenoviral helper (e.g. pFΔ6; Stratagene, La Jolla, USA) by using standard transfection (e.g. CaPO$_4$ transfection). At, for example, 48-72h after transfection, cells may be lysed in 0.5% sodium deoxycholate (Sigma-Aldrich, D6750) and 50 U/ml Benzonase endonuclease (Sigma-Aldrich, 1.01697). rAAV viruses may be purified using HiTrap heparin column purification (GE Healthcare). rAAV particles may be concentrated to a final volume of 400 μl, for example, by Amicon Ultra Centrifugal Filters (Millipore). Purity of the viruses may be validated by coomassie blue staining of SDS-polyacrylamide gels. Functional titers of the fluorescent protein vectors with or without doxycycline (DOX; e.g. Sigma-Aldrich, D3072-1ML) may be determined by transduction of cultured primary neurons. In this context, non-limiting guidance and illustrative reference is made to Schoch (The Journal of neuroscience: the official journal of the Society for Neuroscience 41 (39), 2021, 8111-25); van Loo (The Journal of neuroscience: the official journal of the Society for Neuroscience 39 (17), 2019, 3175-87); va n Loo (Nature communications 6, 2015, 8688).

**Step 4: Performing SMS knockdown (or modification of (an)other enzyme(s) of the polyamine metabolism).**

Some particulars pertaining to this may be as described in the context of step 2 of Embodiment B, above, or as described in the context of Example 9 (what is said there may apply also here, *mutatis mutandis).* For shRNA-mediated knockdown, primary cortical neuronal cultures from, for example, C57BL/N mice may be infected at DIV4 with AAV1/2-Syn1-shControl-EGFP, AAV1/2-Syn1-shSms-1-T2A-EGFP, and AAV1/2-msyn1-shSms-2-T2A-EGFP. If conditional SMS knockout mice/animals are used (or mice/animals with conditional modification of (an)other enzyme(s) of the polyamine metabolism), primary cortical neuronal cultures may be used. At DIV4, primary cortical neurons may be infected, for example, with a combination of AAV1/2-Syn1-EGFP and AAV1/2-Syn1-Cre-T2A-EGFP. Two weeks later (at DIV 18), reduction of the SMS protein levels may be confirmed using Western blotting (see above for non-limiting details). Na$_V$CBs and candidate drugs may then be administered at DIV18-25, when protein levels of SMS are reduced (or protein levels of (an)other enzyme(s) are modified).

**Step 5: Performing microelectrode/MEA analyses.**

Any approach to measure activity in neurons is generally suitable. MEA recordings are appropriate because they have a high throughput and are straightforward to analyse. MEA recordings can be conducted on any suitable system. For example, they may be conducted using Maestro Edge System with CytoView-MEA24 well plates from Axion Biosystems (M384-tMEA-24W, $4 \times 4$ grids (1.1 mm $\times$ 1.1 mm) = 16 electrodes, 1 grid = 50 μm diameter). Neurons may be cultured on these MEA 24 plates and may be recorded every 2-3 days. Baseline recording may initially be captured without test compounds/NavCBs, followed by experiments to observe the acute effects of compounds/NavCBs at DIV 20-23. To maintain the robustness and reproducibility of MEA data, the medium may not be changed through the experiments. The Maestro settings may be as follows: a sampling frequency of 12.5 kHz, a "Neural Spikes" gain of 1000$\times$, and a band-pass filter of 200-3000 Hz as the default neural setting. To visually inspect the activity of each plate during recording, the data processing may be performed by, for example, using "Spike Detector (6 $\times$ STD)" and "Burst Detector". Spikes may be detected when the signal crossed the threshold of 6$\times$ standard deviation of the baseline, while bursts may be identified when the interval between spikes was within 100 ms and at least 5 spikes overlapped. For MEA recordings, the Maestro system may be maintained at 37°C and 5% CO$_2$. The 24-well MEA plate containing primary cortical neuron culture may be placed into a docking bay and may be allowed to equilibrate for 5 minutes. Following this, a 5-minute baseline recording may be initiated. After applying a Na$_V$CB(s)/a test compound, a 5-minute wash-in recording may be started. Post wash-in, 75% of medium may be replaced with NeuroBasal medium 700 μl/well supplemented with 2% B27 and 0.4% L-glutamine, and a 5-minute wash-out recording may be conducted.

The raw data (RAW files) may be analyzed by Axion Integrated Studio (Axis) software (Axion BioSystems; version 3.7.3) and the spike files (SPK files) may be analyzed by the NeuralMetric Tool (Axion BioSystems; version 4.0.5). This Ax!S navigator identifies spikes and bursts using previously described spike and burst detectors. Activities may be broadly grouped into four categories: spiking, single-electrode bursting, network bursting, and synchrony. To compare the activity of baseline, wash-in, and wash-out in every parameter among ASMs, activities may be normalized in the activities of DMSO to account for the potential DMSO effects. Additionally, the effects of Na$_V$CBs may be evaluated by normalizing the baseline prior to drug wash-in.

Embodiment D relates to a method of screening for a NavCB by using genetically modified iPSC-derived systems, in particular human iPSC-derived systems. The purpose of this screening method is to assess the activity of test compounds/NavCBs on neuronal network activity; for example, with reduced SMS expression/function (or with modification of (an)other enzyme(s) of the polyamine metabolism; corresponding to a situation of the presence of an increased level of N1aSPD, and/or an increased ratio of the levels of N1aSPD/N1aSPM). A non-limiting example of such a method is described in detail in appended Example 10.

The targeting sequences, for example of SMS-shRNAs (or of shRNAs which target (an)other enzyme(s) of the polyamine metabolism), may be synthesized in the same way as the constructs for mice/other animals as described above (e.g. shControl, shSMS-1, and shSMS-2; see Embodiment C, above; what is said there may apply also here, *mutatis mutandis*). Then the AAV vectors, for example pAAV-Syn1-shControl-EGFP, pAAV-hSyn1-shSMS-1-T2A-EGFP, and pAAV-hSyn1-shSMS-2-T2A-EGFP, may be ordered to VectorBuilder.

To generate a lack of SMS (or a modification of (an)other enzyme(s) of the polyamine metabolism), a knockdown may be performed. Alternatively, long-term maintained spontaneously synchronously active (human) neuronal cultures may be used. This may result in itself to a significant reduction of, for example, SMS expression. These systems may also be used for screening. Measuring neuronal activity may be performed with any suitable method that assesses neuronal spiking. For example, microelectrode approaches, such as MEAs, may be used. These enable semi-automated, automated or high-throughput measurements. All those techniques provide respective testing devices. Semi-automated, automated or high-throughput approaches, and the respective testing devices, are particularly useful in primarily identifying compounds as the NaVCBs in accordance with the invention, for example from compound libraries. In principle, the particulars and steps pertaining to Embodiment D are essentially identical to the respective particulars and steps as described in the context of Embodiment C, above, or in the context of Example 10 (what is said there may apply also here, *mutatis mutandis*).

An exemplary, non-limiting detailed protocol of the method of screening of Embodiment D may be established on the basis of the description of Embodiment C, above. One or more respective particular step(s) may be performed in this respect (preferably in the described order); with any or several of the respective particular details.

Embodiment E relates to a method of screening for a NavCB by using an *in vivo* model (mouse or another animal). In this context, unconditional or, preferably, conditional knockout of SMS to generate an *in vivo* loss-of-function model may be generated (or unconditional/conditional knockout of (an)other enzyme(s) of the polyamine metabolism). Knockout (for example of SMS) may be combined with an epilepsy model (or with another NHD/Na$_V$CHD model); for example, an epilepsy model (or another NHD/Na$_V$CHD model) which is used for preclinical or clinical screening and/or validation of NavCBs/ASMs. This combination converts respective epilepsy/NHD models (for example for PRE or for other PRNHDs) to models for screening for NHDs which overcome the pharmacoresistance mechanism. So-screened NavCBs may be used for treating PRNHDs, in particular PRNavCHDs, in accordance with the present the invention. A non-limiting example of such a screening method is described in appended Example 11.

Male Gyro mice lacking SMS due to a deletion of part of the X chromosome are viable on the B6C3H background. Such mice may, in principle, be used as models in the context of the screening methods of Embodiment E. However, the use of such mice is less preferred. More preferably, conditional knockout mice/animals may thus be used. In such mice/animals, the effects can be seen more directly. For example, any conditional knockout mouse line may be used, including the following:

- C57BL/6J/Sms$^{em6Lutzy}$/J (Strain #: 032064; The Jackson Laboratory);
- CS7BL/6Smoc-$^{SMSem1(flox)Smoc}$ (Cat. No. NM-CKO-2117943, Shanghai Model Organisms); or
- C57BL/6JGpt-Sms-flox (T016254, GemPharmatech Co., Ltd.).

Some particulars pertaining to this may be as described in the context of step 1 of the Embodiment C, above, or as described in the context of Example 9 (what is said there may apply also here, *mutatis mutandis*). The (SMS) knockout/modification approach may also be used with any other *in vivo* (PR)epilepsy/(PR)NavCHD model.

The respective targeting sequences of SMS-shRNAs (or of shRNAs which target (an)other enzyme(s) of the polyamine metabolism) may be constructed and synthesized in the same way as the constructs as described above (see, for example, shControl, shSMS-1, and shSMS-2; cf. Embodiment C; what is said there may apply also here, *mutatis mutandis*). Further, crossing with appropriate Cre driver lines may be applied. This allows generation of inducible loss-of-function of SMS (or of (an)other enzyme(s) of the polyamine metabolism). In general, standard procedures for *in vivo* models may be applied in the context of Embodiment E.

The knockout/modification models (e.g. SMS knockout models) may be used in conjunction with tests which are widely used for screening for novel NavCBs (see, for example, Kehne, Neurochemical research 42 (7), 2017, 1894-1903). For instance, the following tests may be applied in this respect:

- an electrical stimulation test (for example the 6 Hz electrical stimulation test);
- a maximal electroshock test/model;
- a corneal kindling test and another kindling test/model;
- a spontaneous bursting slice test/model, for example based on post-kainic acid models;

All those techniques and test/models provide also respective testing devices.

In principle, at least some particulars and steps pertaining to Embodiment E may be essentially identical to the respective particulars and steps as described in the context of Embodiment C, above, or in the context of Example 10 (what is said there may apply also here, *mutatis mutandis*).

The here described method of screening for a NavCB by using an *in vivo* model is particularly useful as a high-reliability screening method; for example as a confirmatory testing whether an $Na_V$CB as identified according to a screening method for primarily identifying compounds as the $Na_V$CBs in accordance with the invention, for example from compound libraries (by, for example, semi-automated, automated or high-throughput approaches), inhibits/blocks (the activity of) a functional $Na_V$C in the presence of an increased level of N1aSPD (and/or in the presence of an increasing ratio of the levels of N1aSPD/N1aSPM). The here described method of screening for a $Na_V$CB by using an *in vivo* model may therefore be applied in combination with a (preceding) screening method for a NavCB in a semi-automated, automated or high-throughput mode (such as those screening methods as described in the context of the Embodiments A to D, above).

An exemplary, non-limiting detailed protocol of the method of screening of Embodiment E may be established on the basis of the above, and also of the description of Embodiment B or C, above. One or more respective particular step(s) may be performed in this respect (preferably in the described order); with any or several of the respective particular details.

The present invention further relates to the following items:

1. A method of screening for a voltage-gated sodium (Nav) channel blocker (NavCB), wherein said method comprises (the step of) contacting a test compound with a functional Nav channel in the presence of an increased level of N1-acetylspermidine (N1aSPD), and
wherein said test compound is identified as said NaVCB if it inhibits/blocks (the activity of) said functional Nav channel in the presence of said increased level of N1aSPD.

2. A method of screening for an $Na_V$CB which is pharmacologically effective in the treatment of a pharmacoresistant neuronal hyperexcitability disease/disorder (PRNHD), in particular in the treatment of a pharmacoresistant Nav channel-hyperactivity disease/disorder (PRNavCHD),

wherein said method comprises (the step of) contacting a test compound with a functional Nav channel in the presence of an increased level of N1-acetylspermidine (N1aSPD), and
wherein said test compound is identified as said NavCB if it inhibits/blocks (the activity of) said functional Nav channel in the presence of said increased level of N1aSPD.

3. The method of screening of item 1 or 2, wherein said (step of) contacting is in the presence of an increased ratio of the levels of N1aSPD/N1aSPM, and
wherein said test compound is identified as said NavCB if it inhibits/blocks (the activity of) said functional Nav channel in the presence of said increased ratio of the levels of N1aSPD/N1aSPM.

4. The method of screening of any one of items 1 to 3, wherein said increased level of N1aSPD and/or said increased ratio of the levels of N1aSPD/N1aSPM is so that a control NavCB (e.g. carbamazepine (CBZ); or another $Na_V$CB which is pharmacologically ineffective in the treatment of a PRNHD, like a pharmacoresistant epilepsy (PRE)) shows a reduced inhibition/blocking of said functional $Na_V$ channel in the presence of said increased level of N1aSPD and/or said increased ratio of the levels of N1aSPD/N1aSPM (as compared to a control/normal level of N1aSPD and/or control/normal ratio of the levels of N1aSPD/N1aSPM; and/or as compared to a control NavCB which is effective in the treatment of a PRNHD, like a PRE (e.g. oxcarbazepine (OXC)), i.e. in the presence of said increased level of N1aSPD and/or said increased ratio of the levels of N1aSPD/N1aSPM).

5. The method of screening of any one of items 1 to 4, wherein said increased level of N1aSPD and/or said increased ratio of the levels of N1aSPD/N1aSPM equals the respective level(s) in a (cell, neuronal culture system or animal) model of PRNHD (e.g. in the pilocarpine epilepsy model or the SMS knockout mouse models, C57BL/6J/Sms[em6-Lutzy]/J, C57BL/6Smoc-[SMSem1(flox)Smoc] or C57BL/6JGpt-Sms-flox) or is increased as compared to a (cell, neuronal culture system or animal) model of pharmacoresponsive NHD.

6. The method of screening of any one of items 1 to 5, wherein said increased level of N1aSPD and/or said increased ratio of the levels of N1aSPD/N1aSPM is increased by ≥2-fold, ≥3-fold, ≥4-fold, ≥5-fold, ≥6-fold, ≥7-fold, ≥8-fold, ≥9-fold, ≥10-fold, ≥11-fold, ≥12-fold, ≥13-fold, ≥14-fold, ≥15-fold, ≥20-fold, ≥25-fold or ≥30-fold (as compared to a control/normal level; e.g. 2- to 15-fold, 3- to 11-fold or 4- to 10-fold) and/or is increased by ≥10%, ≥20%, ≥30%, ≥40%, ≥50%, ≥60%, ≥70%, ≥80%, ≥90% or ≥100% (as compared to a control/normal level).

7. The method of screening of any one of items 1 to 6, wherein

(i) said increased level of N1aSPD is ≥0.5 µM, ≥1 µM, ≥10 µM, ≥20 µM, ≥30 µM, ≥50 µM, ≥75 µM, ≥100 µM, ≥150 µM or ≥200 µM (said increased level of N1aSPD may be in the range of 50 µM to 2 mM, in the range of 75 µM to 1.5 mM, in the range of 100 µM to 1.5 mM, in the range of 100 µM to 1.4 mM, in the range of 100 µM to 1.3 mM or in the range of 100 µM to 1.2 mM; preferably in the range of 100 µM to 1.1 mM; more preferably in the range of 100 µM to 1 mM); and/or

(ii) said increased ratio of the levels of N1aSPD/N1aSPM is >0.4, ≥0.5, ≥0.6, ≥0.7, ≥0.8, ≥0.9 or ≥1.0; or >1.1, ≥1.2, ≥1.3, ≥1.4, ≥1.5, ≥1.6, ≥1.7, ≥1.8, ≥1.9 or ≥2.0.

8. The method of screening of any one of items 1 to 7, wherein said functional Nav channel is located in a phospholipid bilayer membrane.

9. The method of screening of any one of items 1 to 8, wherein said functional Navchannel is located in the cell membrane of a cell.

10. The method of screening of any one of items 1 to 9, wherein said increased level of N1aSPD and/or said increased ratio of the levels of N1aSPD/N1aSPM is present at the intracellular side of said functional $Na_V$ channel, e.g. in the lumen of a/said cell (intracellularly).

11. The method of screening of any one of items 1 to 10, wherein, in the context of said (step of) contacting said test compound with said functional Nav channel, said test compound is contacted with the extracellular side of said functional Nav channel, e.g. from the outside of a/said cell (extracellularly).

12. The method of screening of any one of items 1 to 11, which is an *in vitro, ex vivo* or an *in vivo* method.

13. The method of screening of any one of items 1 to 12, wherein, in the context of said (step of) contacting said test compound with said functional Nav channel, said test compound is contacted with a cell which expresses said functional Nav channel in its cell membrane.

14. The method of screening of any one of items 1 to 13, wherein, in the context of said (step of) contacting said test compound with said functional Nav channel, said test compound is contacted with a cell in which said increased level of N1aSPD and/or said increased ratio of the levels of N1aSPD/N1aSPM occur(s) intracellularly (cell model for PRNHD/PRNavCHD).

15. The method of screening of any one of items 9 to 14, wherein said cell is an animal cell or, preferably, a human cell; or is derived from an animal cell line or, preferably, from a human cell line.

16. The method of screening of any one of items 9 to 15, wherein said cell is a Human Embryonic Kidney (HEK) cell (e.g. a HEK293 cell, like a HEK293T, HEK293F, HEK293FT or HEK293-AAV cell), a Chinese Hamster Ovary (CHO) cell, a xenopus oocyte, a neuron, a glia or a neuroglia, or a cell of a neuroblastoma cell line.

17. The method of screening of any one of items 9 to 15, wherein said cell is a (primary) neuron.

18. The method of screening of any one of items 1 to 12, wherein, in the context of said (step of) contacting said test compound with said functional Nav channel, said test compound is contacted with a (human) neuronal culture system (e.g. (human) iPSC-derived) which comprises cells which express said functional Nav channel in their cell membranes.

19. The method of screening of any one of items 1 to 12 and 18, wherein, in the context of said (step of) contacting said test compound with said functional Nav channel, said test compound is contacted with a (human) neuronal culture system (e.g. iPSC-derived) which comprises neuronal cells in which said increased level of N1aSPD and/or said increased ratio of the levels of N1aSPD/N1aSPM occur(s) intracellularly (complex neuronal culture system/tissue model for PRNHD/PRNavCHD).

20. The method of screening of any one of items 1 to 12, wherein, in the context of said (step of) contacting said test compound with said functional Nav channel, said test compound is contacted with an animal which comprises (neuronal) cells which express said functional Nav channel in their cell membranes.

21. The method of screening of any one of items 1 to 12 and 20, wherein, in the context of said (step of) contacting said test compound with said functional Nav channel, said test compound is contacted with an animal which comprises (neuronal) cells in which said increased level of N1aSPD and/or said increased ratio of the levels of N1aSPD/N1aSPM occur(s) intracellularly (animal model for PRNHD/PRNavCHD).

22. The method of screening of any one of items 9 to 21, wherein said cell is a recombinant (genetically engineered) cell, said neuronal culture system is a recombinant (genetically engineered) neuronal culture system or said animal is a recombinant (genetically engineered) animal.

23. The method of screening of any one of items 9 to 22, wherein said increased intracellular level of N1aSPD and/or said increased intracellular ratio of the levels of N1aSPD/N1aSPM is achieved by modifying the expression of at least one enzyme of the polyamine metabolism in said cell, in said neuronal culture system or in said animal.

24. The method of screening of any one of items 9 to 23, wherein said increased intracellular level of N1aSPD and/or said increased intracellular ratio of the levels of N1aSPD/N1aSPM is achieved by reducing the expression of the spermine synthase (SMS) in said cell, in said neuronal culture system or in said animal.

25. The method of screening of any one of items 20 to 24, wherein said animal is a model of a PRNHD/PRNavCHD (e.g. a model of a pharmacoresistant seizure disorder (PRSD)).

26. The method of screening of any one of items 20 to 25, wherein said animal is a model of PRE.

27. The method of screening of any one of items 20 to 26, wherein said animal is a model of chronic epilepsy.

28. The method of screening of any one of items 20 to 27, wherein said animal is a model of chronic temporal lobe epilepsy (cTLE).

29. The method of screening of any one of items 20 to 28, wherein said animal is a model of refractory epilepsy.

30. The method of screening of any one of items 20 to 29, wherein said animal is a model of acquired epilepsy.

31. The method of screening of any one of items 20 to 30, wherein said animal is a pilocarpine model of epilepsy.

32. The method of screening of any one of items 20 to 29, wherein said animal is a model of genetic epilepsy.

33. The method of screening of item 32, wherein said animal is a KCNT1 model of genetic epilepsy.

34. The method of screening of item 33, wherein said animal harbours the gain-of-function (GOF) missense mutation p.!335N in KCNT1 (KCNT1 p.I335N).

35. The method of screening of any one of items 20 to 30, wherein said animal is an SMS knockout animal.

36. The method of screening of item 35, wherein said animal is a conditional SMS knockout animal.

37. The method of screening of any one of items 20 to 36, wherein said animal is a mouse or a rat.

38. The method of screening of any one of items 35 to 37, wherein said animal is an SMS knockout mouse selected from the group consisting of C57BL/6J/Sms[em6Lutzy]/J (Strain #: 032064, The Jackson Laboratory), CS7BL/6Smoc-[SMSem1(flox)Smoc] (Cat. No. NM-CKO-2117943, Shanghai Model Organisms) and C57BL/6JGpt-Sms-flox (T016254, GemPharmatech Co., Ltd.).

39. The method of screening of any one of items 1 to 17 and 22, wherein said increased level of N1aSPD and/or said increased ratio of the levels of N1aSPD/N1aSPM is set/controlled by a control device.

40. The method of screening of item 39, wherein said increased level of N1aSPD and/or said increased ratio of the levels of N1aSPD/N1aSPM is set/controlled at the intracellular side of said functional $Na_V$ channel by said control device.

41. The method of screening of item 39 or 40, wherein said increased level of N1aSPD and/or said increased ratio of the levels of N1aSPD/N1aSPM is set/controlled in a/said cell by said control device.

42. The method of screening of any one of items 1 to 19 and 22 to 24, wherein for/in the context of said (step of) contacting said test compound with said functional Nav channel a contact device is used.

43. The method of screening of items 42, wherein said test compound is contacted with the extracellular side of said functional Nav channel by using said contact device.

44. The method of screening of any one of items 39 to 43, wherein said control or contact device is/comprises a micropipette or a vial.

45. The method of screening of any one of items 39 to 44, wherein said control or contact device is/comprises a patch-clamp device.

46. The method of screening of any one of items 39 to 45, wherein said control or contact device is/comprises a planar patch-clamp device.

47. The method of screening of any one of items 39 to 46, wherein said control or contact device is/comprises a patch-clamp micropipette or a patch-clamp chip.

48. The method of screening of any one of items 39 to 47, wherein said control device is/comprises a whole-cell patch-clamp device.

49. The method of screening of any one of items 39 to 47, wherein said control device is/comprises an outside-out patch-clamp device.

50. The method of screening of any one of items 42 to 47, wherein said contact device is/comprises a cell-attached patch-clamp device.

51. The method of screening of any one of items 42 to 47, wherein said contact device is/comprises a inside-out patch-clamp device.

52. The method of screening of any one of items 42 to 50, wherein for/in the context of said (step of) contacting said test compound with said functional Nav channel a vial is used in/as the contact device.

53. The method of screening of any one of items 42 to 44, wherein for/in the context of said (step of) contacting said test compound with said functional Nav channel a vial of a microelectrode or of a microelectrode array (MEA) plate is used in/as the contact device.

54. The method of screening of any one of items 42 to 47 and 51, wherein a vial is used in/as the control device for setting/controlling said increased level of N1aSPD and/or said increased ratio of the levels of N1aSPD/N1aSPM at the intracellular side of said functional Nav channel.

55. The method of screening of any one of items 1 to 54, wherein said method further comprises (the step of) testing whether, upon said (step of) contacting said test compound with said functional Nav channel, said NaVCB inhibits/-

blocks (said activity of) said functional Nav channel in the presence of said increased level of N1aSPD and/or said increased ratio of the levels of N1aSPD/N1aSPM.

56. The method of screening of any one of items 1 to 17, 22 to 24 and 39 to 54, wherein said method further comprises (the step of) testing by a test device whether, upon said (step of) contacting said test compound with said functional Nav channel, said NavCB inhibits/blocks (said activity of) said functional $Na_V$ channel in the presence of said increased level of N1aSPD and/or said increased ratio of the levels of N1aSPD/N1aSPM.

57. The method of screening of any one of items 1 to 17, 22 to 24, 39 to 54 and 56, wherein said method further comprises (the step of) testing in a/said cell by a test device whether, upon said (step of) contacting said test compound with said functional Nav channel, said NavCB inhibits/blocks (said activity of) said functional Nav channel in the presence of said increased level of N1aSPD and/or said increased ratio of the levels of N1aSPD/N1aSPM.

58. The method of screening of items 56 or 57, wherein said test device is/comprises a voltage-clamp device.

59. The method of screening of any one of items 56 to 58, wherein said test device is/comprises a patch-clamp device.

60. The method of screening of any one of items 56 to 59, wherein said test device is/comprises a planar voltage/patch-clamp device.

61. The method of screening of any one of items 56 to 60, wherein said test device is/comprises a voltage/patch-clamp micropipette or a voltage/patch-clamp chip.

62. The method of screening of any one of items 56 to 61, wherein said test device is/comprises a multichannel voltage/patch-clamp device.

63. The method of screening of any one of items 56 to 62, wherein said test device is/comprises a semi-automated, automated or high-throughput voltage/patch-clamp device (e.g. a Sophion Bioscience Qube system, like QPatch Compact, QPatch or Qube 384).

64. The method of screening of any one of items 56 to 63, wherein said test device is/comprises a whole-cell patch-clamp device.

65. The method of screening of any one of items 56 to 63, wherein said test device is/comprises an outside-out patch-clamp device.

66. The method of screening of any one of items 56 to 63, wherein said test device is/comprises a cell-attached patch-clamp device.

67. The method of screening of any one of items 56 to 63, wherein said test device is/comprises an inside-out patch-clamp device.

68. The method of screening of any one of items 56 to 65, wherein a control device as defined in any one of items 39 to 41 and 44 to 49 is also used as said test device.

69. The method of screening of any one of items 56 to 63, 66 and 67, wherein said contact device as defined in any one of items 42 to 47, 50 and 51 is also used as said test device.

70. The method of screening of any one of items 1 to 15, 17 to 19 and 39, 40, 42 to 44, 52 and 53, wherein said method further comprises (the step of) testing by a test device whether, upon said (step of) contacting said test compound with said functional Nav channel, said NavCB inhibits/blocks (said activity of) said functional Nav channel in the presence of said increased level of N1aSPD and/or said increased ratio of the levels of N1aSPD/N1aSPM.

71. The method of screening of any one of items 1 to 12, 18, 19, 22 to 24, 39, 40, 42 to 44, 52 and 53, wherein said method further comprises (the step of) testing in a neuronal culture system by a test device whether, upon said (step of) contacting said test compound with said functional $Na_V$ channel, said $Na_V$CB inhibits/blocks (said activity of) said functional $Na_V$ channel in the presence of said increased level of N1aSPD and/or said increased ratio of the levels of N1aSPD/N1aSPM.

72. The method of screening of 70 or 71, wherein said test device is/comprises a microelectrode.

73. The method of screening of any one of items 70 to 72, wherein said test device is/comprises a microelectrode array (MEA).

74. The method of screening of any one of items 70 to 73, wherein said test device is/comprises a microelectrode array (MEA) plate.

75. The method of screening of any one of items 70 to 74, wherein said contact device as defined in any one of items 42 to 44 and 53 is also used as said test device.

76. The method of screening of any one of items 1 to 12 and 20 to 38, wherein said method further comprises (the step of) testing whether, upon said (step of) contacting said test compound with said functional $Na_V$ channel, said NaVCB inhibits/blocks (said activity of) said functional Nav channel in the presence of said increased level of N1aSPD and/or said increased ratio of the levels of N1aSPD/N1aSPM.

77. The method of screening of any one of items 1 to 12, 20 to 38 and 76, wherein said method further comprises (the step of) testing in a/said animal whether, upon said (step of) contacting said test compound with said functional Nav channel, said NavCB inhibits/blocks (said activity of) said functional $Na_V$ channel in the presence of said increased level of N1aSPD and/or said increased ratio of the levels of N1aSPD/N1aSPM.

78. The method of screening of item 77, wherein said (step of) testing comprises

(a) an electrical stimulation (e.g. at 6 Hz);
(b) the use of an electroshock model (like, for example, a maximal electroshock model);
(c) the use of a kindling model (like, for example, a corneal kindling model) and/or kindling testing (like, for example, corneal kindling testing); and/or
(d) the use of a sspontaneous bursting slice model from (a) post-kainic acid model(s).

79. The method of screening of any one of items 1 to 78, wherein said functional Nav channel is a $Na_V1.1$ channel, $Na_V1.2$ channel, $Na_V1.3$ channel, $Na_V1.4$ channel, $Na_V1.5$ channel, $Na_V1.6$ channel, $Na_V1.7$ channel, $Na_V1.8$ channel or $Na_V1.9$ channel; preferably a $Na_V1.2$ channel or a $Na_V1.6$ channel.

80. The method of screening of any one of items 1 to 79, wherein said NavCB is a NHD medication.

81. The method of screening of item 80, wherein said NHD medication is an antiseizure medication (AMS), an antiepilepsy medication (AEM; sometimes also termed antiepileptic drug (AED)) or a medication for a hyperexcitability disorder.

82. The method of screening of item 81, wherein said hyperexcitability disorder is a neurodegenerative or neurological disorder, such as a pain disorder/pain-related disorder (e.g. neuropathic pain; hereditary pain syndromes etc.), trigeminal neuralgia, postherpetic neuralgia, diabetic neuropathy, Alzheimers disease (AD) or stroke.

83. The method of screening of any one of items 2 to 80, wherein said PRNHD is a pharmacoresistant seizure disorder (PRSD) or a pharmacoresistant hyperexcitability disorder (PRHD).

84. The method of screening of item 83, wherein said PRSD is a pharmacoresistant epilepsy (PRE).

85. The method of screening of item 83, wherein said PRSD is a pharmacoresistant form of a hyperexcitability disorder as defined in item 82.

86. The method of screening of any one of items 1 to 85, which is a high-throughput method of screening/operated in a high-throughput mode.

87. A two-step method of screening for an NaVCB comprising

(i) (a first step of) operating a method of screening for an NavCB according to any one of items 1 to 19, 39 to 75 and 79 to 86 (in vitro or ex vivo method/use of a cell or neuronal culture system model);
(ii) (a second step of) operating a method of (confirmatory) testing whether the NaVCB as identified according to (i) inhibits/blocks (the activity of) a functional Nav channel in the presence of said increased level of N1aSPD according to a method of screening of any one of items 1 to 12, 20 to 38, 55 and 76 to 86 (in vivo method/use of an animal model).

88. The two-step method of screening of item 87, wherein (i) is operated in a semi-automated, automated or high-throughput mode; and/or (ii) is operated in a low capacity/high-reliability mode.

89. The two-step method of screening of item 87 or 88, wherein (i) is operated in a patch-clamp- or MEA-based mode (e.g. as defined in any one of items 45 to 78); and/or (ii) is based on the use of an animal model (e.g. as defined in any one of items 20 to 38).

90. An NaVCB as identified according to a method of screening according to any one of items 1 to 89.

91. A pharmaceutical composition comprising the NaVCB of item 90.

92. The pharmaceutical composition of item 91 which is for use in treating a NHD.

93. The pharmaceutical composition of item 92 wherein said NHD is defined as in item 81 or 82.

94. The pharmaceutical composition of item 92 or 93 wherein said NHD is a seizure disorder.

95. The pharmaceutical composition of any one of items 92 to 94, wherein said NHD is an epilepsy.

96. The pharmaceutical composition of any one of items 91 to 95 for use in treating a seizure.

97. The pharmaceutical composition of any one of items 91 to 96 for use in treating a seizure in/coming along with epilepsy.

98. The pharmaceutical composition of any one of items 95 to 97, wherein said epilepsy is defined as in any one of items 26 to 30 and 32 to 34.

99. The pharmaceutical composition of any one of items 91 to 98, for use in treating a PRNHD.

100. The pharmaceutical composition of item 99, wherein said PRNHD is a pharmacoresistant form of a disease or disorder as defined in any one of items 81, 82 and 94 to 98.

101. The pharmaceutical composition of item 99 or 100 wherein said PRNHD is defined in any one of items 83 to 85.

102. A $Na_VCS$ which inhibits/blocks (the activity of) a functional $Na_V$ channel in the presence of an increased level of N1aSPD; and/or which inhibits/blocks (the activity of) a functional Nav channel in the presence of an increased ratio of the levels of N1aSPD/N1aSPM.

103. A pharmaceutical composition comprising the NavCB of item 102.

104. The NavCB of item 102 or the pharmaceutical composition of item 103 for use in treating a NHD, in particular a PRNHD.

105. A pharmaceutical composition comprising oxcarbazepine (OXC) and/or Eslicarbazepine acetate/Eslicarbazepine (ESL) for use in treating a PRNHD.

If not indicated differently herein elsewhere or contradicted by context, "inhibiting", "decreasing", "blocking", "suppressing" or "reducing" and the like, in the context of the present invention is envisaged to mean that an initial status (for example initial status of NavC function/activity, SMS expression/activity, expression/activity of (an) other enzyme(s), efficacy of test compound/ $Na_V$CB etc.) is lowered *(in vitro* and/or *in vivo)* by, for example, at least 10%, by at least 20%, by at least 30%, by at least 50%, by at least 80%, by at least 90%, at least 95%, by at least 99% or even by 100% (in principle, the higher values of percentage are preferred). The skilled person is readily in the position to test the respective degree of "inhibiting", "decreasing", "blocking", "suppressing" or "reducing".

Likewise, if not indicated differently herein elsewhere or contradicted by context, "increasing", "inducing" or "improving", and the like, in the context of the present invention particularly means that the initial status (for example status of NavC function/activity, SMS expression/activity, expression/activity of (an) other enzyme(s), efficacy of test compound/ $Na_V$CS etc.) is increased *(in vitro* and/or *in vivo)* by, for example, at least 10%, by at least 20%, by at least 30%, by at least 50%, by at least 80%, by at least 90%, at least 95%, by at least 99% or by at least 100%; or by at least 2, 3, 4, 5, 6, 7, 8, 9 or 10-fold (in principle, the higher values of percentage are preferred). In particular, "increasing" means that there is already an initial degree of activity/status (baseline) which is further "increased". In particular, "inducing" means that there is (substantially) no initial degree of activity/status which is then "induced". The skilled person is readily in the position to test the respective degree of "increasing", "inducing" or "improving".

If not explicitly indicated differently herein elsewhere, terms like "about", "similar", "essentially" mean at most +/- 5%, +/- 3%, +/- 2% or +/- 1% of the respective reference/control point (e.g. reference/control value). It is preferred, however, that the deviation from the reference point is as low as possible.

The present invention is further described by reference to the following non-limiting figures and examples.

The Figures show:

**Figure 1. ASM effects on sodium channels are controlled by $N_1$-acetylspermine and $N_1$-acetylspermidine. a,** Diagram of polyamine metabolism and major conversion pathways. **b,** Protocol used to study effects on recovery from fast inactivation. Original traces of recovery from fast inactivation generated by the double pulse voltage protocol shown on top right. Recovery from fast inactivation is shown for bath solution (black, n = 10, mean $\pm$ SEM) and 100 $\mu$M CBZ (dotted line, lower left, n = 10, mean $\pm$ SEM). The magnitude of CBZ effects was quantified by subtracting both curves (bell shaped curve, lower right) for all experiments. **c,** Effects of 100 $\mu$M intracellular spermine or spermidine on CBZ efficacy (Bath n = 10, Spermidine (SPD) n = 7, Spermine (SPM) n = 6, mean $\pm$ SEM) ( Bath vs. SPD, p = 0.20, Bath vs. SPM, p = 0.67, one-way ANOVA with Dunnett's multiple comparisons test). No significant effects were seen. **d,** Effects of $N_1$-acetylspermine and $N_1$-acetylspermidine at two different concentrations on CBZ efficacy (Bath n = 10, Nacetyl-SPD 1mM n = 6, Nacetyl-SPD 100 $\mu$M n = 8, Nacetyl-SPM 1mM n = 6, Nacetyl-SPM 100 $\mu$M n = 6, mean $\pm$ SEM). **e-f,** Effects of oxcarbazepine (100 $\mu$M, bath n = 9, Nacetyl-SPD n = 9, Nacetyl-SPM n = 9, mean $\pm$ SEM) and lamotrigine (100 $\mu$M, bath n = 11, Nacetyl-SPD n = 7, Nacetyl-SPM n = 7, mean $\pm$ SEM) in the presence of $N_1$-acetyl-polyamines. g, Overall magnitude of the effects of acetyl-polyamine variants (difference in CBZ effects for 1 mM $N_1$-acetylspermidine on the one hand, and 1 mM $N_1$-acetylspermine on the other hand, indicating how prominently the acetylpolyamine modulation is) (CBZ, n = 6, 19.0 $\pm$ 2.31, OXC, n = 9, 7.96 $\pm$ 1.83, ESL, n = 10, 7.83 $\pm$ 2.76, LTG, n = 6, 13.9 $\pm$ 2.07, mean $\pm$ SEM. CBZ vs. OXC *p = 0.011, CBZ vs. ESL **p = 0.0087, CBZ vs. LTG p = 0.40, one-way ANOVA with Dunnett's multiple comparisons test, *p < 0.05, **p < 0.01).

**Figure 2. Expression system-based model replicating pharmacoresistance via down-regulation of SMS. a,** siRNA mediated inhibition of SMS expression (Nav1.2, Control n = 11, 1.0 $\pm$ 0, SMS1 n=11, 0.54 $\pm$ 0.031, SMS2 n=11, 0.57 $\pm$ 0.035, Control vs. SMS1 ****p < 0.0001, Control vs. SMS2 ****p < 0.0001, Nav1.6, Control n = 8, 1.0 $\pm$ 0, SMS1 n=8, 0.44 $\pm$ 0.059, SMS2 n=8, 0.46 $\pm$ 0.040, Control vs. SMS1 ****p < 0.0001, Control vs. SMS2 ****p < 0.0001, mean $\pm$ SEM, one-way ANOVA with Dunnett's multiple comparisons test). **b,** Altered spermine-spermidine ratio after siRNA mediated inhibition of SMS expression (SPD/SPM in HEK cells expressing Nav1.2, Control n = 0.39 $\pm$ 0.013, SMS1 n=8, 0.48 $\pm$ 0.014, SMS2 n=8, 0.56 $\pm$ 0.036, Control vs. SMS1 *p = 0.024, Control vs. SMS2 ***p = 0.0001, mean $\pm$ SEM, one-way ANOVA with Dunnett's multiple comparisons test, *p < 0.05, ***p < 0.001) **c,** siRNAs do not affect sodium channel alpha subunit expression. **d,** Recovery of SMS expression in a rescue experiment. **e, f,** CBZ sensitivity of sodium channels after siRNA treatment and rescue, respectively, in cell lines expressing $Na_V$1.2 **(e.** Control siRNA, n = 10, Without siRNA, n = 10, SMS1 siRNA, n = 11, SMS2 siRNA, n=10, mean $\pm$ SEM) **(f.** Control + Mock, n = 8, SMS1 + Mock, n = 8, SMS2 + Mock, n = 8, mean $\pm$ SEM). **g,** CBZ sensitivity of sodium channels after siRNA treatment in cell lines expressing $Na_V$1.6 (Control siRNA, n = 11, Without siRNA, n = 10, SMS1 siRNA, n = 9, mean $\pm$ SEM).

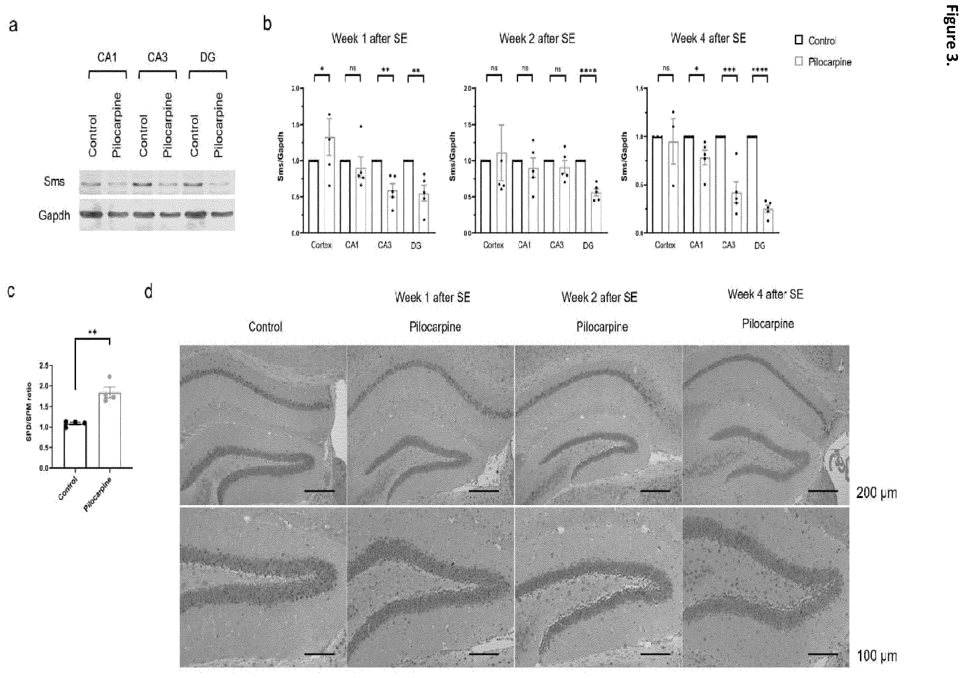

**Figure 3. Regulation of SMS in a chronic temporal lobe epilepsy (cTLE) model (pilocarpine model of epilepsy). a, b,** Western blot analysis of SMS protein expression in hippocampal subfields. **a,** Western blot, hippocampal microslices of hippocampal subregions CA1, CA3, and dentate gyrus (DG), as well as neocortex. **b,** Quantification of Western blots (Week 1, Cortex, Control n = 5, $1.0 \pm 0$, Pilocarpine n = 5, $1.33 \pm 0.26$, CA1, Control n = 5, $1.0 \pm 0$, Pilocarpine n = 5, $0.91 \pm 0.14$, CA3, Control n = 5, $1.0 \pm 0$, Pilocarpine n = 5, $0.59 \pm 0.091$, DG, Control n = 5, $1.0 \pm 0$, Pilocarpine n = 5, $0.55 \pm 0.11$, Cortex p = 0.23, CA1 p = 0.54, CA3 **p = 0.0021, DG **p = 0.0034, mean $\pm$ SEM, two-tail unpaired t-test, **p < 0.01) (Week 2, Cortex, Control n = 5, $1.0 \pm 0$, Pilocarpine n = 5, $1.11 \pm 0.38$, CA1, Control n = 5, $1.0 \pm 0$, Pilocarpine n = 5, $0.90 \pm 0.14$, CA3, Control n = 5, $1.0 \pm 0$, Pilocarpine n = 5, $0.91 \pm 0.092$, DG, Control n = 5, $1.0 \pm 0$, Pilocarpine n = 5, $0.56 \pm 0.050$, Cortex p = 0.78, CA1 p = 0.50, CA3 p = 0.37, DG ****p < 0.0001, mean $\pm$ SEM, two-tail unpaired t-test) (Week 4, Cortex, Control n = 5, $1.0 \pm 0$, Pilocarpine n = 3, $0.95 \pm 0.23$, CA1, Control n = 5, $1.0 \pm 0$, Pilocarpine n = 6, $0.77 \pm 0.065$, CA3, Control n = 5, $1.0 \pm 0$, Pilocarpine n = 6, $0.55 \pm 0.16$, DG, Control n = 5, $1.0 \pm 0$, Pilocarpine n = 6, $0.31 \pm 0.062$, Cortex p = 0.84, CA1 *p = 0.010, CA3 *p = 0.031, DG ****p < 0.0001, mean $\pm$ SEM, two-tail unpaired t-test, *p < 0.05). **c,** Spermine (SPM) and spermidine (SPD) levels as well as ratios of both in the hippocampus of the pilocarpine model of epilepsy (SPD/SPM ratio, Control n = 4, $1.08 \pm 0.033$, Pilocarpine n = 4, $1.84 \pm 0.13$, **p = 0.0013, mean $\pm$ SEM, two-tail unpaired t-test). **d,** Immunohistochemistry for SMS in the hippocampus of the pilocarpine model of epilepsy.

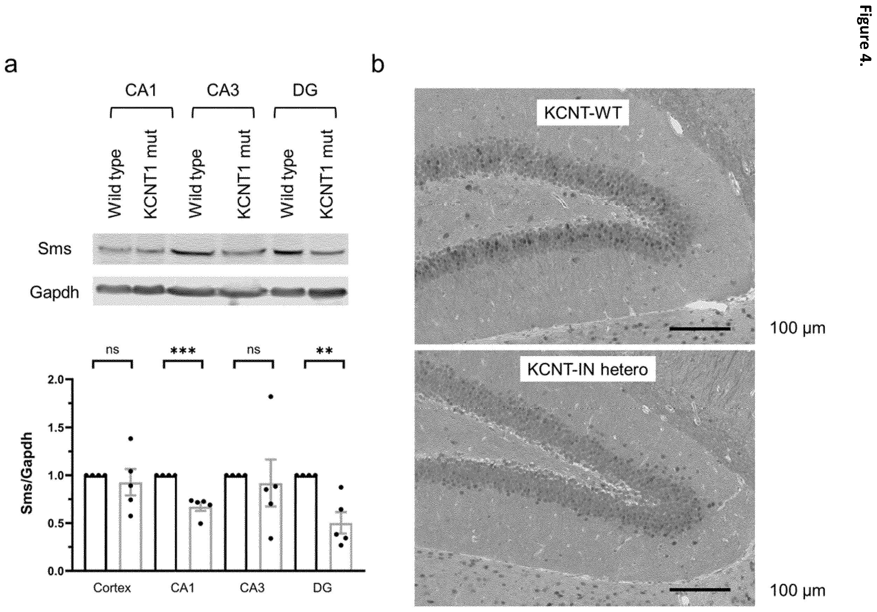

**Figure 4. Regulation of SMS in** a **genetic epilepsy model** *(KCNT1 p.I335N mutation)*. **a, b,** Western blot analysis of SMS protein expression in hippocampal subfields. **a,** Western blot, hippocampal microslices of hippocampal subregions CA1, CA3, and dentate gyrus (DG), as well as neocortex (Cortex, WT n = 4, $1.0 \pm 0$, Mut n = 5, $0.93 \pm 0.14$, CA1, WT n = 4, $1.0 \pm 0$, Mut n = 5, $0.67 \pm 0.045$, CA3, WT n = 4, $1.0 \pm 0$, Mut n = 5, $0.92 \pm 0.25$, DG, WT n = 4, $1.0 \pm 0$, Mut n = 5, $0.50 \pm 0.11$, Cortex p = 0.66, CA1 ***p = 0.0004, CA3 p = 0.78, DG **p = 0.0059, mean $\pm$ SEM, two-tail unpaired t-test, **p < 0.01, ***p < 0.001). Bargraph shows quantification in wild-type littermates and mice heterozygous for the **p.I335N** mutation. **b,** Immunohistochemistry for SMS in the hippocampus of KCNT1 wild-type littermate control mice and mice heterozygous for the **p.I335N** mutation.

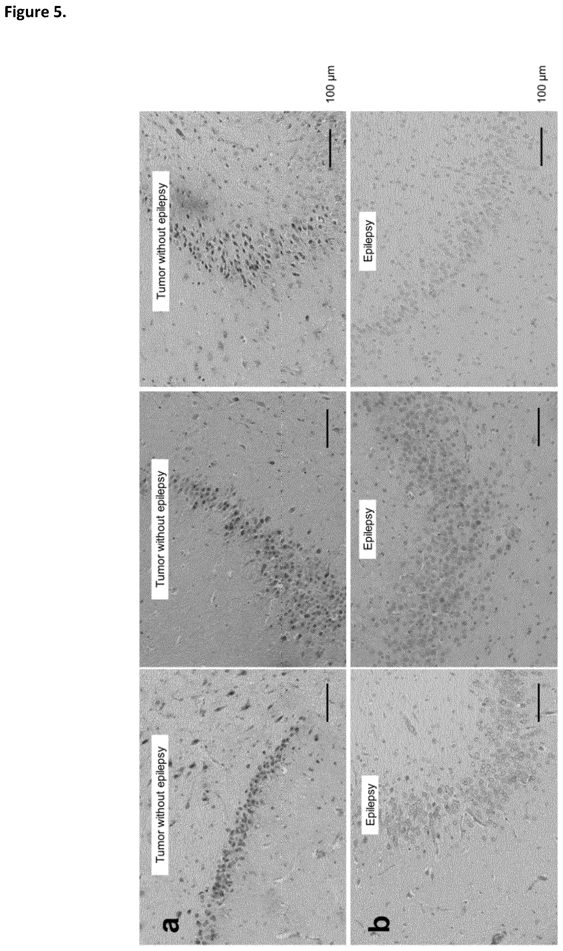

**Figure 5. Regulation of SMS in human pharmacoresistant epilepsy vs non-epileptic subjects. a,** Immunohistochemistry for SMS in the dentate gyrus (DG) of patient's material from non-epileptic tumor-related resections, three representative examples from different patients. **b,** the same for three representative patients with pharmacoresistant epilepsy. There is a clear reduction of SMS staining in all patients with pharmacoresistant epilepsy (Tumor without epilepsy, n = 3, Hippocampal sclerosis (HS), n = 3).

**Figure 6. Patch-clamp measurements allow control of intracellular milieu.** Left: Standard patch-clamp configuration with a glass pipette from a single HEK cell expressing sodium channels. In this variant, polyamines at known concentration can be added to the pipette intracellular solution. Right panel: In multichannel patch-clamp approaches, i.e. with the Qube system produced by Sophion Bioscience, an intracellular solution exchange is also possible.

**Figure 7. Summary of exemplary stepwise screening protocols.** Panel a: exemplary stepwise screening protocol for HEK cell-based screening with modified intercellular solutions (as applied in the context of Example 7; may be applied, for example, in the context of Embodiment A described below). Panel b: exemplary stepwise screening protocol for HEK cell-based screening with genetically modified HEK cell line (RNAi technique; SMS knockdown; as applied in the context of Example 8; may be applied, for example, in the context of Embodiment B described below).

**Figure 8. Chemical structure of N$_1$-Acetylspermidine (N$_1$-aSPD) and N$_1$-Acetylspermine (N$_1$-aSPM).**

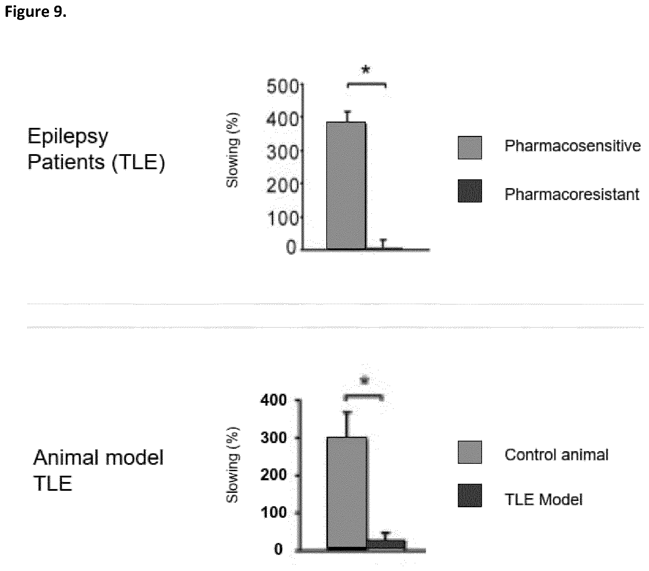

**Figure 9. Demonstration of the loss of effect of CBZ in pharmacoresistant human and experimental epilepsy.** Quantified is the slowing of the recovery from inactivation by CBZ (% increase in recovery time constant) for temporal lobe epilepsy (TLE) patients and for a TLE animal model. Left columns, pharmacosensitive patients/control animals; right columns, pharmacoresistant patients/experimental epilepsy model (pilocarpine model of epilepsy). Adapted from Remy 2003 *loc. cit.*

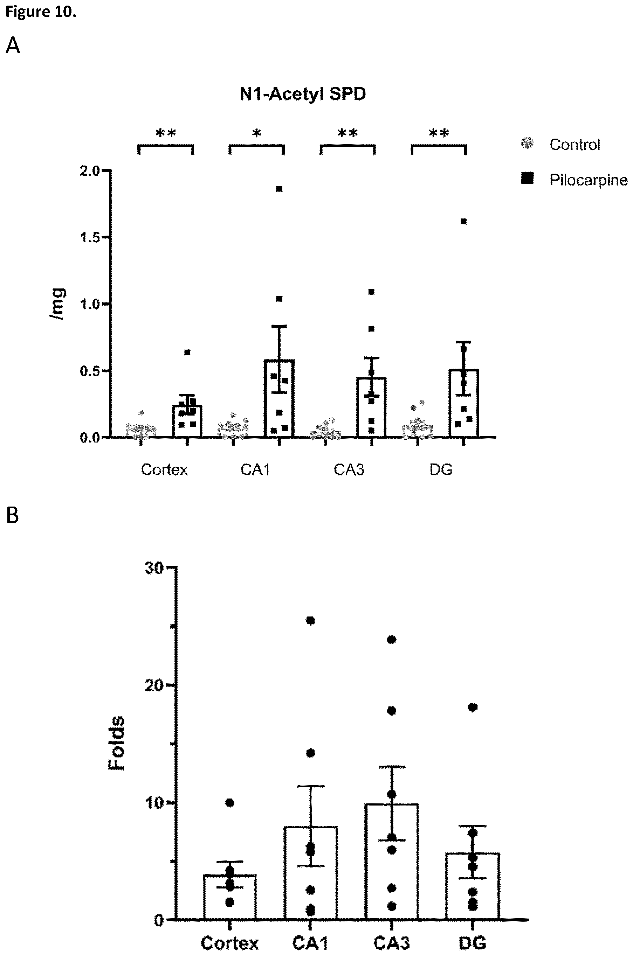

**Figure 10. Quantification of N$_1$-acetylspermidine using LC-MS in hippocampal tissue derived from control animals and the pilocarpine model of epilepsy.** Panel A shows the N$_1$-acetylspermidine content in pmol per mg wet weight. Panel B shows the average fold increase in N$_1$-acetylspermidine content in epileptic mice vs. control mice. Cortex: $3.87 \pm 1.10$; CA1: $7.99 \pm 3.40$; CA3: $9.89 \pm 3.12$; DG: $5.76 \pm 2.22$.

**Figure 11. Diagram showing variations of the patch-clamp technique (patch-clamp modes).** A micropipette is used as an exemplary contact, control and/or testing device. The first image depicts the initial situation; the other images show how the different patch-clamp modes can be achieved when starting from the initial situation. The cell may be placed in a vial as the respective contact or control device (depending on the particular patch-clamp mode applied). The image is extracted from https://en.wikipedia.org/wiki/Patch_clamp.

In the foregoing detailed description of the invention, a number of individual elements, characterizing features, techniques and/or steps are disclosed. It is readily recognized that each of these has benefit not only individually when considered or used alone, but also when considered and used in combination with one another. Accordingly, to avoid exceedingly repetitious and redundant passages, this description has refrained from reiterating every possible combination and permutation. Nevertheless, whether expressly recited or not, it is understood that such combinations are entirely within the scope of the presently disclosed subject matter.

All technical and scientific terms used herein, unless otherwise defined, are intended to have the same meaning as commonly understood by one of ordinary skill in the art. Reference to techniques employed herein are intended to refer to the techniques as commonly understood in the art, including variations on those techniques or substitutions of equivalent techniques that would be apparent to one of skill in the art.

In this specification, a number of documents including patent applications are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Further, reference is made herein to the following RNA sequences (and also to the respective coding nucleotide sequences (DNA)):

| **SEQ ID NO. 1:** *SMS* **siRNA-set1-S1** | **SEQ ID NO. 2:** *SMS* **siRNA-set2-S2** |
|---|---|
| 5'-CGACAGUAUUUUGAACAAA-3' | 5'-GAUUCACCUUAUCAAAAUA-3' |

The invention will now be described by reference to the following examples which are merely illustrative and are not to be construed as a limitation of the scope of the present invention.

**Example 1: Materials and Methods**

*Polyamine synthesis inhibitors.* In some experiments in HEK293T cells, polyamine synthesis was blocked (data not shown). In this context, sardomozide hydrochloride (SAM486A, CGP 48664) and DL-$\alpha$-difluoromethylornithine (hydrochloride hydrate, DFMO) were applied. Twenty-four hours after the last passage, 3 $\mu$M SAM486A and 3 mM DFMO were added to the culture medium. Analyses were performed after 24 hours of incubation.

*Measurement of polyamines in HEK cells (putrescine, spermidine, spermine) by ultra-high performance liquid chromatography (uHPLC).* Polyamine content in HEK cells was measured using uHPLC (Fig. 2b). For the polyamine assay, the HEK cells were treated with 300 $\mu$L volume 4% perchloric acid (244252, Sigma-Aldrich) and sonicated (40 W, 60 s, UP50H, Dr. Hielscher GmbH) on ice, followed by extraction of polyamines overnight at 4°C. After centrifugation at 10,000 $g$ for 20 min, 150 $\mu$L of the supernatants were mixed with 500 $\mu$L of 2N NaOH (109913, Sigma-Aldrich) and 3 $\mu$L of benzoyl chloride (259950, Sigma-Aldrich). After incubation for 20 min at 23°C, the reaction was stopped by the addition of 800 $\mu$L of a saturated NaCl solution (S7653, Sigma-Aldrich). Polyamines were extracted in 500 $\mu$L chloroform (34854, Sigma-Aldrich). After centrifugation at 10,000 $g$ for 10 min, the chloroform layer was collected, evaporated to dryness with nitrogen gas, and redissolved in 200 $\mu$L acetonitrile (20060.32, VWR Chemicals). Polyamine levels were detected on a Smartline Series HPLC system (Knauer). For this purpose, 3 $\mu$L aliquots were injected onto a Nucleodur 110-1.8 Gravity C18; 1,8 $\mu$m; 100 mm $\times$ 2 mm column (760076.02, Macherey-Nagel). Temperature was 40°C, detection wavelength was 200 nm, flow rate was 0.6 mL/min, and the measuring range was 20 Hz. Gradient solution started acetonitrile/water (40%/60%), followed by 50%/50% for 3 min, and then became 50%/50% for 4 min. Polyamine separation was done as previously described (Ditzen, Molecular psychiatry 15 (7), 2010, 702-711). The calculated concentration was multiplied by 300 $\mu$L volume 4% perchloric acid used for extraction to obtain the molar number. The molar number per 1 mg was calculated by dividing the weight of each tissue from the molar number.

*Measurement of polyamines (spermidine, spermine, N-acetyl spermidine, N-acetyl spermine, Diacetyl spermidine, Diacetyl spermine) by liquid chromatography mass spectrometry (LC-MS).* For the polyamine assay, the brain was immediately removed under deep anesthesia with ketamine (10%, Serumwerk, 100 mg/kg KM) and xylazine (1 vial = 20 mg/ml, Serumwerk, 16 mg/kg KM) (0.1 ml/10 g KM i. p.), and kept in cold PBS for 5-10 minutes, and horizontal slices (1000 $\mu$m thick) were prepared with a vibrating microslicer (Microm HM 650V, left, or Micotome with vibrating blade Leica VT1200, middle, or Acrylic Brain Slicers, Muromachi, MK-MS-01, right). The coronal slices of mice brain were visually divided into each area under microscope (Bilateral cortex, CA1, CA3, and dentate gyrus (DG)), followed by weighed and put into liquid nitrogen. Each tissue was treated with 100 $\mu$L volume 5% formic acid (F0507, Sigma-Aldrich) and sonicated (40 W, 60 s, UP50H, Dr. Hielscher GmbH) on ice, followed by extraction of polyamines overnight at 4°C. After centrifugation at 10,000 $g$ for 20 min, 100 $\mu$L of the supernatants were taken. The LC-MS-MS instrument consisted of a 1290 Infinity II HPLC (Agilent) coupled to a QTRAP6500+ (SCIEX). Chromatography started with 10 % A (water containing 0.5% of formic acid) and 90% of solvent B (acetonitrile containing 0.5% of formic acid) for 1.5 min. Then, a gradient started to reach 40% of A and 60 % of B within 8.5 min. The equilibration time with 10% of A/90% of B was 20 min. The flow rate was 0.5 ml/min using an EC 125/2 Nucleodur HILIC 3 $\mu$m column (Macherey und Nagel) at 40°C. Spermidine (85558-1G, Sigma-Aldrich), Spermine dihydrate (85588-5G, Sigma-Aldrich), N1-Acetylspermidine dihydrochloride (Cay 9001535-10, Cayman chemical), N1-Acetylspermine trihydrochloride (Sigma-Aldrich, 01467-25G), N1-N8-Diacetylspermidine hydrochloride (Cay 21588-1, Cayman chemical), and N1-N12-Diacetylspermine dihydrochloride (Cay 17918-1, Cayman chemical) were used as a standard. The calculated concentration was multiplied by 100 $\mu$L volume 5% formic

acid used for extraction to obtain the molar number. The molar number per 1 mg was calculated by dividing the weight of each tissue from the molar number.

*Mouse models of epilepsy, general.* All animal experiments were conducted in accordance with European (2010/63/EU) and federal law (TierSchG, TierSchVersV) on animal care and use, and approved by the country of North Rhine-Westphalia (Landesamt für Natur, Umelt und Verbraucherschutz, LANUV, AZ 81-02.04.2022A043). All mice were housed at 22 ± 2°C and humidity of 65% under a 12-hour light-dark cycle with food and water *ad libitum.* Adult male mice (4 weeks, > 15g) were obtained from Charles River (C57BL/6-N, MGI catalog #5651595). All efforts were made to minimize animal suffering and to reduce the number of animals used. *Pilocarpine model of temporal lobe epilepsy.* Established protocols were used to develop an acquired status epilepticus (SE) by systemic injection of pilocarpine (van Loo 2019 *loc. cit.).* Briefly, adult male C57BL/6-N mice were injected with 1mg/kg s.c. scopolamine methyl nitrate (Sigma-Aldrich, S2250-5G), and 20 minutes later, injected with 335 mg/kg s.c. pilocarpine hydrochloride (Sigma-Aldrich, P6503-10G). Forty minutes after SE onset, they were injected once with 4 mg/kg s.c. diazepam (5 mg/2 ml, Ratiopharm). Control mice were given scopolamine methyl nitrate and diazepam, but 0.9% NaCl instead of pilocarpine. Post-SE 1 week, 2 weeks, and 4 weeks mice are used for Immunoblotting and Immunohistochemistry.

*KCNT1 mouse model of genetic epilepsy.* Humanized mouse lines are useful disease models for studying pathophysiological processes and drug development in neurological disorders. Mouse lines carrying unpublished *KCNT1* mutations identified in patients *(KCNT1* p.I335N) were established in collaboration with the transgenic core unit of the CECAD research center (Cologne) using the CRISPR/Cas9 system. 8-10 weeks aged mice were used for immunoblotting. *Immunohistochemistry (IHC) for mouse brain tissue.* Mouse brain tissue was fixed in a 4 % buffered formalin solution (Sigma-Aldrich, 252549-2.5L) overnight at 4°C and embedded in paraffin. Formalin-fixed paraffin embedded (FFPE) brain tissue blocks were sectioned at 4 $\mu$m with a Microtome (HM-335-E, GMI) and dried overnight at 37°C. For histological analysis, all tissue sections were stained with hematoxylin (Waldeck) and eosin (Roth; HE). For immunohistochemical (IHC) analysis, sections were deparaffinized, and heated in a microwave for 15 minutes in 10 mM citrate buffer (pH 6.0, 1818.1, Roth). Endogenous peroxidase activity was quenched with 1% $H_2O_2$ for 30 min at room temperature. Slices were blocked in 5% normal goat serum (NGS; Thermo Fischer Scientific) in PBS for 30 min at room temperature, and incubated for overnight at room temperature with the primary antibodies; anti-sms rabbit polyclonal antibody (1:50; 15979-1-AP, Proteintech). After washing, samples were subsequently treated with biotinylated anti-rabbit antibody (1:500, BA1000, Vector Laboratories), which were detected using a Vectastain ABC-HRP kit (PK-6100, Vector Laboratories) for 30 min at room temperature and diaminobenzidine (DAB) staining (D4293, SIGMAFAST, Sigma Aldrich) for 3 min at room temperature. A counterstain was performed with hematoxylin incubation, and photographed with an optical microscope (BZ-X710; Keyence).

*Immunohistochemistry (IHC) for human surgery and autopsy samples.* Biopsy brain tissues of brain tumor or intractable epilepsy patients were neurosurgically removed for diagnostic and therapeutic purposes. Biopsy brain tissue from patients with hippocampal sclerosis, glioblastoma with or without epilepsy was analyzed. The biopsied brain tissues were fixed immediately in a 10% buffered formalin solution. Formalin-fixed paraffin embedded (FFPE) brain sections (4 $\mu$m) were deparaffinized, heated in a microwave for 15 minutes in 10 mM citrate buffer (pH 6.0, 1818.1, Roth). Endogenous peroxidase activity was quenched with 1% $H_2O_2$ for 30 min at room temperature. Slices were blocked in 5% normal goat serum (NGS; Thermo Fischer Scientific) in PBS for 30 min at room temperature, and incubated for overnight at room temperature with the primary antibodies; anti-sms rabbit polyclonal antibody (1:50; 15979-1-AP, Proteintech). After washing, samples were subsequently treated with biotinylated anti-rabbit antibody (1:500, BA1000, Vector Laboratories), which were detected using a Vectastain ABC-HRP kit (PK-6100, Vector Laboratories) for 30 min at room temperature and diaminobenzidine (DAB) staining (D4293, SIGMAFAST, Sigma Aldrich) for 3 min at room temperature. A counterstain was performed with hematoxylin incubation, and photographed with an optical microscope (BZ-X710; Keyence).

*Statistics.* For immunoblotting in vitro and in vivo, the data were obtained from at least 3 independent experiments and are presented as relative value to the control. Statistical analysis throughout the study was performed by applying a 2-tailed Student's t test for 2-group comparison and 1-way ANOVA with post hoc test for multi-group comparison. In all experiments, graph and other data are expressed as mean ± SEM. Significance was defined at P less than 0.05 using GraphPad Prism 8 (GraphPad Software). For experiments using cell cultures and mice, the number of experiments or animals is stated in the figure legends.

*Study approval.* All animal experiments were conducted in accordance with European (2010/63/EU) and federal law (TierSchG, TierSchVersV) on animal care and use, and approved by the country of North Rhine-Westphalia (Landesamt für Natur, Umelt und Verbracherschutz, LANUV, AZ 81-02.04.2022A043). This human study adhered to the ethics guidelines for human genome/gene analysis research and those for medical and health research involving human subjects endorsed by the country of North Rhine-Westphalia, and was approved by the ethics review committees of Bonn University. All participants provided written informed consent. Experimental procedures involving human subjects were conducted according to the principles expressed in the Declaration of Helsinki.

**Example 2: Screening the effects of key polyamines on NavCB efficacy, in particular antiseizure medication (ASM) efficacy**

HEK cells expressing (an) $Na^+$ channel(s) were used as a model system to screen how different polyamine species interact with the efficacy of the NaVCB/antiseizure drug (ASM), carbamazepine (CBZ). A focus was on spermine and spermidine, as well as on the $N_1$-acetylated derivatives of these two polyamines ($N_1$-acetylspermine and $N_1$-acetylspermidine) **(Fig. 1a)**. The recovery from inactivation was examined using standard protocols before and after application of 100 $\mu$M CBZ **(Fig. 1b**, Methods) in HEK cells expressing $Na_v 1.2$ channels. Using this approach, experiments were interleaved with or without different polyamine species included in the intracellular solution. Because the intracellular solution is continuous with the intracellular milieu, it equilibrates with the intracellular milieu, allowing introduction of polyamine species (see also Fig. 6). It was found that neither 100 $\mu$M intracellular spermine nor spermidine modified CBZ effects **(Fig. 1c).** in contrast, the $N_1$ acetylated polyamines dramatically modified the CBZ response. $N_1$-acetylspermidine at 100 $\mu$M and 1 mM potently decreased CBZ effects **(Fig. 1d).** Conversely, $N_1$-acetylspermine, in particular at high concentrations of 1 mM, increased CBZ effects **(Fig. 1d).**

These data show that the magnitude of the drug response to CBZ is dictated by the amount of intracellular **$N_1$-acetylspermine** and $N_1$-acetylspermidine.

Next, it was examined if the regulation by $N_1$-acetyl-polyamines is also observed for other, $Na^+$ channel blocking NaVCBs. It was found that effects of the AEDs oxcarbazepine (100 $\mu$M) and lamotrigine (100 $\mu$M) were similarly modified by $N_1$-acetyl-polyamines **(Fig. 1e, f).** The effects of these AEDs, however, were less pronounced than those for CBZ (summary of the overall magnitude of modulation by $N_1$-acetyl-polyamines in **Fig. 1g).**

This suggests that increased intracellular $N_1$-acetylspermidine concentrations can significantly reduce the efficacy of multiple, commonly used AEDs. It also shows that structural modification of antiseizure medications (such as modification of CBZ to OXC) can quantitatively modify this effect. This shows that in principle, the screening method described below is suitable to do hit-to-lead development to systematically generate compounds that are quantitatively less susceptible to modulation by $N_1$-acetyl-polyamines.

**Example 3: Modification of Spermine Synthase (SMS) Expression regulates N1-acetyl-polyamine species, and modifies Na+ channel drug response**

As shown in **Fig. 1a,** the two $N_1$-acetylated polyamine species are generated from either spermine or spermidine by the same $N_1$-acetyltrasferase (SSAT). Because there is no known direct interconversion pathway between $N_1$-acetylspermine and $N_1$-acetylspermidine, the only enzyme capable of regulating the ratio of these two isoforms is spermine synthase (SMS). To test the idea that spermine synthase (SMS) controls the efficacy of AEDs, SMS was genetically deleted in HEK cells expressing voltage gated sodium channels.

Reduction of spermine synthase (SMS) in HEK cells expressing either $Na_v 1.2$ or $Na_v 1.6$ channels was achieved with two different siRNAs. Western blots revealed a significant reduction of SMS expression by ~50% for both siRNAs (SMS1, SMS2, see **Fig. 2a**), and in both cell lines. The reduction in SMS led to a significantly altered ratio of spermine (SPM) and spermidine (SPD), as expected **(Fig. 2b).** At the same time, there were no changes in $Na^+$ channel alpha subunit expression **(Fig. 2c).** It was then tested if SMS knockdown affects CBZ sensitivity of $Na^+$ channels. Both siRNAs led to a significant reduction in CBZ effects **(Fig. 2e).** Finally, a rescue experiment was performed. SMS levels could be effectively reinstated by introduction of siRNA-resistant SMS variant **(Fig. 2d).** SMS rescue significantly recovered CBZ effects to control values **(Fig. 2f).** Following knockdown of SMS in HEK cells expressing $Na_v 1.6$ channels, a similar effect as in $Na_v 1.2$ channels was observed **(Fig. 2g).**

These results show that the reduction of SMS is sufficient to generate CBZ-resistant $Na^+$ channels.

It was also examined whether the inhibition of polyamine synthesis upstream of SMS leads to altered CBZ response. HEK cells were incubated with polyamine biosynthesis inhibitors of both ornithine decarboxylase (DFMO, 3 mM) and S-adenosylmethionine decarboxylase (SAMDC, SAM486A 3uM). This led to a significant reduction of both spermine and spermidine (data not shown). Inhibition of polyamine synthesis did not cause significant changes in CBZ effects on recovery from inactivation (data not shown). Thus, loss of use-dependent block only occurs with down-regulation of SMS function.

Further, it was previously shown that the enzyme that acetylates both spermine and spermidine, spermidine/-spermine-$N_1$-acetyltransferase (SSAT), is up-regulated in chronic epilepsy, supporting the idea that acetylation is particularly efficient in epileptic animals (Beckonert *loc. cit.*). This change would additionally lead to efficient conversion of accumulated spermidine to $N_1$-acetylspermidine, leading to an efficient accumulation of the latter polyamine.

**Example 4: Regulation of the polyamine system in chronic epilepsy models**

It was tested whether SMS reduction can underlie the emergence of pharmacoresistant $Na^+$ channels in chronic epilepsy models. First, it was addressed if SMS is down-regulated in chronic epilepsy models. Using Western blot analysis of tissue dissertates from hippocampal subfields, a down-regulation of SMS was shown in all hippocampal subfields in the pilocarpine model of epilepsy **(Fig. 3a)**. Quantitatively, the down-regulation was present both in ventral (not shown) and dorsal hippocampus, and was largest in the dentate gyrus (DG), the subregion where most pronounced resistance to CBZ was observed **(Fig. 3b)**. The down-regulation was already present 1 week after induction of epilepsy, and was persistent for up to a month. No down-regulation was seen in neocortex **(Fig. 3b)**. These results suggest reduction of SMS expression and should result in reduced conversion of spermidine to spermine. Spermine and spermidine levels were examined in the hippocampus of sham-control and pilocarpine treated animals, and it was found that the ratio of spermidine to spermine was significantly increased in epileptic animals **(Fig. 3c)**. Immunohistochemistry for SMS revealed a characteristic expression pattern, with highest levels of this enzyme in DG, and slightly lower levels in the CA1 and CA3 subfields, and a clear reduction of SMS in the chronic epilepsy model **(Fig. 3d)**.

### Example 5: Regulation of the polyamine system in a genetic epilepsy model

The question whether the loss of SMS generalizes to genetic epilepsy models was addressed by an analysis of SMS expression using western blots and immunohistochemistry in a mouse model carrying a Gain-of-function (GOF) mutation in the KCNT1 gene. Such GOF mutations in *KCNT1* are associated with developmental epileptic encephalopathies that are refractory to conventional antiseizure medications and cause a persistent developmental delay with a poor outcome. The model which was investigated harbors a GOF missense mutation (p.I335N). In this model, a robust hippocampal down-regulation of SMS with a particularly pronounced loss in DG was indeed found **(Fig. 4a)**. This was also reflected in the immunohistochemistry, which showed a qualitative down-regulation **(Fig. 4b)**.

### Example 6: Regulation of the polyamine system in human refractory epilepsy

It was also shown that SMS is down-regulated in human pharmacoresistant patients using immunohistochemistry (Fig. 5).

Thus, the examples provide evidence that SMS is down-regulated, and $N_1$-aSPD is increased, respectively, across models of acquired and genetic epilepsies, and also in human pharmacoresistant epilepsy.

### Example 7: Interaction-based screening with modified intracellular solutions

*General approach:*

This particular method makes use of the novel finding that $N_1$-Acetylspermidine significantly reduces ASD efficacy, as established in **Fig. 1d-f**. The goal of this screening method will be to primarily identify compounds in which addition of $N_1$-Acetylspermidine is not effective in reducing compound efficacy. In other words, curves for control and $N_1$-Acetylspermidine generated as in **Fig. 1d** should be overlapping, at least to some extent. Regarding $N_1$-Acetylsper-mine, compounds could also be sought that have an improvement of compound efficacy or no effect. A scheme of an exemplary stepwise protocol for this particular screening (using modified intracellular solutions) is depicted in **Fig. 7a**.

Thus, measurements with control intracellular solution, and intracellular solutions with $N_1$-Acetylspermidine may be performed. $N_1$-Acetylspermine measurements could be also performed with each compound. This approach can conveniently be done in the whole-cell patch-clamp configuration, because the patch-clamp method entails obtaining low-resistance whole-cell access with an intracellular solution filled glass pipette. The intracellular solution then equilibrates with the intracellular milieu, allowing to introduce specific polyamine species. Of note, this could conveniently be done with manual patch-clamping as well as with automated or high-throughput patch-clamp screening, all of which allow the control of the intracellular solution (**see Fig. 6** for schematic). This is also a broad method to test the involvement of other polyamine species.

*Detailed Protocol:*

**Step 1: HEK cell model:**

Any HEK cell line or other cell line expressing the sodium channel of interest can be used. In case of epilepsy, the CNS sodium channel isoforms $Na_V1.2$ and $Na_V1.6$ will be of most interest. In the case of pain syndromes, additional sodium channel isoforms including, for example, $Na_V1.7$ and $Na_V1.8$ will be of interest. In this particular implementation, HEK293T cells, a line derived from human embryonic kidney at passage 10-20, have been used. The HEK cell line stably expresses the endogenous human *SCN2A* or *SCN8A* gene encoding $Na_V1.2$ and $Na_V1.6$ voltage-gated sodium channels. The HEK cell line used in this particular implementation was a gift from Holger Lerche (Hertie Institute for Clinical Brain Research, Tübingen). The cells were cultured in Dulbecco's modified Eagle's medium (61965026, Thermo Fisher Scientific) containing 10% (vol/vol) FBS (10270106, Thermo Fisher Scientific) with 500 $\mu$g/mL Geneticin (10131035, Thermo Fisher Scientific), 1 mM sodium pyruvate (11360039, Thermo Fisher Scientific), and 1% (vol/vol) MEM Non-essential amino acids solution (11140035, Thermo Fisher Scientific) at 37 °C and 10% $CO_2$. HEK293T cells were then plated on poly-D-lysine coated glass for whole-cell voltage-clamp recordings.

***Step 2: Preparation of intra- and extracellular solutions:***

For recording sodium currents, standard solutions can be used for patch-clamp experiments. In this particular implementation, an extracellular solution for superfusion during patch-clamp experiments may be used containing (in mM): 40 $CH_3SO_3Na$, 90 TEA-Cl, 10 HEPES (free), 1.6 $CaCl_2 \cdot 2H_2O$, 2 $MgCl_2 \cdot 6H_2O$, 0.2 $CdCl_2 \cdot 2 H_2O$, five 4-Aminopyridine (4-AP), and 15 glucose. The pH was adjusted to 7.4 with HCl and the osmolarity to 310 mOsm/L with glucose.

The control intracellular solution containing the following (in mM): 110 CsF, 10 HEPES-Na, 11 EGTA, 2 $MgCl_2$, 20 tetraethylammonium chloride (TEA-Cl), 0.5 GTP-Na, 5 ATP-$Na_2$, pH 7.25 adjusted with CsOH and 300 mOsm/L with sucrose.

To generate intracellular solutions with polyamines, these can be added to this intracellular solution. In this particular implementation, **$N_1$-Acetylspermidine** dihydrochloride (100 $\mu$M, 1 mM, Cayman chemical, Cay9001535-10) or **$N_1$-Acetylspermine** trihydrochloride (100 $\mu$M, 1 mM, Sigma-Aldrich, 01467-25MG) may be added. The structures of **$N_1$-Acetylspermidine** and $N_1$-Acetylspermine are displayed in **Fig. 8.**

***Step 3: Establishing of a whole-cell voltage-clamp recording:***

Voltage-clamp recordings can be carried out using standard methods to record voltage-gated channels. In this particular implementation, manual patch-clamp approaches may be used, but automated patch-clamp approaches will also be applicable. All voltage-clamp experiments were performed at room temperature (22±1 °C). The applicability at room temperature further facilitates the use of this method for screening. Patch pipettes were pulled with a GB150F-8P borosilicate glass (Science Products) with a micropipette puller (Sutter Instruments). The tip resistance was 3.0 to 5.0 M$\Omega$. Tight-seal whole-cell recordings were obtained with a seal resistance of >1 G$\Omega$ using a patch-clamp amplifier (Axopatch 200B, Molecular Devices). Series resistance was compensated by 70-90%, with a maximal residual voltage error of < 5.3 nA. At least 10 minutes were allowed to pass after establishing whole-cell configuration to allow intracellular solution and the internal milieu of the cell to equilibrate. During voltage-clamp recordings data was sampled with a 50 kHz sampling rate using a Digidata 1440A AD converter (Molecular Devices), and filtered with a 10 kHz low-pass filter. High sampling rates and filter settings are necessary in the case of voltage-gated sodium channels, as the kinetics of these currents are very fast. The Clampex 10.7 acquisition suite (Molecular Devices) was used for data acquisition. A liquid junction potential of 10 mV was calculated and corrected offline for all measured HEK293T cells.

***Step 4: Application of Medicament/Drug:***

Carbamazepine (CBZ, Sigma-Aldrich, C4024-5G), oxcarbazepine (OXC, Merck, PHR 1635-1G), eslicarbazepine acetate (ESL, Merck, B5435-10MG), and lamotrigine (LTG, Merck, PHR 1392-1G) were dissolved in dimethyl sulfoxide (DMSO) and added to the extracellular solution (final concentration, 100 $\mu$M). Any $Na_VCS$ candidate can be dissolved in an analogous manner, taking care to include the same DMSO concentration in all measurement conditions. Medicament/drug solutions can be applied with any device leading to exchange of the extracellular solution. In this particular case, a simple self-constructed super fusion device was employed that leads to extracellular solution exchange in 1-2 minutes. After solution change, 5 minutes were left to elapse to ensure complete solution exchange.

***Step 5: Setting of Voltage-clamp paradigms - data analysis:***

During whole-cell recording of sodium channels, the voltage-clamp model of the amplifier is used to run different voltage protocols. The most important voltage protocol is the one assessing recovery from fast inactivation of $Na^+$ currents. For this purpose, a double-pulse experiment with increasing intervals t = 1ms * 2n (n = 0, 1, 2, 3, 4, 5, 6, 7, 8, 9, 10, 11, 12, 13) between the first and second pulse was applied for evaluation of recovery from fast inactivation. To ensure full recovery from inactivation, an interval of 5 s at -80 mV holding potential was interspersed between single protocols. The results from such a protocol are depicted in **Fig. 1b** (left panel). Subsequently, the NavCB of choice is super fused. Following complete exchange of the extracellular solution, the double pulse protocol is repeated (as shown in **Fig. 1b,** left panel for CBZ). This allows quantitative evaluation of NavCB effects on recovery from inactivation with the following analysis steps:

First, peak sodium channel amplitude from the second pulse was normalized to that from the first pulse. Secondly, a subtraction is carried out, subtracting the normalized peak values after NavCB application from those under control conditions (as shown for CBZ in **Fig. 1b,** right panel). The peak of this curve indicates the magnitude of use-dependent block, and can be averaged across cells. In this particular implementation, the overall magnitude of the effects of acetyl-polyamine variants were then assessed as a difference in CBZ effects for 1 mM $N_1$-acetylspermidine on the one hand, and 1 mM $N_1$-acetylspermine on the other hand. This value indicates how prominently the acetylpolyamine modulation is influencing the use-dependent effects of a given compound (as shown for the NavCBs CBZ, OXC, ESL and LTG in **Fig. 1g).** All patch-clamp data analyses were done with Clampfit 10.7 software (Molecular Devices) and GraphPad Prism 8 (GraphPad Software).

It should be noted that, in addition to recovery from inactivation, all other voltage- and time-dependent properties of sodium channels, and their dependence on polyamines, may also be easily assessed by adding other types of voltage protocols.

### Example 8: Screening with a genetically modified HEK cell line

*General approach:*

The second method replicates the reduction in SMS expression found in experimental and human epilepsies in the HEK system. For this approach, any cell line that expresses the sodium channel of interest and SMS can be used. In addition, a variant of the same cell line with reduced expression of SMS has to be generated. In this implementation, a HEK cell line stably expressing sodium channels may be used, and a siRNA based knockdown of SMS may be performed to generate a test system. A scheme of an exemplary stepwise protocol for this particular screening (using the RNAi technique) is depicted in **Fig. 7b.**

*Detailed Protocol:*

### *Step 1: HEK cell model:*

Any HEK cell line or other cell line expressing the sodium channel of interest can be used. In case of epilepsy, the CNS sodium channel isoforms $Na_V 1.2$ and $Na_V 1.6$ will be of most interest. In the case of pain syndromes, additional sodium channel isoforms including, for example, $Na_V 1.7$ and $Na_V 1.8$ will be of interest. In this particular implementation, HEK293T cells, a line derived from human embryonic kidney at passage 10-20, have been used. The HEK cell line stably expresses the endogenous human *SCN2A* or *SCN8A* gene encoding $Na_V 1.2$ and $Na_V 1.6$ voltage-gated sodium channels. The HEK cell line used in this particular implementation was a gift from Holger Lerche (Hertie Institute for Clinical Brain Research, Tübingen). The cells were cultured in Dulbecco's modified Eagle's medium (61965026, Thermo Fisher Scientific) containing 10% (vol/vol) FBS (10270106, Thermo Fisher Scientific) with 500 $\mu$g/mL Geneticin (10131035, Thermo Fisher Scientific), 1 mM sodium pyruvate (11360039, Thermo Fisher Scientific), and 1% (vol/vol) MEM Non-essential amino acids solution (11140035, Thermo Fisher Scientific) at 37 °C and 10% $CO_2$.

### *Step 2: SMS knockdown:*

Any approach suitable to knock down or reduce SMS function would be suitable to generate a screening system according to this example. In this particular implementation, twenty-four hours after the last passage, the cells were transfected with siRNAs using lipofectamine 2000 (11668027, Thermo Fisher Scientific), according to the manufacturer's instructions. Oligonucleotide siRNA duplexes were synthesized by Sigma-Aldrich with the following sequences:

- control-siRNA (mission® sirna universal negative control #1, sic001-10nmol, Sigma-Aldrich)
- SMS siRNA-set1-S1, 5'-CGACAGUAUUUUGAACAAA-3' (SEQ ID NO. 1)
- SMS siRNA-set2-S2, 5'-GAUUCACCUUAUCAAAAUA-3' (SEQ ID NO. 2)

As set1 and set 2 siRNA yielded equivalent results regarding the knockdown efficacy for SMS (see **Fig. 1a, b)** without affecting sodium channel expression in HEK cells expressing either $Na_V 1.2$ or $Na_V 1.6$ **(Fig. 1c),** set1 siRNA (S1) for SMS knockdown throughout the experiments may be used. For validation that any effects of knockdown are specific to SMS reduction, rescue experiments may be performed, in particular in a new system. In this particular implementation, SMS rescue experiments may be performed by seeding HEK cells on a 24-well plate and transfecting them with 50 pmol control siRNA, 250 ng mock plasmid, 50 pmol S1-siRNA, and 250 ng SMS plasmid using lipofectamine 2000 as described above. The pCMV6-SMS-GFP construct was purchased as SMS plasmid from Origene (rg233729). Rescue experiments were successful, resulting in re-establishment of SMS expression **(Fig. 1d).** As was also shown herein, rescue results in reinstatement of CBZ efficacy on use-dependent block, confirming that SMS reduction is causal in modification of these effects **(Fig. 2f).**

*Step 3: Verify SMS knockdown:*

To quantitatively verify SMS knockdown, immunoblotting may be used. To this end, the following approach may be applied. The protein samples were homogenized with RIPA buffer (182-02451, Wako) containing 10% protease inhibitors (4693116001, Roche) and phosphatase inhibitors (4906845001, Roche). Homogenates were subsequently incubated on ice for 20 min and centrifuged at 10,000 rpm for 10 min at 4°C. The lysates were mixed with $4 \times$ NuPAGE LDS sample buffer (NP0008, Novex) and heated at 95°C for 5 min. After denaturation, each cell lysate was separated by SDS-PAGE (10% gel) for anti-spermine synthase (SMS) antibody and by SDS-PAGE (8% gel) for anti-Nav1.2 antibody and transferred to a nitrocellulose membrane, which was blocked in 5% milk subsequently. The primary and secondary antibodies were the followings: Recombinant anti-spermine synthase rabbit monoclonal [EPR9252(B)] (1:2000, ab156879, abcam); anti-Nav1.2 rabbit polyclonal (1:200; ACC-002, Alomone Labs); anti- GAPDH mouse monoclonal (1:1000, NB300-221, Novus); IRDye® 800CW goat anti-rabbit IgG secondary antibody (1:10000, 926-32211, LI-COR Biosciences); IRDye® 680LT donkey anti-mouse IgG secondary antibody (1:10000, 926-68022, LI-COR Biosciences). The primary antibodies were incubated for 2 hours at RT, while the fluorescently labeled IRDye anti-rabbit 800 nm IgG (LI-COR Biosciences) or IRDye anti-mouse 680 nm were incubated for 45 minutes at RT, and detected with the infrared Odyssey system (LI-COR Biosciences). The band intensity was quantified by Image Studio Lite version 5.2 software (LI-COR Biosciences). The protein band intensity was determined as a ratio of the housekeeping protein glycerinaldehyde-3-phosphate-dehydrogenase (GAPDH) within the same membrane and normalized to the average ratio obtained in sham-control. The quantitative reduction of SMS expression at the protein level should be calibrated to be similar to that observed in animal models of epilepsy (around 50%).

*Step 4: Perform patch-clamp analyses:*

Subsequently, patch-clamp analyses may be performed. These may methodically be identical to steps 2-5 as described in the context of Example 7. However, in this particular implementation, all recordings are performed by controlling the intracellular solution without an addition of polyamines. In this case, measurements of use-dependent block of compounds of interest in a control cell line (no loss/reduction of SMS), which would be compared with an experimental cell line (knockdown/reduction of SMS) for each compound. Successful Compounds considered to be positive hits according to the invention would be those that do not exhibit a loss of efficacy (or only to a reduced extent as compared to a control; e.g. CBZ) if SMS expression is reduced.

**Example 9: Screening using genetically modified primary neurons**

General approach:

The predominant purpose of this screening method is to determine the activity of NavCBs on neuronal network activity in a neuronal cell population with reduced expression of SMS. For this type of screening method, primary neuronal cultures may be used. To generate a lack/reduction of SMS, a knockdown may be utilized in primary cultures. Alternatively, primary neuronal cultures can be generated from heterozygous SMS knockout mice. Measuring neuronal activity may be done with any method that assesses neuronal spiking. The method used in the context of this particular implementation employs multielectrode arrays (MEAs). This would enable high-throughput screenings.

Detailed Protocol:

*Step 1: SMS loss-of-function mice:*

SMS loss-of-function can be generated in different ways. First, shRNA-based knockdown may be used; with methods for knockdown analogous to those as described herein elsewhere, for example in the context of Example 8 or in the context of Embodiment B described herein). Primary neurons generated from mice with a genetic deletion of SMS may also be used. This includes mice with a spontaneously occurring loss-of-function of SMS (i.e. gyro mice). Male Gyro mice lacking SMS due to a deletion of part of the X chromosome are viable on the B6C3H background. However, they have a variety of abnormalities including deafness, neurological problems, small size, and a tendency to early death. Therefore, their use in nonoptimal, as other genes can be affected (Wang, molecular biology (Clifton, N.J.) 720, 2011, 159-70). Conditional knockout mouse lines may also be used (preferred option). Examples of commercially available mouse lines are as follows:

-    C57BL/6J/Sms[em6Lutzy]/J (Strain #: 032064, The Jackson Laboratory)
-    CS7BL/6Smoc-[SMSem1(flox)Smoc] (Cat. No. NM-CKO-2117943, Shanghai Model Organisms)

- C57BL/6JGpt-Sms-flox (T016254, GemPharmatech Co., Ltd.)

Primary neuron cultures from different regions (for example CNS neurons for applications such as epilepsy, DRG neurons for pain-related applications) can be generated from such mice, viral gene transfer of Cre-recombinase then leads to a loss of SMS expression.

***Step 2: Primary neuronal culture generation:***

To generate primary cultures multiple protocols are available. In this particular version of the protocol, embryos staged at E14-16 were taken under deep anaesthesia with isoflurane inhalation and transferred to a 10-cm Petri dish on ice. The heads were decapitated, and the brains were dissected under a stereo microscope at room temperature. The dissected cortices were transferred in 1.8 ml HBSS (-) (14170-088, 500 ml, Gibco) and 200 $\mu$L Trypsin (15090046, 2.5% Trypsin, 100 ml, Gibco), and then incubated at 37°C and 5% $CO_2$ for 15-20 minutes. The cortical tissues were triturated with 1ml of 10 mg/mL DNase I (Final concentration 1mg/mL) (11284932001, 100 mg, Roche) and 120 $\mu$L of 1M $MgSO_4$-$7H_2O$ and 9 ml of cold HBSS (-). The triturated tissue was washed with the NeuroBasal medium (21103-049, 500ml, Gibco) complemented with 2% B27 (17504-044, 10ml, Gibco) and 0.4% L-glutamine (25030-024, 2ml, Invitrogen, Gibco). The cell suspension containing 40,000 neurons per 5-20 $\mu$L was plated at 1 well on 24 well microelectrode array (MEA) plate (CytoView MEA 24, M384-tMEA-24W, Axion Biosystems) and incubated at 37°C and 5% $CO_2$ for 30-60 minutes. It was added with the NeuroBasal medium 700 $\mu$l/well (21103-049, 500ml, Gibco) complemented with 2% B27 and 0.4% L-glutamine.

***Step 3: AAV production (SMS Knockdown constructs or Cre-driven by Synapsin-1 promotor constructs):***

Targeting sequences of shRNA are Sms; shControl, shSms-1, and shSms-2. Then, pAAV-Syn1-shControl-EGFP, pAAV-msyn1-shSms-1-T2A-EGFP, and pAAV-Syn1-shSms-2-T2A-EGFP are ordered to VectorBuilder.

Regarding Cre-driver, the pAAV-msyn1-EGFP and pAAV-Syn1-Cre-T2A-EGFP were purchased from Vector-Builder.

These AAV vector plasmids containing synapsin promotor and two fluorescent proteins. Recombinant AAV1/2 genomes were generated by large scale triple transfection of HEK293-AAV cells; the AAV plasmid, helper plasmids encoding *rep* and *cap* genes (pRV1 and pH21), and adenoviral helper pF$\Delta$6 (Stratagene, La Jolla, USA) using standard $CaPO_4$ transfection. At 48-72 h after transfection, cells were lysed in 0.5% sodium deoxycholate (Sigma-Aldrich, D6750) and 50 U/ml Benzonase endonuclease (Sigma-Aldrich, 1.01697). rAAV viruses were purified using HiTrap heparin column purification (GE Healthcare). rAAV particles were concentrated to a final volume of 400 $\mu$l by Amicon Ultra Centrifugal Filters (Millipore). Purity of the viruses was validated by coomassie blue staining of SDS-polyacrylamide gels. Functional titers of the fluorescent protein vectors with or without doxycycline (DOX, Sigma-Aldrich, D3072-1ML) were determined by transduction of cultured primary neurons. (Schoch, The Journal of neuroscience: the official journal of the Society for Neuroscience 41(39), 2021, 8111-25; van Loo, The Journal of neuroscience: the official journal of the Society for Neuroscience 39(17), 2019, 3175-87; van Loo, Nature communications 6, 2015, 8688).

***Step 4: Knockdown of SMS:***

For shRNA-mediated knockdown, primary cortical neuronal cultures from C57BL/N mice may be infected at DIV4 with AAV1/2-Syn1-shControl-EGFP, AAV1/2-Syn1-shSms-1-T2A-EGFP, and AAV1/2-msyn1-shSms-2-T2A-EGFP. If conditional SMS knockout mice are used, primary cortical neuronal cultures are used. At DIV4, primary cortical neurons are infected with a combination of AAV1/2-Syn1-EGFP and AAV1/2-Syn1-Cre-T2A-EGFP. Two weeks later (at DIV 18), reduction of the SMS protein levels should be confirmed using Western blotting. NavCBs (e.g. antiseizure medications) and candidate drugs may then be administered at DIV18-25, when protein levels of SMS are reduced.

***Step 5: Perform MEA Analyses:***

Any approach to measure the activity in neurons is generally suitable. MEA recordings are particular appropriate because they are suitable for a high-throughput approach, and are straightforward to analyse. MEA recordings may be conducted on any suitable system. In this particular implementation, they were conducted using Maestro Edge System with CytoView-MEA24 well plates from Axion Biosystems (M384-tMEA-24W, $4 \times 4$ grids (1.1 mm $\times$ 1.1 mm) = 16 electrodes, 1 grid = 50 $\mu$m diameter). Neurons were cultured on these MEA24 plates and recorded every 2-3 days. Baseline recording was initially captured without NavCBs/ASMs, followed by experiments to observe the acute effects of NavCBs/ASMs at DIV 20-23. To maintain the robustness and reproducibility of MEA data, the medium was not changed through the experiments. The Maestro settings are as follows; a sampling frequency of 12.5 kHz, a "Neural Spikes" gain of 1000×, and a band-pass filter of 200-3000 Hz as the default neural setting. To visually inspect the activity of each plate during recording, the data processing was performed using "Spike Detector (6 × STD)" and "Burst Detector". Spikes were detected when the signal crossed the threshold of 6× standard deviation of the baseline, while bursts were identified when the interval between spikes was within 100 ms and at least 5 spikes overlapped. For MEA recordings, the Maestro system was maintained at 37°C and 5% $CO_2$. The 24-well MEA plate containing primary cortical neuron culture was placed into a docking bay and allowed to equilibrate for 5 minutes. Following this, a 5-minute baseline recording was initiated. After applying Na$_V$CBs/ASMs, a 5-minute wash-in recording was started. Post wash-in, 75% of medium was replaced with NeuroBasal medium 700 μl/well supplemented with 2% B27 and 0.4% L-glutamine, and a 5-minute wash-out recording was conducted.

The raw data (RAW files) were analyzed by Axion Integrated Studio (Axis) software (Axion BioSystems; version 3.7.3) and the spike files (SPK files) was analyzed by the NeuralMetric Tool (Axion BioSystems; version 4.0.5). This AxIS navigator identifies spikes and bursts using previously described spike and burst detectors. Activities were broadly grouped into four categories: spiking, single-electrode bursting, network bursting, and synchrony. To compare the activity of baseline, wash-in, and wash-out in every parameter among ASMs, activities were normalized in the activities of DMSO to account for the potential DMSO effects. Additionally, the effects of Na$_V$CBs/ASMs should be evaluated by normalizing the baseline prior to drug wash-in.

**Example 10: Screening using human iPSC derived systems**

*General approach:*

This approach uses human-derived neuronal systems to assess the effects of SMS loss on neuronal network activity.

The targeting sequences of SMS-shRNA were synthesized in the same way as of the mice construct described above (Example 9); shControl, shSMS-1, and shSMS-2. Then, pAAV-Syn1-shControl-EGFP, pAAV-hSyn1-shSMS-1-T2A-EGFP, and pAAV-hSyn1-shSMS-2-T2A-EGFP are ordered to VectorBuilder.

To generate a lack of SMS, a knockdown may be utilized. Alternatively, it was found that long-term maintenance of spontaneously synchronously active human neuronal cultures leads in itself to a significant reduction of SMS expression. Such systems may also be used for screening. Measuring neuronal activity may be done with any method that assesses neuronal spiking. The method used in the context of this particular implementation employs multielectrode arrays (MEAs). This enables highe-throughput approaches. The the experimental details of the screening method of this particular implementation are essentially identical to those as described in the context of Example 9.

**Example 11: Screening using mouse *in vivo* models**

*General approach:*

This approach uses conditional knockout of SMS to generate an *in vivo* loss-of-function model. Conditional knockout of SMS may be combined with any epilepsy model, for example an epilepsy model used for preclinical screening and validation of ASMs. For this purpose, these models may be converted to models suitable for particular screening for antiseizure compounds which are capable of overcoming the pharmacoresistance mechanism.

Male Gyro mice lacking SMS due to a deletion of part of the X chromosome are viable on the B6C3H background. However, they have a variety of abnormalities including deafness, neurological problems, small size, and a tendency to early death. Therefore, their use in nonoptimal, as other genes can be affected (Wang *loc. cit.*). In the context of the described screening methods, conditional knockout mice are preferred/more suitable (the effects can be seen more directly in such mice).

For this kind of screening, any conditional knockout mouse line may be used, including the following

- C57BL/6J/Sms$^{em6Lutzy}$/J (Strain #: 032064, The Jackson Laboratory)
- C57BL/6Smoc-$^{SMSem1(flox)Smoc}$ (Cat. No. NM-CKO-2117943, Shanghai Model Organisms)
- C57BL/6JGpt-Sms-flox (T016254, GemPharmatech Co., Ltd.)

Crossing with appropriate Cre-driver lines allows to generate inducible loss-of-function of SMS. Following this, standard procedures for *in vivo* models may be followed.

In this context, SMS knockout models may be used, for instance, in conjunction with the following tests, widely used for screening for novel NavCB/anticonvulsant drugs (Kehne, Neurochemical research 42 (7), 2017, 1894-1903).

- 6 Hz electrical stimulation test;

- Maximal electroshock models;
- Corneal kindling test and other kindling models; and/or
- Spontaneous bursting slice model from post-kainic acid models_

The SMS knockout approach may also be used with any other *in vivo* epilepsy model. It is expected to be most useful with models that are suitable/already being used for screening with semi-automated, automated or high-throughput, such as the above.

## Claims

1. A method of screening for a voltage-gated sodium (Nav) channel blocker (NavCB),

   wherein said method comprises contacting a test compound with a Nav channel (NavC) in the presence of an N1-acetylspermidine (N1aSPD) concentration of $\geq 0.5\ \mu M$, and
   wherein said test compound is identified as said NavCB if it blocks said NavC in the presence of said N1aSPD concentration.

2. A method of screening for a $Na_V CS$ which is pharmacologically effective in the treatment of a pharmacoresistant neuronal hyperexcitability disease/disorder (PRNHD),

   wherein said method comprises contacting a test compound with a NavC in the presence of an N1aSPD concentration of $\geq 0.5\ \mu M$, and
   wherein said test compound is identified as said NavCB if it inhibits/blocks said NavC in the presence of said N1aSPD concentration.

3. The method of screening of claim 1 or 2, wherein said N1aSPD concentration is an N1aSPD concentration of $\geq 1\ \mu M$, $\geq 10\ \mu M$, $\geq 20\ \mu M$, $\geq 30\ \mu M$, $\geq 50\ \mu M$, $\geq 75\ \mu M$, $\geq 100\ \mu M$, $\geq 150\ \mu M$ or $\geq 200\ \mu M$.

4. The method of screening of any one of claims 1 to 3, wherein said N1aSPD concentration is an N1aSPD concentration in the range of 75 $\mu M$ to 1.5 mM or 100 $\mu M$ to 1 mM.

5. The method of screening of any one of claims 1 to 4, wherein said NavC

   (i) conducts sodium ions ($Na^+$) through a phospholipid bilayer membrane;
   (ii) displays voltage-dependent fast ($\leq 15$ ms) activation and inactivation of $Na^+$ currents conducted over a phospholipid bilayer membrane; and/or
   (iii) displays fast ($\leq 20$ ms) recovery from inactivation of inactivated $Na^+$ currents over a phospholipid bilayer membrane.

6. The method of screening of any one of claims 1 to 5, wherein said NavC is located in the outer cell membrane of a human or animal cell and said N1aSPD concentration is present in the lumen of said cell; and wherein, in the context of said contacting said test compound with said NavC, said test compound is contacted with the extracellular side of said $Na_V C$, i.e. from the outside of said cell.

7. The method of screening of claim 6, wherein said cell is a Human Embryonic Kidney (HEK) cell, for example a HEK293 cell (e.g. a HEK293T, HEK293F, HEK293FT or HEK293-AAV cell), a Chinese Hamster Ovary (CHO) cell, a xenopus oocyte, or a cell of a neuroblastoma cell line; or wherein said cell is a neuron, a primary neuron, a glia or a neuroglia.

8. The method of screening of any one of claims 1 to 7, wherein, in the context of said contacting said test compound with said NavC, said test compound is contacted with a human or animal neuronal culture system which comprises (a) neuron(s), (a) primary neuron(s), (a) glia(s) and/or (a) neuroglia(s) as defined in claim 7.

9. The method of screening of any one of claims 1 to 7, wherein, in the context of said contacting said test compound with said NavC, said test compound is contacted with an animal which comprises (a) cell(s) as defined in claim 6 or 7.

10. The method of screening of any one of claims 6 to 9, wherein said cell, neuronal culture system or animal is genetically engineered so that the spermine synthase (SMS) activity is inhibited in said cell, in said neuronal culture system or in said animal.

11. The method of screening of any one of claims 6 to 10, wherein said animal is an animal model selected from the group consisting of animal models of chronic epilepsy, chronic temporal lobe epilepsy (cTLE), refractory epilepsy, acquired epilepsy, pilocarpine models of epilepsy, models of genetic epilepsy (for example a KCNT1 model of genetic epilepsy which harbours the gain-of-function (GOF) missense mutation p.I335N in KCNT1).

12. The method of screening of any one of claims 1 to 11, wherein said NavC is a $Na_V1.2$ channel, $Na_V1.6$ channel, $Na_V1.1$ channel, $Na_V1.3$ channel, $Na_V1.4$ channel, $Na_V1.5$ channel, $Na_V1.7$ channel, $Na_V1.8$ channel or $Na_V1.9$ channel.

13. The method of screening of any one of claims 1 to 8 and 10 to 12, wherein said method is an *in vitro* or *ex vivo* method practiced on a cell or on a neuronal culture system as defined in any one of claims 6 to 8 and 10 to 12; and wherein said method comprises

   (i) contacting said test compound with said NavC in the presence of said N1aSPD concentration by a contact device;
   (ii) setting, controlling and/or modifying said N1aSPD concentration by a control device; and/or
   (iii) testing by a test device whether, upon said contacting said test compound with said NavC, said NavCB blocks said NavC in the presence of said N1aSPD concentration.

14. The method of screening of claim 13, wherein said contact device is a patch-clamp device, said control device is a patch-clamp device, and/or said test device is a patch-clamp device; or wherein said contact device is a microelectrode device or a microelectrode array (MEA) device, said control device is a microelectrode device or a MEA device, and/or said test device is a microelectrode device or a MEA device.

15. The method of screening of claim 14, wherein said patch-clamp device(s) is/are (a) whole-cell patch-clamp device(s), an outside-out patch-clamp device(s), a cell-attached patch-clamp device(s) or an inside-out patch-clamp device(s); or wherein said microelectrode device(s) or said MEA device(s) is/are or comprise(s) a MEA plate.

16. The method of screening of claim 14 or 15, wherein said patch-clamp device(s) is/are, or comprise(s), a patch-clamp micropipette or a planar patch-clamp device (for example a patch-clamp chip; e.g. of the Sophion Bioscience Qube system, like QPatch Compact, QPatch or Qube 384); and/or a patch-clamp chamber (for example a superfusion device).

17. The method of screening of any one of claims 14 to 16, wherein

   (i) said patch-clamp devices are whole-cell patch-clamp devices;
   (ii) said patch-clamp micropipette or said planar patch-clamp device is used as both, as said control device and as said test device;
   (iii) said patch-clamp chamber is used as said contact device;
   (iv) said patch-clamp micropipette or said planar patch-clamp device of (ii) comprises an intracellular solution which contains said N1aSPD concentration as defined in claim 4;
   (v) said patch-clamp chamber contains a bath solution containing said test compound;
   (vi) said whole-cell patch-clamp devices are practiced on said cell, i.e. said cell is placed in said bath solution as contained in said patch-clamp chamber of (v); and said patch-clamp micropipette penetrates said cell, or said cell is adhered to said planar patch-clamp device, so that the intracellular milieu within said cell equilibrates with said intracellular solution; and
   (vii) it is measured with said whole-cell patch-clamp devices whether said test compound blocks said NavC as located in the outer cell membrane of said cell in the presence of said N1aSPD concentration.

18. The method of screening of any one of claims 2 to 17, wherein said PRNHD is a pharmacoresistant epilepsy (PRE) or a pharmacoresistant pain disorder/pain-related disorder (e.g a pharmacoresistant neuropathic pain; a pharmacoresistant hereditary pain syndrome etc.), a pharmacoresistant trigeminal neuralgia, a pharmacoresistant postherpetic neuralgia, a pharmacoresistant diabetic neuropathy, a pharmacoresistant Alzheimers disease (AD) or a pharmacoresistant stroke.

19. A two-step method of screening for a NavCB comprising

(i) operating a method of screening for a NavCB according to any one of claims 13 to 18;
(ii) subsequently operating a method of screening of any one of claims 9 to 12 and 18, with the NavCB as identified according to (i) as said test compound.

**20.** A genetically engineered cell as defined in claim 10 or 12.

**Amended claims in accordance with Rule 137(2) EPC.**

**1.** A method of screening for a voltage-gated sodium channel blocker ($Na_V$CB),
wherein said method comprises contacting a test compound with a voltage-gated sodium channel ($Na_V$C) in the presence of an N1-acetylspermidine (N1aSPD) concentration of $\geq 0.5$ $\mu$M, and wherein said test compound is identified as said $Na_V$CB if it inhibits/blocks said $Na_V$C in the presence of said N1aSPD concentration,

(i) wherein said method is an *in vitro* method; or
(ii) wherein said method is an *in vivo* method practiced on a non-human animal model.

**2.** The method of screening of claim 1, which is a method of screening for a $Na_V$CB which is pharmacologically effective in the treatment of a pharmacoresistant neuronal hyperexcitability disease/disorder (PRNHD).

**3.** The method of screening of claim 1 or 2, wherein said N1aSPD concentration is an N1aSPD concentration of $\geq 1$ $\mu$M, $\geq 10$ $\mu$M, $\geq 20$ $\mu$M, $\geq 30$ $\mu$M, $\geq 50$ $\mu$M, $\geq 75$ $\mu$M, $\geq 100$ $\mu$M, $\geq 150$ $\mu$M or $\geq 200$ $\mu$M.

**4.** The method of screening of any one of claims 1 to 3, wherein said N1aSPD concentration is an N1aSPD concentration in the range of 75 $\mu$M to 1.5 mM or 100 $\mu$M to 1 mM.

**5.** The method of screening of any one of claims 1 to 4, wherein said $Na_V$C

(i) conducts sodium ions ($Na^+$) through a phospholipid bilayer membrane;
(ii) displays voltage-dependent fast activation and inactivation of $Na^+$ currents conducted over a phospholipid bilayer membrane, wherein said fast activation and inactivation is within $\leq 15$ ms; and/or
(iii) displays fast recovery from inactivation of inactivated $Na^+$ currents over a phospholipid bilayer membrane, wherein said fast recovery is within $\leq 20$ ms.

**6.** The method of screening of any one of claims 1 to 5, wherein said $Na_V$C is located in the outer cell membrane of a human or animal cell and said N1aSPD concentration is present in the lumen of said cell; and wherein, in the context of said contacting said test compound with said $Na_V$C, said test compound is contacted with the extracellular side of said $Na_V$C, i.e. from the outside of said cell.

**7.** The method of screening of claim 6, wherein said cell is a Human Embryonic Kidney (HEK) cell, for example a HEK293 cell (e.g. a HEK293T, HEK293F, HEK293FT or HEK293-AAV cell), a Chinese Hamster Ovary (CHO) cell, a xenopus oocyte, or a cell of a neuroblastoma cell line; or wherein said cell is a neuron, a primary neuron, a glia or a neuroglia.

**8.** The method of screening of any one of claims 1 to 7, wherein, in the context of said contacting said test compound with said $Na_V$C, said test compound is contacted with a human or animal neuronal culture system which comprises (a) neuron(s), (a) primary neuron(s), (a) glia(s) and/or (a) neuroglia(s) as defined in claim 7.

**9.** The method of screening of any one of claims 1 to 7, wherein, in the context of said contacting said test compound with said $Na_V$C, said test compound is contacted with an animal model which comprises (a) cell(s) as defined in claim 6 or 7.

**10.** The method of screening of any one of claims 1 to 9, wherein said cell, neuronal culture system or animal model is genetically engineered so that the spermine synthase (SMS) activity is inhibited in said cell, in said neuronal culture system or in said animal model.

**11.** The method of screening of any one of claims 1 to 10, wherein said animal model is an animal model selected from the group consisting of animal models of chronic epilepsy, chronic temporal lobe epilepsy (cTLE), refractory epilepsy, acquired epilepsy, pilocarpine models of epilepsy, models of genetic epilepsy.

**12.** The method of screening of claim 11, wherein said animal model is a KCNT1 model of genetic epilepsy which harbours the gain-of-function (GOF) missense mutation p.I335N in KCNT1.

13. The method of screening of any one of claims 1 to 12, wherein said animal model is a rat, a mouse, a rabbit, a guinea pig, or a monkey.

14. The method of screening of any one of claims 1 to 13, wherein said Na$_V$C is a Na$_V$1.2 channel, Na$_V$1.6 channel, Na$_V$1.1 channel, Na$_V$1.3 channel, Na$_V$1.4 channel, Na$_V$1.5 channel, Na$_V$1.7 channel, Na$_V$1.8 channel or Na$_V$1.9 channel.

15. The method of screening of any one of claims 1 to 8 and 10 to 14, wherein said method is an *in vitro* method practiced on a cell or on a neuronal culture system as defined in any one of claims 6 to 8 and 10 to 14; and wherein said method comprises

(i) contacting said test compound with said Na$_V$C in the presence of said N1aSPD concentration by a contact device;
(ii) setting, controlling and/or modifying said N1aSPD concentration by a control device; and/or
(iii) testing by a test device whether, upon said contacting said test compound with said Na$_V$C, said Na$_V$CB blocks said Na$_V$C in the presence of said N1aSPD concentration.

16. The method of screening of claim 15, wherein said contact device is a patch-clamp device, said control device is a patch-clamp device, and/or said test device is a patch-clamp device; or wherein said contact device is a microelectrode device or a microelectrode array (MEA) device, said control device is a microelectrode device or a MEA device, and/or said test device is a microelectrode device or a MEA device.

17. The method of screening of claim 16, wherein said patch-clamp device(s) is/are (a) whole-cell patch-clamp device(s), an outside-out patch-clamp device(s), a cell-attached patch-clamp device(s) or an inside-out patch-clamp device(s); or wherein said microelectrode device(s) or said MEA device(s) is/are or comprise(s) a MEA plate.

18. The method of screening of claim 16 or 17, wherein said patch-clamp device(s) is/are, or comprise(s), a patch-clamp micropipette or a planar patch-clamp device; and/or a patch-clamp chamber.

19. The method of screening of claim 18, wherein said planar patch-clamp device is a patch-clamp chip; and/or wherein said patch-clamp chamber is a superfusion device.

20. The method of screening of any one of claims 16 to 19, wherein

(i) said patch-clamp devices are whole-cell patch-clamp devices;
(ii) said patch-clamp micropipette or said planar patch-clamp device is used as both, as said control device and as said test device;
(iii) said patch-clamp chamber is used as said contact device;
(iv) said patch-clamp micropipette or said planar patch-clamp device of (ii) comprises an intracellular solution which contains said N1aSPD concentration as defined in claim 4;
(v) said patch-clamp chamber contains a bath solution containing said test compound;
(vi) said whole-cell patch-clamp devices are practiced on said cell, i.e. said cell is placed in said bath solution as contained in said patch-clamp chamber of (v); and said patch-clamp micropipette penetrates said cell, or said cell is adhered to said planar patch-clamp device, so that the intracellular milieu within said cell equilibrates with said intracellular solution; and
(vii) it is measured with said whole-cell patch-clamp devices whether said test compound blocks said Na$_V$C as located in the outer cell membrane of said cell in the presence of said N1aSPD concentration.

21. The method of screening of any one of claims 2 to 20, wherein said PRNHD is a pharmacoresistant epilepsy (PRE) or a pharmacoresistant pain disorder/pain-related disorder, a pharmacoresistant trigeminal neuralgia, a pharmacoresistant postherpetic neuralgia, a pharmacoresistant diabetic neuropathy, a pharmacoresistant Alzheimers disease (AD) or a pharmacoresistant stroke.

22. The method of screening of claim 21, wherein said pharmacoresistant pain disorder/pain-related disorder is a pharmacoresistant neuropathic pain or a pharmacoresistant hereditary pain syndrome.

23. A two-step method of screening for a Na$_V$CB comprising

(i) operating an *in vitro* method of screening for a $Na_VCB$ according to any one of claims 1 to 22;

(ii) subsequently operating an *in vivo* method of screening of any one of claims 1 to 22, with the $Na_VCB$ as identified according to (i) as said test compound.

**Figure 1.**

Figure 2.

EP 4 772 879 A1

45

**Figure 3.**

**Figure 4.**

**Figure 5.**

**Figure 6.**

**Figure 7.**

**Figure 8.**

a

N₁-acetylspermidine

b

N₁-acetylspermine

**Figure 9.**

**Figure 10.**

A

B

**Figure 11.**

Europäisches
Patentamt

European
Patent Office

Office européen
des brevets

**EUROPEAN SEARCH REPORT**

Application Number

EP 25 15 0474

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAO XIANZUN ET AL: "Reduction of spermine synthase enhances autophagy to suppress Tau accumulation", CELL DEATH & DISEASE, vol. 15, no. 5, 13 May 2024 (2024-05-13), XP093280086, GB ISSN: 2041-4889, DOI: 10.1038/s41419-024-06720-8 Retrieved from the Internet: URL:https://www.nature.com/articles/s41419-024-06720-8> * page 2, section "SMS knockdown in human cells"; pages 6-7, section "SMS knockdown reduces Tau accumulation in human neuronal and glial cell lines" *<br><br>-----<br><br>-/-- | 20 | INV.<br>G01N33/50<br>G01N33/68<br>C12N5/10 |
| | | | TECHNICAL FIELDS SEARCHED (IPC) |
| | | | G01N<br>C12N |

The present search report has been drawn up for all claims

| Place of search | Date of completion of the search | Examiner |
|---|---|---|
| The Hague | 23 May 2025 | Adida, Anne |

page 1 of 3

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | VETTER IRINA ET AL: "Characterisation of Nav types endogenously expressed in human SH-SY5Y neuroblastoma cells", BIOCHEMICAL PHARMACOLOGY, vol. 83, no. 11, 1 June 2012 (2012-06-01), pages 1562-1571, XP093280087, US ISSN: 0006-2952, DOI: 10.1016/j.bcp.2012.02.022 Retrieved from the Internet: URL:https://pdf.sciencedirectassets.com/271311/1-s2.0-S0006295212X00091/1-s2.0-S0006295212001645/main.pdf?hash=e72e5e33a60a06323ab1d0d38110f16d2e60d582d68e8ccc3b28544cbb3c63c4&host=68042c943591013ac2b2430a89b270f6af2c76d8dfd086a07176afe7c76c2c61&pii=S0006295212001645&tid=spdf-3c97026c-526c-4f37-8a4e-67e> This document completes the disclosure of D1, Tao at al., regarding the voltage-gated sodium channels expressed in SH-SY5Y cells.; * abstract * | 20 | |
| X | WO 2007/056099 A2 (ICAGEN INC [US]; WANG XIAODONG [US] ET AL.) 18 May 2007 (2007-05-18) * Paragraphs [0112]-[0117]; [0151]-[0157]; Table I * | 20 | **TECHNICAL FIELDS SEARCHED (IPC)** |

-/--

The present search report has been drawn up for all claims

| Place of search | Date of completion of the search | Examiner |
|---|---|---|
| The Hague | 23 May 2025 | Adida, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIU JIAYU ET AL: "The Potential Role of Polyamines in Epilepsy and Epilepsy-Related Pathophysiological Changes", BIOMOLECULES, vol. 12, no. 11, 29 October 2022 (2022-10-29), page 1596, XP093279703, CH ISSN: 2218-273X, DOI: 10.3390/biom12111596 * abstract * * figure 3 and section 2.2; figure 4 and section 5.1.1. * ----- | 1-20 | |
| A,D | WILCOX KAREN S ET AL: "The current approach of the Epilepsy Therapy Screening Program contract site for identifying improved therapies for the treatment of pharmacoresistant seizures in epilepsy", NEUROPHARMACOLOGY, ELSEVIER, AMSTERDAM, NL, vol. 166, 30 November 2019 (2019-11-30), XP086046069, ISSN: 0028-3908, DOI: 10.1016/J.NEUROPHARM.2019.107811 [retrieved on 2019-11-30] * the whole document * ----- | 1-20 | TECHNICAL FIELDS SEARCHED (IPC) |

The present search report has been drawn up for all claims

| Place of search | Date of completion of the search | Examiner |
|---|---|---|
| The Hague | 23 May 2025 | Adida, Anne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................................

& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 25 15 0474

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2025

| Patent document cited in search report | Publication date | Patent family member(s) | | Publication date |
|---|---|---|---|---|
| WO 2007056099 A2 | 18-05-2007 | AU | 2006311954 A1 | 18-05-2007 |
| | | CA | 2628312 A1 | 18-05-2007 |
| | | EP | 1945029 A2 | 23-07-2008 |
| | | JP | 2009514868 A | 09-04-2009 |
| | | US | 2007135493 A1 | 14-06-2007 |
| | | US | 2010234343 A1 | 16-09-2010 |
| | | WO | 2007056099 A2 | 18-05-2007 |

EPO FORM P0459

For more details about this annex : see Official Journal of the European Patent Office, No. 12/82

## REFERENCES CITED IN THE DESCRIPTION

**Patent documents cited in the description**

- US 20130345526 A1

**Non-patent literature cited in the description**

- **WIFFEN**. *Cochrane Database Syst Rev.*, vol. 2011 (1), 1-40
- **WIFFEN**. *Cochrane Database Syst Rev.*, 2014 (4), 1-41
- **THAM**. *Journal of Pain Research*, 2018, vol. 11, 1689-98
- **FISCHER**. *Ann Neurol.*, 2009, vol. 65 (6), 733-41
- **PERUCCA**. *The Lancet. Neurology*, 2007, vol. 6 (9), 793-804
- **PERUCCA**. *The Lancet. Neurology*, 2020, vol. 19 (6), 544-56
- **GOLYALA**. *Seizure*, 2017, vol. 44, 147-56
- **CHEN**. *JAMA neurology*, 2018, vol. 75 (3), 279-86
- **PERUCCA**. *Epilepsia*, 2024, vol. 65, 533-41
- **REMY**. *Brain: A Journal of Neurology*, 2006, vol. 129 (1), 18-35
- **REMY**. *Annals of Neurology*, 2003, vol. 53 (4), 469-79
- **ELLERKMANN**. *Neuroscience*, 2003, vol. 119 (2), 323-33
- **UEBACHS**. *The Journal of Neuroscience*, 2010, vol. 30 (25), 8489-501
- **LIU**. *Biomolecules*, 2022, vol. 12, 1-25
- **BECKONERT**. *The Journal of Neuroscience*, 2018, vol. 38 (24), 5596-605
- **SANABRIA**. *J Physiol*, 2001, vol. 532, 205-16
- **MÄNNIKKÖ**. *Handb Clin Neurol*, 2024, vol. 203, 1-23
- **DOESER**. *Brain*, vol. 138 (2), 371-87, https://en.wikipedia.org/wiki/Voltage-gated_sodium_channel
- **COMINI**. *Handb Clin Neurol*, 2024, 89-109
- Sodium Channelopathies in Human and Animal Models of Epilepsy and Neurodevelopmental Disorders. **YAMAKAWA**. Jasper's Basic Mechanisms of the Epilepsies. Oxford University Press, 2024
- **PAL**. *Bioorg Chem*, 2021, vol. 115, 105230
- **HABIBEY**. *Front Neurosci.*, 2022, vol. 16, 1-18
- **SCHOCH**. *J Neurosci.*, 2021, vol. 41 (39), 8111-25
- **NEGGERS**. *Nat Commun*, 2018, vol. 9 (1), 502
- **PROMMERSBERGER**. *Current Protocols in Immunology*, 2020, vol. 128, e93
- Patch Clamp Techniques. From Beginning to Advanced Protocols. Springer Protocols Handbooks, 2012
- **JOHNSTONE**. *Neurotoxicology*, 2010, vol. 31, 331-50
- **SPIRA**. *Nature Nanotech*, 2013, vol. 8, 83-94
- **WILCOX**. *Neuropharmacology*, 2020, vol. 166, 1-20
- **GUIGNET**. Noebels, Jasper's Basic Mechanisms of the Epilepsies. Oxford University Press, 2024
- **SCHOCH**. *The Journal of neuroscience: the official journal of the Society for Neuroscience*, 2021, vol. 41 (39), 8111-25
- **VAN LOO**. *The Journal of neuroscience: the official journal of the Society for Neuroscience*, 2019, vol. 39 (17), 3175-87
- **LOO**. *Nature communications*, 2015, vol. 6, 8688
- **KEHNE**. *Neurochemical research*, 2017, vol. 42 (7), 1894-1903
- **DITZEN**. *Molecular psychiatry*, 2010, vol. 15 (7), 702-711
- **WANG**. *molecular biology (Clifton, N.J.)*, 2011, vol. 720, 159-70
- **VAN LOO**. *Nature communications*, 2015, vol. 6, 8688